# EUROPEAN PATENT SPECIFICATION

(11) **EP 3 092 492 B1**
(45) Date of publication and mention of the grant of the patent: **04.12.2019**
(21) Application number: 15734826.9
(22) Date of filing: 08.01.2015
(51) Int. Cl.: G01N 33/574

(54) **TREATMENT OF TUMORS EXPRESSING MUTANT P53**
BEHANDLUNG VON MUTIERTEM P53 EXPRIMIERENDEN TUMOREN
TRAITEMENT DE TUMEURS EXPRIMANT LA P53 MUTANTE

(30) Priority: 09.01.2014 US 201461925278 P
(43) Date of publication of application: 16.11.2016
(73) Proprietor: Sloan-Kettering Institute for Cancer Research, New York, NY 10065 (US)
(72) Inventor: MANCHADO, Eusebio, New York, NY 10065 (US); WEISSMUELLER, Susann, New York, NY 10065 (US); LOWE, Scott, W., New York, NY 10065 (US); SABOROWSKI, Michael, New York, NY 10065 (US)
(74) Representative: Callaghan, Dayle Anne
(86) International application number: PCT/US2015/010653
(87) International publication number: WO 2015/105996

(56) References cited:
- US-A- 5 720 937
- US-A1- 2012 082 659
- US-A1- 2013 324 430
- US-A9- 2013 237 436
- US-B2- 8 377 636
- MONICA HERMANSON ET AL: "Association of Loss of Heterozygosity on Chromosome 1'7p with High Platelet derived Growth Factor a Receptor Expression in Human Malignant Gliomas", CANCER RESEARCH, vol. 56, 1 January 1996 (1996-01-01), pages 164-171, XP055387833,
- Gouri Chatterjee ET AL: "Acceleration of mouse mammary tumor virus-induced murine mammary tumorigenesis by a p53 172H transgene: influence of FVB background on tumor latency and identification of novel sites of proviral insertion", The American journal of pathology, 1 December 2002 (2002-12-01), pages 2241-2253, XP055387923, United States Retrieved from the Internet: URL:http://ac.els-cdn.com/S000294401064500 2/1-s2.0-S0002944010645002-main.pdf?_tid=a 8db6690-6149-11e7-b02f-00000aab0f26&acdnat =1499235645_dbb89f88d9608aa8a4709e45168bc7 de
- XIANGBING MENG ET AL: "Strategies for Molecularly Enhanced Chemotherapy to Achieve Synthetic Lethality in Endometrial Tumors with Mutant p53", OBSTETRICS AND GYNECOLOGY INTERNATIONAL, vol. 2013, 1 January 2013 (2013-01-01), pages 1-10, XP055387917, ISSN: 1687-9589, DOI: 10.1155/2013/828165
- T. J. ABOUANTOUN ET AL: "Imatinib blocks migration and invasion of medulloblastoma cells by concurrently inhibiting activation of platelet-derived growth factor receptor and transactivation of epidermal growth factor receptor", MOLECULAR CANCER THERAPEUTICS, vol. 8, no. 5, 1 May 2009 (2009-05-01), pages 1137-1147, XP055052348, ISSN: 1535-7163, DOI: 10.1158/1535-7163.MCT-08-0889
- SANNA-MARIA HEDE ET AL: "GFAP promoter driven transgenic expression of PDGFB in the mouse brain leads to glioblastoma in a Trp53 null background", GLIA, vol. 57, no. 11, 15 August 2009 (2009-08-15), pages 1143-1153, XP055387930, ISSN: 0894-1491, DOI: 10.1002/glia.20837
- PODTCHEKO A ET AL: "The selective tyrosine kinase inhibitor, STI571, inhibits growth of anaplastic thyroid cancer cells", JOURNAL OF CLINICAL ENDOCRINOLOGY AND METABO, THE ENDOCRINE SOCIETY, US, vol. 88, no. 4, 20 April 2003 (2003-04-20) , pages 1889-1896, XP009017078, ISSN: 0021-972X, DOI: 10.1210/JC.2002-021230
- CARL-HENRIK HELDIN: "Targeting the PDGF signaling pathway in tumor treatment", CELL COMMUNICATION AND SIGNALING, BIOMED CENTRAL, LONDON, GB, vol. 11, no. 1, 20 December 2013 (2013-12-20), page 97, XP021171368, ISSN: 1478-811X, DOI: 10.1186/1478-811X-11-97
- WEISSMUELLER ET AL.: 'Mutant p53 Drives Pancreatic Cancer Metastasis through Cell Autonomous PDGF Receptor b Signaling' CELL vol. 157, no. ISS. 2, 10 April 2014, pages 382 - 394, XP055180902

## Description

### CROSS REFERENCE TO RELATED APPLICATIONS

This application claims the benefit of U.S. Provisional Application Serial No. 61/925,278, filed January 9, 2014.

### BACKGROUND

The p53 gene, called a tumor suppressor gene, is present in all cells. Expression of p53 protein is induced following different forms of cellular stress, where it leads to various anti-proliferative outcomes. Normally, p53 acts to induce expression of various anti-proliferative genes by activating transcription. Mutation in the p53 gene is the most common mutation in cancer; it is present in about half of all cancer tumors, 80% in all colon cancer tumors, 50% of lung cancer tumors, and 40% of breast cancer tumors. Thus, there is a need to identify and develop agents that target the effects of mutant p53 for cancer therapeutics.

Xiangbing Meng et al. (2013) Obstetrics and Gynecology International. 2013:1-10 reports the effects of BIBF1120 on endometrial cancer cells. Abouantoun et al. (2009) Molecular Cancer Therapeutics. 8:1137-1147 demonstrates the effect of imatinib on D556 cancer cells. Sanna-Maria Hede et al. (2009) Glia. 57:1143-1153 demonstrates the formation of brain tumours in mice overexpressing PDGFb. Podtcheko A et al. (2003) Journal of Clinical Endocrinology and Metabolism. 88:1889-1896 demonstrate the activity of a tyrosine kinase inhibitor, STI571, on thyroid cancer cells. Carl-Henrik Heldin (2013) Cell Communication and Signaling. 11:97 reviews the role of PDGF signaling in tumour development.

### SUMMARY

The present disclosure encompasses the discovery that mutant p53-induced upregulation of the platelet-derived growth factor receptor b (PDGFRb) contributes to invasion and/or metastasis of tumors expressing mutant p53. Specifically, the present disclosure encompasses the discovery that mutant p53 can disrupt a p73/NF-Y complex (which can repress PDGFRB transcription), leading to transcription of PDGFRb. The present disclosure therefore provides, at least in part, PDGFRb modulators (e.g., activators or inhibitors) for use in medicine, and specifically in diagnosis, treatment and/or prevention (e.g., delay of onset) of certain disorders, e.g., cancer.

In one aspect the invention is directed to a therapeutic agent that targets a platelet derived growth factor receptor beta (PDGFRβ) for use in treating or preventing tumor metastasis, in a subject having a gain of function p53 mutation that increases its oncogenic properties relative to wild type, wherein the tumor is a pancreatic cancer tumor, a colorectal cancer tumor, or an ovarian cancer tumor.

### BRIEF DESCRIPTION OF THE DRAWINGS

The following figures are presented for the purpose of illustration only, and are not intended to be limiting.
FIG. 1A (left panel) is a graph of quantifications of quantifications of wound distances in scratch-wound assays from 0, 3, 7, and 10 hrs after wounding of polyclonal KPC cells stably expressing a nontargeting control shRNA (sh.Ctrl) or a shRNA targeting mutant p53 (sh.p53) or polyclonal KP_{fl}C cells stably expressing a sh.Ctrl. The average gap width from three independent locations ±SD is shown. *** indicates p < 0.001 as determined by a Student's t test. FIG. 1A (right panel) are representative phase contrast images from live cell recordings of each condition at 0 and 10 hours.

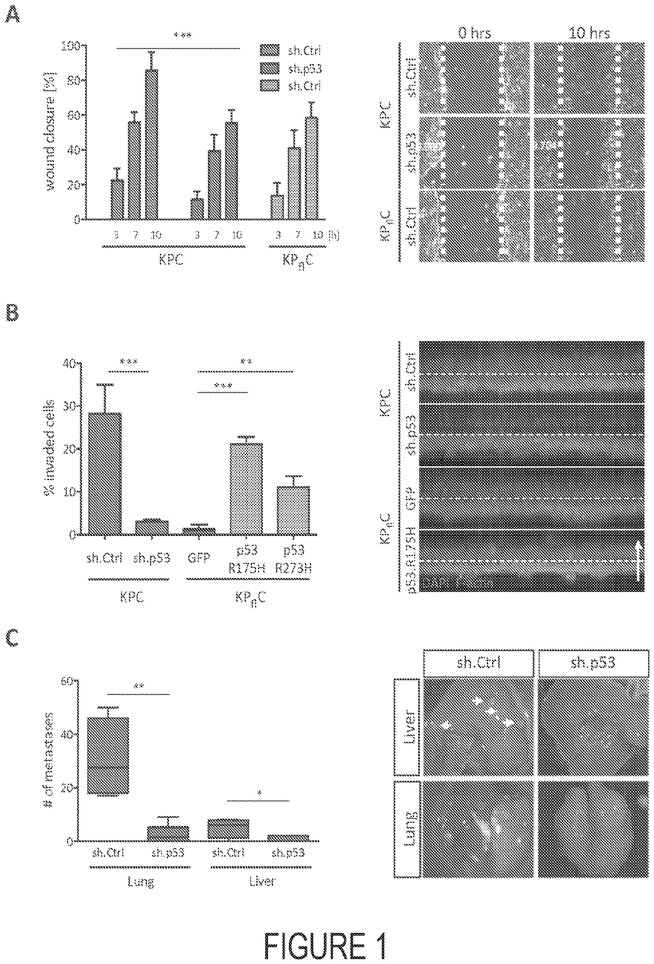

FIG. 1B (left panel) is a graph of invasion of KPC+sh.p53 and +sh.Ctrl as well as KP_{fl}C cells expressing the GFP control and mutant p53 (175H and 273H) vector. Cells were allowed to invade into Collagen for 72 h before quantification. The average of invaded cells from 9 replicates ±SD is shown. A representative result of three repeated experiments is shown. ** and **** indicates p < 0.005 and p < 0.0001, respectively. FIG. 1B (right panel) are representative 3D reconstructions of each condition described in FIG. 1A. Cells were stained for F-actin (red) and DAPI (blue); dashed line indicates the approximate position of the Transwell membrane; the arrow indicates the direction of movement.
FIG. 1C (left panel) is a graph of number of metastases following orthotopic injection of KPC+sh.p53 or +sh.Ctrl cells in the pancreas of athymic mice. When symptomatic, mice were euthanized and metastatic spread in lung and liver was quantified by counting GFP-positive macroscopic nodules. Data are represented as mean ±SD. FIG. 1C (right panel) are representative merged brightfield/GFP images from lung and liver.
FIG. 2A is a schematic workflow of RNA sequencing. 1, 2, and 3 represent individual clonal cell lines of KPC+sh.p53 and +sh.Ctrl.
FIG. 2B is a graph (p < 0.05) of molecular and cellular functions that are significantly changed following mutant p53 depletion, analyzed using ingenuity pathways analysis (Ingenuity Systems, www.ingenuity.com).
FIG. 2C is a graph of one-by-one invasion assay screen. Quantification of invaded KPC cells infected with individual shRNA-pools (average of 3.6 shRNAs per gene) targeting the top 40 upregulated genes identified by RNAseq. The three genes that abrogated invasion most efficiently are marked in black.
FIG. 2D is a graph of qRT-PCR for PDGFRb in KPC+sh.p53 (1 or 2) or +sh.Ctrl cells. Data represent mean normalized PDGFRb expression ±SD of triplicate samples. A representative result of three repeated experiments is shown.
FIG. 2E depicts Western blotting analysis of PDGFRb and p53 in sh.p53(1 or 2)- or sh.Ctrl-expressing KPC cells. The two bands of PDGFRb represent differentially glycosylated forms of the protein. Actin expression was used as loading control.
FIG. 3A is a graph of PDGFRa, PDGFRb and Actin levels of KPC cells infected with shRNAs targeting PDGFRa, PDGFRb or a nontargeting control (Ctrl) as determined by western blotting.
FIG. 3B is a graph of quantification of the invasion into collagen of cell lines from FIG. 3A, compared to KPC+sh.p53. Values are means ±SD of 9 replicates from one representative experiment. ** and *** indicates p < 0.005 and p < 0.001, respectively, as determined by a Student's t test.
FIG. 3C is a graph of qRT-PCR for PDGFRb in 21 human pancreatic cancer cell lines of different p53 status. Data represent mean normalized PDGFRb expression ±SD of triplicate samples.
FIG. 3D (top panel) is a graph of qRT-PCR for PDGFRb in the human pancreatic cancer cell lines Miapaca2, BXPC3, CFPAC, and A2.1 expressing a shRNA targeting p53 or a nontargeting control (Ctrl). Data represent mean normalized PDGFRb expression ±SD of triplicate samples from one representative experiment. * and *** indicates p < 0.05 and p < 0.001, respectively. Mutation status of p53 in each cell line as indicated. FIG. 3D (bottom panel) depicts p53 levels of indicated cell populations determined by western blotting. Actin was used as a loading control.
FIG. 3E (right panel) is a graph of quantification of the invaded human A2.1 cells infected with shRNAs targeting PDGFRb, p53 or for a nontargeting control (Ctrl). Values are means ±SD of 9 replicates from one representative experiment. FIG. 3E (left panel) are representative 3D reconstructions of invaded cells infected with constructs as indicated. Cells were stained for F-actin (red) and DAPI (blue); dashed line indicates the approximate position of the Transwell membrane; the arrow indicates the direction of movement.
FIG. 4A depict Western blots of KP_{fl}C cells stably expressing a GFP control or mutant p53 (175H or 273H) vector that were transfected with HA.TAp73α. Either p53 or HA were immunoprecipitated and the expression of HAp73, mutant p53, or NF-YB was determined in both the input (10% of lysates) and immunoprecipitation.
FIG. 4B is a graph of chromatin immunoprecipitation (ChIP) using NF-YB antibodies in KP_{fl}C cells stably expressing shCtrl, shNF-Y or HAp73. Values are means ±SD.

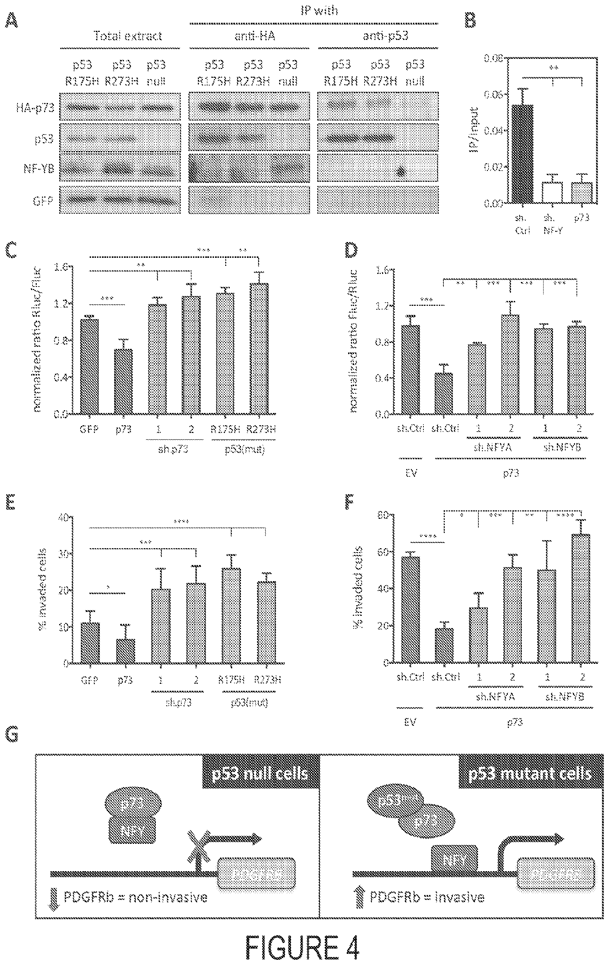

FIG. 4C is a graph of KP_{fl}C cells stably expressing a GFP control, HAp73, sh.p73 (1 or 2), or mutant p53 (175H or 273H) vector that were co-transfected with the PDGFRB-promoter-luciferase construct and renilla-luciferase vector. Firefly-luciferase expression was determined and displayed as relative Firefly-luciferase (Fluc) units normalized by renilla expression (Rluc). The firefly-luciferase activity of the cell lysates expressing the GFP control vector was set to 1. Values are mean promoter activity ±SD of quadruplicate samples. A representative result of three repeated experiments is shown.
FIG. 4D is a graph of KP_{fl}C cells expressing the GFP control vector together with sh.Ctrl as well as KP_{fl}C cells stably expressing HAp73 construct together with sh.Ctrl, sh.NF-YA (1 or 2), or sh.NF-YB (1 or 2) that were co-transfected with the PDGFRB-promoter-luciferase construct and renilla-luciferase vector. Luciferase activity was measured as described in FIG. 4C.
FIG. 4E is a graph of quantification of invasion of the same cells as in FIG. 4C. Values are means ±SD of 9 replicates from one representative experiment.
FIG. 4F is a graph of quantification of invasion of same cells as in FIG. 4D. Values are means ±SD of 9 replicates from one representative experiment.
FIG. 4G is a schematic representation summarizing a proposed mechanism of action of mutant p53 in the promotion of an invasive pancreatic cancer phenotype.
FIG. 5A (left panel) is a graph of lung colonization assays after tail vein injection of PDGFRa-, PDGFRb (1 or 2)-, p53-, and control-depleted KPC cells. Total number of lung metastatic nodules in individual mice was counted on serial histological sections (at least six mice per each group). Data are represented as mean ±SD. FIG. 5A (right panel) are representative merged brightfield/GFP images of whole lung from indicated mice.
FIG. 5B is a graph of MTS assay (E₄₉₀) of murine KPC and human A2.1 cells treated with crenolanib with various doses for 72 hours. Normalized values are expressed as means ± SD form quadruple replicates.
FIG. 5C depicts immunoprecipitation of PDGFRb from KPC cells starved for 12 hours, treated with either increasing doses of crenolanib or DMSO for 4 hours and subsequently stimulated with 50 ng/ml of PDGF-BB recombinant protein. Protein levels of PDGFRb, phospho-Tyrosine and Tubulin were determined by western blotting.
FIG. 5D (left panel) is a graph of quantification of invasion of murine KPC and human A2.1 treated with either DMSO or crenolanib at 300 nM. Values are means ±SD of 9 replicates from one representative experiment. FIG. 5D (right panel) are representative 3D reconstructions of invaded cells infected with constructs as indicated. Cells were stained for F-actin (red) and DAPI (blue); dashed line indicates the approximate position of the Transwell membrane; the arrow indicates the direction of movement.
FIG. 5E (left panel) are representative merged brightfield/GFP imaged of whole lung as well as H&E stains of representative sections of pulmonary lobes after tail vein injection of crenolanib- or DMSO-treated KPC cells into mice. FIG. 5E (right panel) is a graph of quantification of total number of lung metastatic nodules in individual mouse (at least six mice per each group). Data are represented as mean ±SD.
FIG. 6A depicts immunoprecipitation of PDGFRb from KPC cells starved for 12 hours, treated with either increasing doses of imatinib or DMSO for 4 hours and subsequently stimulated with 50 ng/ml of PDGF-BB recombinant protein. Protein levels of PDGFRb, phospho-Tyrosine and Tubulin were determined by western blotting.
FIG. 6B depicts results of lung colonization assays after tail vein injection of imatinib- or DMSO-treated KPC cells. FIG. 6B (left panel) are representative merged brightfield/GFP images of whole lung. FIG. 6B (middle panel) are H&E stainings of representative sections of pulmonary lobes. Arrows indicate metastases. FIG 6B (right panel) is a graph of quantification of total number of lung metastatic nodules in individual mouse (at least six mice per each group. Data are represented as mean ±SD.
FIG. 6C is a graph of weight of pancreatic tumors of KPC mice treated with vehicle or imatinib at time of death.
FIG. 6D is a graph of quantification of the number of mice with metastatic disease at the time of death. Values are percentages of the total number of mice in each cohort. Colored columns represent mice with metastases (METS) and white columns represent disease-free (DF) animals.
FIG. 6E is a graph of quantification of the number of mice with lung, lymph nodes or liver metastatic disease at the time of death. Values are percentages of the total number of mice in each cohort. Colored columns represent mice with metastases (METS) and white columns represent disease-free (DF) animals.
FIG. 6F are H&E stainings of representative sections of harvested organs (primary tumor, lung, liver, lymphatic system) from vehicle and imatinib-treated animals. Images at 10x and 40x (insert) magnification.
FIG. 6G are representative immunofluorescence images of pancreatic tumors of KPC mice treated either with vehicle or imatinib. DAPI, blue; CK8, red and p-PDGFRb, green.
FIG. 7A are Kaplan-Meier survival curves of 103 pancreatic cancer patients (clinical variable = DFS) as a function of PDGFRb-high versus PDGFRb-low expressing tumors.
FIG. 7B is a box plot of PDGFRb expression versus tumor grade of pancreatic tumors.
FIG. 7C is a graph of clinicopathologic analysis of vascular space invasion of pancreatic cancer patients stratified by the expression levels of PDGFRb in the primary tumor.
FIG. 7D is a graph of quantification of pPDGFRb expression in human PDAC samples stratified by p53 expression levels. p53 and pPDGFRb levels were assessed by IHC in a panel TMA of human pancreatic cancer (n = 961) and scored using a relative scale from 0 to 3.
FIG. 7E are Kaplan-Meier survival curves of colorectal cancer patients (clinical variable = DFS) as a function of PDGFRb-high versus PDGFRb-low expressing tumors.
FIG. 7F is a box plot of PDGFRb expression versus tumor grade of colon tumors.
FIG. 8A is a heatmap of significantly changed genes (p_{adj} < 0.05) following mutant p53 depletion, as identified by RNA sequencing. Three individual clonal cell lines of KPC+sh.p53 and +sh.Ctrl were analyzed and representative top scoring genes are labeled.
FIG. 8B depicts Western blotting analysis of activated downstream PDGF receptor b pathways following knockdown of mutant p53 in KPC cells. Actin expression was used as loading control.
FIG. 8C is a graph depicting representative pathways in KPC cells. Blue bars that cross the threshold line (p < 0.05) represent top scoring pathways that are significantly changed in mutant p53-depleted KPC cells. Data was analyzed through the use of ingenuity pathways analysis.
FIG. 9A (left panel) is a graph of quantification of the invasion into collagen of non-invasive KP_{fl}C cells stably expressing a GFP control, mutant p53 (175H or 273H) or PDGFRb cDNA vectors. Values are means ±SD of 9 replicates from one representative experiment. ** and **** indicates p < 0.005 and p < 0.0001, respectively, as determined by a Student's t test. FIG. 9A (right panel) depicts Western blotting analysis of PDGFRb and p53 from same cells. Actin expression was used as loading control.
FIG. 9B is a graph of cell number over time of KPC cells stably expressing sh.Ctrl or sh.PDGFRb (1 or 2).
FIG. 9C is a graph of negative selection RNAi studies in KPC cells stably expressing dox-inducible sh.Ctrl, sh.RPA3 or sh.PDGFRb (1 or 2) using the tet-on TRMPV system. Graphs represent the percentage of shRNA-expressing (Venus⁺ dsRed⁺) cells over time, normalized to initial measurement 1 d after dox treatment.
FIG. 9D (left panel) is a schematic of dual-color competitive proliferation assay in vivo for evaluating effects of RNAi-mediated PDGFRb suppression in tumor growth. KPC cells were transduced with indicated experimental shRNAs (GFP⁺) and a neutral control shRNA (Cherry⁺). FIG. 9D (right panel) is a graph of percentage of cells expressing indicated experimental shRNA (GFP⁺) or the neutral control shRNA (Cherry⁺) in pre-injected cells and tumors 2 weeks after injection. Values represent the mean of multiple pre-injected or tumor-derived cell lines.
FIG. 10A (top panel) is a graph of quantification of individual PDGFRb levels of 21 human pancreatic cancer cell lines as measured by qRT-PCR. Data represent mean normalized PDGFRb expression ±SD of triplicate samples. FIG. 10A (bottom panel) depicts Western blotting analysis for p53 of representative cell lines.
FIG. 10B is a graph of quantification of the invasion into collagen of human *p*53-/- ASPC pancreatic cancer cells stably expressing a GFP control or PDGFRb cDNA vector. Values are means ±SD of 9 replicates from one representative experiment. *** indicates p < 0.001 as determined by a Student's t test.
FIG. 10C depicts Western blotting analysis of PDGFRb and p53 levels in human pancreatic, colon, lung, and breast cancer cells stably expressing sh.RNA or sh.p53 (1 or 2). Actin was used as a loading control. p53 mutation is given together with the name of the cell line.
FIG. 11A is a graph of qRT-PCR for c in KPC+sh.p73 (1 or 2) or +sh.Ctrl cells. Data represent mean normalized p73 expression ±SD of triplicate samples. A representative result of three repeated experiments is shown.
FIG. 11B depicts NF-YA, NF-YB and Actin levels of KP_{fl}C cells infected with shRNAs targeting NF-YA, NF-YB or a non-targeting control (Ctrl) as determined by western blotting.

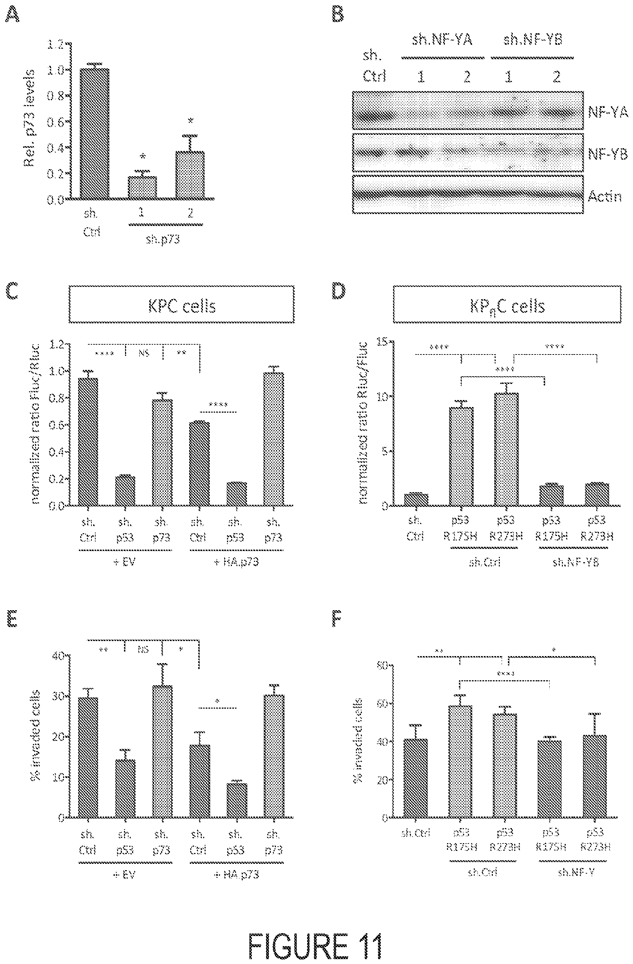

FIG. 11C is a graph of firefly-luciferase expression in KPC cells. After double infection using an empty control or HAp73 vector together with sh.Ctrl, sh.p53, or sh.p73, KPC cells were co-transfected with the *PDGFRB*-promoter-luciferase construct and renilla-luciferase vector. Firefly-luciferase expression was determined and displayed as relative Firefly-luciferase (Fluc) units normalized by renilla expression (Rluc). The firefly-luciferase activity of the cell lysates expressing the GFP control vector was set to 1. Values are mean promoter activity ±SD of quadruplicate samples. A representative result of three repeated experiments is shown.
FIG. 11D is a graph of firefly-luciferase expression in KP_{fl}C cells. KP_{fl}C cells stably expressing sh.Ctrl or sh.NF-YB together with mutant p53 (175H or 273H) were co-transfected with the PDGFRB-promoter-luciferase construct and renilla-luciferase vector. Luciferase activity was measured as described in FIG. 11C.
FIG. 11E is a graph of quantification of invasion of the same cells as in FIG. 11C. Values are means ±SD of 9 replicates from one representative experiment.
FIG. 11F is a graph of quantification of invasion of the same cells as in FIG. 11D. Values are means ±SD of 9 replicates from one representative experiment.
FIG. 12A depict hematoxylin and eosin (H&E) staining of representative sections of pulmonary lobes from indicated mice after tail vein injection of PDGFRa-, PDGFRb (1 or 2)-, p53-, and control-depleted KPC cells. Arrows indicate metastases.
FIG. 12B is a graph of relative nodule size of lung metastases from mice intravenously injected with PDGFRa-, PDGFRb (1 or 2)-, and control-depleted KPC cells. Data are represented as mean ±SD.
FIG. 12C depicts GPF immunohistochemstry on histological lung sections from lung colonization assay in FIG. 12B. shRNA expression correlates with GFP signal, as the fluorescent marker is linked to the hairpin.
FIG. 12D depicts Western blotting for phospho-PDGFRb, PDGFRb, and Actin in human A2.1 cells after treatment with DMSO or crenolanib at varying doses.
FIG. 12E depicts immunoprecipitation of PDGFRb from KPC cells treated with DMSO or crenolanib (300 nM) for different time periods. The input protein levels for PDGFRb, phospho-Tyrosine, and Actin and those present in immunoprecipitates for phospho-Tyrosine were determined by western blotting.
FIG. 12F is a graph of Propidium Iodide staining of KPC cells treated overnight with either DMSO, Crenolanib (0.3 (Creno Low) or 25 µM (Creno High)) or imatinib (3 µM).
FIG. 12G (left panel) depicts representative merged brightfield/GFP images of pulmonary lobes after tail vein injection of crenolanib- or DMSO-treated KP_{fl}C cells into mice. FIG. 12G (middle panel) depicts H&E stainings of representative sections of pulmonary lobes. FIG. 12G (right panel) is a graph of quantification of total number of lung metastatic nodules in individual mouse (at least six mice per each group). Data are represented as mean ±SD.
FIG. 13A is a graph of MTS assay (E₄₉₀) of KPC cells treated with imatinib with various doses for 72 hours. Normalized values are expressed as means ± SD form quadruple replicates.
FIG. 13B is a graph of quantification of invasion of KPC cells treated with either DMSO or imatinib at 3 µM. Values are means ±SD of 9 replicates from one representative experiment.
FIG. 13C are Kaplan-Meier survival curves of mice treated with vehicle or imatinib (50 mg/kg) from 8 weeks of age.
FIG. 13D are representative bright field images of pancreatic tumor, liver, lung and lymphatic system from KPC mice treated with vehicle or imatinib. Black arrows denote metastases.
FIG. 13E is a graph of quantification of p-PDGFRb intensity in pancreatic tumors of KPC mice treated with vehicle or imatinib. pPDGFRb was assessed by immunofluorescence and its intensity scored from 0 (pPDGFRb low levels) to 3 (pPDGFRb high levels). 15 images were analyzed per tumor (n=7).
FIG. 14A (left panel) is a table of stratification of human PDAC samples based on high and low pPDGFRb and p53 expression levels. Chi-Square test was performed. Levels of pPDGFRb were determined by IHC. FIG. 14A (righ panel) depicts representative IHC images.
FIG. 14B are Kaplan-Meier survival curves of colorectal cancer patients (clinical variable = DFS) as a function of the expression levels of the 40 genes from the mutant p53 gene signature, PDGFRb, SLC40A1, and SNED1.
FIG. 14C are Kaplan-Meier survival curves of ovarian cancer patients (clinical variable = DFS). Patients were stratified as in FIG. 14B.

### DEFINITIONS

In order for the present invention to be more readily understood, certain terms are first defined below. Additional definitions for the following terms and other terms are set forth throughout the specification.
*Characteristic portion*: As used herein, the term a "characteristic portion" of a substance, in the broadest sense, is one that shares some degree of sequence or structural identity with respect to the whole substance. In certain embodiments, a characteristic portion shares at least one functional characteristic with the intact substance. For example, a "characteristic portion" of a polypeptide or protein is one that contains a continuous stretch of amino acids, or a collection of continuous stretches of amino acids, that together are characteristic of a polypeptide or protein. In some embodiments, each such continuous stretch generally contains at least 2, 5, 10, 15, 20, 50, or more amino acids. In general, a characteristic portion of a substance (e.g., of a polypeptide or protein) is one that, in addition to the sequence and/or structural identity specified above, shares at least one functional characteristic with the relevant intact substance. In some embodiments, a characteristic portion may be biologically active.
*Characteristic sequence*: A "characteristic sequence" is a sequence that is found in all members of a family of polypeptides or nucleic acids, and therefore can be used by those of ordinary skill in the art to define members of the family.
*Combination therapy*: The term "combination therapy", as used herein, refers to those situations in which two or more different pharmaceutical agents are administered in overlapping regimens so that the subject is simultaneously exposed to both agents. When used in combination therapy, two or more different agents may be administered simultaneously or separately. This administration in combination can include simultaneous administration of the two or more agents in the same dosage form, simultaneous administration in separate dosage forms, and separate administration. That is, two or more agents can be formulated together in the same dosage form and administered simultaneously. Alternatively, two or more agents can be simultaneously administered, wherein the agents are present in separate formulations. In another alternative, a first agent can be administered just followed by one or more additional agents. In the separate administration protocol, two or more agents may be administered a few minutes apart, or a few hours apart, or a few days apart.
*Homology:* As used herein, the term "homology" refers to the overall relatedness between polymeric molecules, e.g., between nucleic acid molecules (e.g., DNA molecules and/or RNA molecules) and/or between polypeptide molecules. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% identical. In some embodiments, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% similar.
*Identity:* As used herein, the term "identity" refers to the overall relatedness between polymeric molecules, e.g., between nucleic acid molecules (e.g., DNA molecules and/or RNA molecules) and/or between polypeptide molecules. Calculation of the percent identity of two nucleic acid sequences, for example, can be performed by aligning the two sequences for optimal comparison purposes (e.g., gaps can be introduced in one or both of a first and a second nucleic acid sequences for optimal alignment and non-identical sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or substantially 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using the algorithm of Meyers and Miller (CABIOS, 1989, 4: 11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix.
*Mutant p53:* As used herein, the term "mutant p53 gene" or "mutant p53 polypeptide" refers to a gene or a polypeptide whose nucleotide or amino acid sequence includes at least one characteristic sequence of and/or shows at least 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, or 70% identity with a wild-type p53 gene or polypeptide described herein, e.g., SEQ ID NO:1 or SEQ ID NO:2. In some embodiments, a mutant p53 polypeptide shares at least one characteristic sequence of and/or shows the specified degree of overall sequence identity with one of the p53 sequences set forth herein (each of which may be considered a "reference" p53). In some embodiments, a mutant p53 polypeptide as described herein shares at least one biological activity with a reference p53 polypeptide as set forth herein. In some embodiments, a mutant p53 polypeptide does not exhibit a detectable p53 tumor suppressive activity. In some embodiments, a mutant p53 gene or polypeptide differs from a wild-type p53 gene or polypeptide at one or more nucleotides or amino acid residues.
*PDGFRb polypeptide*: The term "PDGFRb polypeptide" or "platelet derived growth factor receptor b polypeptide" refers to a polypeptide whose amino acid sequence includes at least one characteristic sequence of and/or shows at least 100%, 99%, 98%, 97%, 96%, 95%, 94%, 93%, 92%, 91%, 90%, 89%, 88%, 87%, 86%, 85%, 84%, 83%, 82%, 81%, 80%, 79%, 78%, 77%, 76%, 75%, 74%, 73%, 72%, 71%, or 70% identity with a PDGFRb polypeptide described herein, e.g., SEQ ID NO:4. A wide variety of PDGFRb sequences from flies, vertebrates, and mammals are known in the art, such as those described herein; in some embodiments, a PDGFRb shares at least one characteristic sequence of and/or shows the specified degree of overall sequence identity with one of the PDGFRb sequences set forth herein (each of which may be considered a "reference" PDGFRb). In some embodiments, a PDGFRb as described herein shares at least one biological activity with a reference PDGFRb as set forth herein. In some such embodiments, the shared biological activity relates to PDGF signaling pathway activation or inhibition.
*PDGFRb inhibitor:* The term "PDGFRb inhibitor", as used herein, refers to any substance that inhibits or reduces the expression and/or activity of a PDGFRb. In some embodiments, a PDGFRb inhibitor is a substance that inhibits the transcription, expression, binding, activity or stability of a PDGFRb and/or a nucleic acid encoding such PDGFRb.
*PDGFRb modulator*: The term "PDGFRb modulator", as used herein, refers to any substance that modulates activity and/or expression of a PDGFRb. In some embodiments, a PDGFRb modulator modulates the transcription, binding, activity and/or stability of a PDGFRb and/or a nucleic acid encoding such PDGFRb. In some embodiments, a PDGFRb modulator is a PDGFRb activator. In some embodiments, a PDGFRb modulator is a PDGFRb inhibitor.
*Polypeptide*: As used herein, a "polypeptide", generally speaking, is a string of at least two amino acids attached to one another by a peptide bond. In some embodiments, a polypeptide may include at least 3-5 amino acids, each of which is attached to others by way of at least one peptide bond. Those of ordinary skill in the art will appreciate that polypeptides sometimes include "non-natural" amino acids or other entities that nonetheless are capable of integrating into a polypeptide chain, optionally.
*Protein*: As used herein, the term "protein" refers to a polypeptide (i.e., a string of at least two amino acids linked to one another by peptide bonds). Proteins may include moieties other than amino acids (e.g., may be glycoproteins, proteoglycans, etc.) and/or may be otherwise processed or modified. Those of ordinary skill in the art will appreciate that a "protein" can be a complete polypeptide chain as produced by a cell (with or without a signal sequence), or can be a characteristic portion thereof. Those of ordinary skill will appreciate that a protein can sometimes include more than one polypeptide chain, for example linked by one or more disulfide bonds or associated by other means. Polypeptides may contain L-amino acids, D-amino acids, or both and may contain any of a variety of amino acid modifications or analogs known in the art. Useful modifications include, e.g., terminal acetylation, amidation, methylation, etc. In some embodiments, proteins may comprise natural amino acids, non-natural amino acids, synthetic amino acids, and combinations thereof. The term "peptide" is generally used to refer to a polypeptide having a length of less than about 100 amino acids, less than about 50 amino acids, less than 20 amino acids, or less than 10 amino acids.
*Reference sample*: As used herein, a reference sample may include, but is not limited to, any or all of the following: a cell or cells, a portion of tissue, blood, serum, ascites, urine, saliva, and other body fluids, secretions, or excretions. The term "sample" also includes any material derived by processing such a sample. Derived samples may include nucleotide molecules or polypeptides extracted from the sample or obtained by subjecting the sample to techniques such as amplification or reverse transcription of mRNA, etc.
*Subject*: As used herein, the term "subject" or "patient" refers to any organism upon which embodiments of the invention may be used or administered, e.g., for experimental, diagnostic, prophylactic, and/or therapeutic purposes. Typical subjects include animals (e.g., mammals such as mice, rats, rabbits, non-human primates, and humans; insects; worms; etc.).
*Suffering from*: An individual who is "suffering from" a disease, disorder, or condition (e.g., a cancer) has been diagnosed with and/or exhibits one or more symptoms of the disease, disorder, or condition. In some embodiments, an individual who is suffering from cancer has cancer, but does not display any symptoms of cancer and/or has not been diagnosed with a cancer.
*Susceptible to*: An individual who is "susceptible to" a disease, disorder, or condition (e.g., cancer) is at risk for developing the disease, disorder, or condition. In some embodiments, an individual who is susceptible to a disease, disorder, or condition does not display any symptoms of the disease, disorder, or condition. In some embodiments, an individual who is susceptible to a disease, disorder, or condition has not been diagnosed with the disease, disorder, and/or condition. In some embodiments, an individual who is susceptible to a disease, disorder, or condition is an individual who displays conditions associated with development of the disease, disorder, or condition. In some embodiments, a risk of developing a disease, disorder, and/or condition is a population-based risk.
*Symptoms are reduced*: According to the present invention, "symptoms are reduced" when one or more symptoms of a particular disease, disorder or condition is reduced in magnitude (e.g., intensity, severity, etc.) or frequency. For purposes of clarity, a delay in the onset of a particular symptom is considered one form of reducing the frequency of that symptom. It is not intended that the present invention be limited only to cases where the symptoms are eliminated. The present invention specifically contemplates treatment such that one or more symptoms is/are reduced (and the condition of the subject is thereby "improved"), albeit not completely eliminated.
*Target cell or target tissue*: As used herein, the terms "target cell" or "target tissue" refer to any cell, tissue, or organism that is affected by a condition described herein and to be treated, or any cell, tissue, or organism in which a protein involved in a condition described herein is expressed. In some embodiments, target cells, target tissues, or target organisms include those cells, tissues, or organisms in which there is a detectable or abnormally high amount of PDGFRb activity and/or expression (e.g., comparable to that observed in subjects suffering from or susceptible to cancer). In some embodiments, target cells, target tissues, or target organisms include those cells, tissues or organisms that display a disease-associated pathology, symptom, or feature.
*Therapeutic regimen*: As used herein, the term "therapeutic regimen" refers to any method used to partially or completely alleviate, ameliorate, relieve, inhibit, prevent, delay onset of, reduce severity of and/or reduce incidence of one or more symptoms or features of a particular disease, disorder, and/or condition. It may include a treatment or series of treatments designed to achieve a particular effect, e.g., reduction or elimination of a detrimental condition or disease such as cancer. The treatment may include administration of one or more compounds either simultaneously, sequentially or at different times, for the same or different amounts of time. Alternatively, or additionally, the treatment may include exposure to radiation, chemotherapeutic agents, hormone therapy, or surgery. In addition, a "treatment regimen" may include genetic methods such as gene therapy, gene ablation or other methods known to reduce expression of a particular gene or translation of a gene-derived mRNA.
*Therapeutic agent*: As used herein, the phrase "therapeutic agent" refers to any agent that, when administered to a subject, has a therapeutic effect and/or elicits a desired biological and/or pharmacological effect.
*Therapeutically effective amount*: As used herein, the term "therapeutically effective amount" refers to an amount of an agent (e.g., a PDGFRb modulator) that confers a therapeutic effect on the treated subject, at a reasonable benefit/risk ratio applicable to any medical treatment. The therapeutic effect may be objective (i.e., measurable by some test or marker) or subjective (i.e., subject gives an indication of or feels an effect). In particular, the "therapeutically effective amount" refers to an amount of a therapeutic agent or composition effective to treat, ameliorate, or prevent a desired disease or condition, or to exhibit a detectable therapeutic or preventative effect, such as by ameliorating symptoms associated with the disease, preventing or delaying the onset of the disease, and/or also lessening the severity or frequency of symptoms of the disease. A therapeutically effective amount is commonly administered in a dosing regimen that may comprise multiple unit doses. For any particular therapeutic agent, a therapeutically effective amount (and/or an appropriate unit dose within an effective dosing regimen) may vary, for example, depending on route of administration, on combination with other pharmaceutical agents. Also, the specific therapeutically effective amount (and/or unit dose) for any particular patient may depend upon a variety of factors including the disorder being treated and the severity of the disorder; the activity of the specific pharmaceutical agent employed; the specific composition employed; the age, body weight, general health, sex and diet of the subject; the time of administration, route of administration, and/or rate of excretion or metabolism of the specific fusion protein employed; the duration of the treatment; and like factors as is well known in the medical arts.
*Treatment*: As used herein, the term "treatment" (also "treat" or "treating") refers to any administration of a substance (e.g., provided compositions) that partially or completely alleviates, ameliorates, relieves, inhibits, delays onset of, reduces severity of, and/or reduces incidence of one or more symptoms, features, and/or causes of a particular disease, disorder, and/or condition (e.g., cancer). Such treatment may be of a subject who does not exhibit signs of the relevant disease, disorder and/or condition and/or of a subject who exhibits only early signs of the disease, disorder, and/or condition. Alternatively or additionally, such treatment may be of a subject who exhibits one or more established signs of the relevant disease, disorder and/or condition. In some embodiments, treatment may be of a subject who has been diagnosed as suffering from the relevant disease, disorder, and/or condition. In some embodiments, treatment may be of a subject known to have one or more susceptibility factors that are statistically correlated with increased risk of development of the relevant disease, disorder, and/or condition.
*Wild-type p53*: As used herein, the term "wild-type p53" refers to a p53 gene ("wild-type p53 gene") or p53 polypeptide ("wild-type p53 polypeptide") that exists in an organism and that has one or more known p53 functions, e.g., tumor repressive activity. A wide variety of p53 sequences from flies, vertebrates, and mammals are known in the art, such as those described herein. In some embodiments, a human wild-type p53 gene has or includes the nucleotide sequence of SEQ ID NO:1, and a human wild type p53 polypeptide has or includes the amino acid sequence of SEQ ID NO:2.

### DETAILED DESCRIPTION

The present disclosure encompasses the surprising discovery that tumor metastasis, e.g., metastasis of a tumor expressing a mutant p53 polypeptide, can be modulated using PDGFRb modulators (e.g., PDGFRb activators or inhibitors). Accordingly, the disclosure provides, among other things, use of PDGFRb as a diagnostic, prognostic or therapeutic target for treating cancer, e.g., a tumor expressing a mutant p53 polypeptide.

### Wild-type and Mutant p53

Mutations in the p53 tumor suppressor gene represent the most common genetic lesions in cancer (Vogelstein et al., 2000). Functional studies indicate that wild-type p53 possesses a series of anti-proliferative activities that limit the proliferation and survival of pre-malignant cells. p53 exerts these activities, at least in part, through its ability to bind DNA in a sequence-specific manner to regulate gene expression, and the vast majority of mutations that occur in human tumors disable this property of p53 and, consequently, its anti-proliferative effects.

p53 mutations typically occur within the DNA-binding region and involve either DNA contact residues or residues important for conformational structure, both resulting in loss of DNA binding (Rolley et al., 1995). Because p53 functions as a tetrameric transcription factor (Wang et al., 1994), mono-allelic p53 mutations can exert dominant-negative effects on a coexpressed wild-type p53 protein. p53 activates E3 ubiquitin ligases that feed back to trigger p53 destruction and its rapid turn over; however, p53 missense mutants defective in regulating gene expression lead to the stable accumulation of the variant proteins (Oren et al., 2010). Interestingly, genetically engineered mice harboring common p53 point mutations develop more aggressive and metastatic tumors compared to those arising in their p53 heterozygous or null counterparts (Lui et al., 2000; Lang et al., 2004; Olive et al., 2004), suggesting that the mutant forms of p53 exert gain-of-function activities independent of their effects on wild-type p53. Thus, not only is inactivation of p53 activity closely related with development of cancerous states, but p53 mutations occurring in some cancers can lead to increased expression of mutant p53 proteins that actively promote cancer. This gain of function may be due to ability of p53 mutants to enhance cellular invasion and to promote metastasis. Human tumors with mutant *p53* are associated with poor patient prognosis (Sorlie et al., 2001; Soussi and Beroud, 2001) and drug resistance (Masciarelli et al., 2013). In addition, mutant p53 proteins have been identified as involved in integrin recycling (Muller et al., 2009), the mevalonate pathway (Freed-Pastor et al., 2012) or miRNA biogenesis (Su et al., 2010).

Pancreatic ductal adenocarcinoma (PDAC) is one cancer type in which mutant *p53* impacts disease progression. PDAC arises from indolent pancreatic intraductal adenomas (PanlNs) that frequently go undetected and persist for many years. However, the conversion of PanlNs to highly aggressive, frankly invasive and metastatic PDACs, in which *p53* is mutated in 75% of cases, carries a dire prognosis due to late stage detection, the presence of metastases, and ineffective treatment options (Li et al., 2004). Even those patients with a surgically approachable pancreatic lesion develop recurrent and metastatic disease after local tumor resection (Hidalgo, 2010). Consistent with a role for mutant p53 in this process, mice harboring pancreatic cancers driven by oncogenic *Kras* and a mutant *p53* allele show more metastases compared to identical mice harboring a *p53* null allele. However, it was not previously known whether mutant p53 is needed to sustain the metastatic phenotype and how it is regulated. As metastasis is a major cause of cancer mortality, and p53 mutations are common, agents that prevent or reduce metastasis by interfering with mutant p53 effects have great potential. As described herein, the present disclosure includes the discovery of the platelet-derived growth factor receptor beta (PDGFRb) as necessary and sufficient to mediate the effects of mutant p53 on invasion and metastasis.

In some embodiments, methods of the present disclosure involve identification and/or treatment of tumors having and/or expressing one or more mutant p53 genes. Wild-type and mutant p53 genes and polypeptides are known. For example, a human wild-type nucleotide sequence is disclosed as Genbank Accession No. AB082923.1 (SEQ ID NO:1), and the corresponding amino acid sequence is disclosed as Genbank Accession No. BAC16799.1 (SEQ ID NO:2). In some embodiments, a wild-type p53 polypeptide includes a proline/arginine polymorphism at amino acid residue 72 and the corresponding nucleotide polymorphism.

Record number P04637 in the UniProt database lists mutations to the amino acid sequence of a wild-type human p53. In some embodiments, a mutant p53 polypeptide contains a mutation of at least one wild-type amino acid residue selected from V143, P151, R175, G245, R248, R249, R273, P274, R280 and R282 of a wild-type p53 polypeptide described herein, e.g., of SEQ ID NO:1 (or a corresponding wild-type amino acid residue of a wild-type p53 polypeptide from another species). In some embodiments, a mutant p53 polypeptide includes at least one amino acid mutation selected from V143A, P151S, R175H, P223L, M237I, G245C, G245H, G245S, R273H, P274L and R280K and/or any combination thereof. Additional p53 mutant polypeptides are disclosed in, e.g., Bai et al., J. Cancer Mol., 2(4):141-153 (2006).

In some embodiments, a mutant p53 polypeptide includes a mutation of one or more wild-type amino acid residue selected from amino acid residues 130-290 (see, e.g., Nigro et al, Nature 342, 705-708 (1989)). In some embodiments, a mutant p53 polypeptide a mutation of one or more wild-type amino acid residue selected from amino acid residues 132-143, 174-179, 236-248, and 272-281.

In some embodiments, a mutant p53 polypeptide has at least one mutation of a wild-type amino acid residue of a p53 N-terminal domain (e.g., of approximately amino acids 1-42). In some embodiments, a mutant p53 polypeptide has at least one mutation of a wild-type amino acid residue of a p53 activation domain (e.g., of approximately amino acids 43-63). In some embodiments, a mutant p53 polypeptide has at least one mutation of a wild-type amino acid residue of a p53 protein rich domain (e.g., of approximately amino acids 64-92). In some embodiments, a mutant p53 polypeptide has at least one mutation of a wild-type amino acid residue of a p53 DNA-binding domain (e.g., of approximately amino acids 100-300). In some embodiments, a mutant p53 polypeptide has at least one mutation of a wild-type amino acid residue of a p53 nuclear localization signaling domain (e.g., of approximately amino acids 316-325). In some embodiments, a mutant p53 polypeptide has at least one mutation of a wild-type amino acid residue of a p53 homo-oligomerization domain (e.g., of approximately amino acids 307-355). In some embodiments, a mutant p53 polypeptide has at least one mutation of a wild-type amino acid residue of a p53 C-terminal domain (e.g., of approximately amino acids 356-393). In some embodiments, a mutant p53 polypeptide has a mutation of one or more wild-type amino acid residues in one or more p53 domains described herein.

In some embodiments, methods of the present disclosure involve identification and/or treatment of tumors having one or more mutant p53 nucleotide sequences, e.g., a nucleotide sequence coding for one or more mutant p53 polypeptides described herein. Additional mutant p53 nucleotide sequences are described in, e.g., Levine et al., Nature 351:453-456 (1991).

### Platelet Derived Growth Factor Receptor

Platelet-derived growth factor (PDGF) is produced by a number of cell types, including platelets (see, e.g., Bowen-Pope et al., Arterioscler Thromb Vasc Biol. 31:2397-401, doi: 10.1161/ATVBAHA.108.179556 (2011)). PDGF is a mitogen for mesenchymally-derived cells, such as blood, muscle, bone/cartilage, and connective tissue cells. At least three forms of PDGF are known. Each form consists of a 30 kDa homo- or heterodimeric combination of two genetically distinct, but structurally related, polypeptide chains which are designated A and B chains, respectively. Each chain is synthesized as a propeptide, splice variants exist for the A chain, and C-terminal proteolytic processing occurs for the B chain and perhaps the A chain.

A number of different cells express PDGF, including fibroblasts, endothelial cells, osteoblasts, platelets, vascular smooth muscle cells, macrophages and Langerhans cells, and fetal fibroblasts. PDGF is a member of a larger family of factors. In addition to PDGF, this family includes the homodimeric factors VEGF (vascular endothelial growth factor) and PIGF (placental growth factor), VEGF/PIGF heterodimers, and CTGF (connective tissue growth factor), a PDGF-like factor secreted by human vascular endothelial cells and fibroblasts.

At least two distinct human PDGF receptor transmembrane binding proteins are known, a 170 kDa, 1066 amino acid residue alpha-receptor (PDGFR alpha, or PDGFRa) and a 190 kDa, 1074 amino acid residue beta-receptor (PDGFR beta, or PDGFRb) (see, e.g., Yu et al., J. Biochem. Mol. Biol. 36:49-59 (2003)). The two receptor proteins are structurally related and consist of an extracellular portion containing five immunoglobulin-like domains, a single transmembrane region, and an intracellular portion with a protein-tyrosine kinase domain. A nucleotide sequence of a human PDGFRb is disclosed as Genbank Accession No. NM_002609.3 (SEQ ID NO:3), and translated amino acid sequence is set forth as Genbank Accession No. NP_002600.1 (SEQ ID NO:4).

A functional PDGF receptor is formed when two chains of a dimeric PDGF molecule each bind a receptor, resulting in their approximation, dimerization and activation. High-affinity binding of PDGF involves dimerization of the receptors, forming either homodimers or heterodimers with the alpha and beta receptors/chains. PDGFR alpha binds each of the three forms of PDGF dimers with high affinity. Although PDGFR beta binds both PDGF-BB and PDGF-AB with high affinity, it does not bind to PDGF-AA.

Cells known to express only alpha-receptors include oligodendroglial progenitors, liver endothelial cells and mesothelium, and platelets. Cells expressing only beta-receptors include CNS capillary endothelium, neurons and Ito (fat storing) cells of the liver, plus monocytes/macrophages. Cells showing coincident expression of alpha and beta receptors include smooth muscle cells, fibroblasts, and Schwann cells.

The PDGF receptors are receptor tyrosine kinases, and receptor binding by PDGF activates intracellular tyrosine kinase, leading to autophosphorylation of the cytoplasmic domain of the receptor as well as phosphorylation of other intracellular substrates. Specific substrates identified with the beta-receptor include Src, GTPase Activating Protein (GAP), phospholipase C gamma (PLC gamma) and phosphotidylinositol 3-phosphate. In addition, a non-tyrosine phosphorylation-associated signal transduction pathway can also be activated that involves the zinc finger protein erg-1 (early growth response gene 1).

### Diagnostic Methods

Certain methods of the present disclosure utilize the presence and/or level of expression of a mutant p53 gene as a biomarker for use of a PDGFRb inhibitor described herein. In some embodiments, a method is provided for determining the likelihood that a tumor can be effectively treated with a PDGFRb inhibitor. Such methods can include determining presence and/or level of expression and/or activity of a mutant p53 gene in a tumor sample obtained from a subject; and based on the presence and/or level of expression and/or activity of a mutant p53 gene, determining that the tumor sample has an increased or decreased probability, relative to the reference, of being effectively treated with a PDGFRb inhibitor.

In some embodiments, the level of expression and/or activity of a PDGFR, e.g., a PDGFRb, is used as a biomarker for use of a PDFRb inhibitor described herein. In some embodiments, a level of expression and/or activity of a PDGFR, e.g., a PDGFRb, in a tumor, e.g., a tumor sample, that is higher than a control indicates that the tumor can be effectively treated with a PDGFRb inhibitor. In some embodiments, a control is level of PDGFRb activity and/or expression in a corresponding non-tumor cell, tissue, or biological sample. For example, a level of expression and/or activity of a PDGFRb in a pancreatic, colorectal, or ovarian cancer tumor can be compared to the level of expression and/or activity of a PDGFRb in non-tumor pancreatic, colorectal, or ovarian cell or tissue. In some embodiments, a level of expression and/or activity of a PDGFRb in a pancreatic, colorectal, or ovarian cancer tumor that is higher relative to the level of expression and/or activity of a PDGFRb in non-tumor pancreatic, colorectal, or ovarian cell or tissue, indicates the tumor can be effectively treated with a PDGFRb inhibitor described herein. In some embodiments, if a level of expression and/or activity of a PDGFRb in a pancreatic, colorectal, or ovarian cancer tumor is higher relative to the level of expression and/or activity of a PDGFRb in non-tumor pancreatic, colorectal, or ovarian cell or tissue, the tumor is treated with a PDGFRb inhibitor described herein.

In one aspect, the disclosure provides a method of determining mutant p53 mRNA expression, polypeptide levels or activity and correlating the results obtained therein with a likelihood of effective treatment with a PDGFRb inhibitor, and/or selecting a subject for treatment with a PDGFRb inhibitor. In another aspect, the disclosure includes a method of determining PDGFRb expression and/or activity in a tumor and correlating the results with likelihood of effective treatment with a PDGFRb inhibitor, and/or selecting a subject for treatment with a PDGFRb inhibitor. Generally, methods include a step of detecting the presence, expression and/or activity of a gene encoding a mutant p53 polypeptide, and/or detecting expression and/or activity of PDGFRb. Gene products can be detected in a tumor sample and can be polypeptides or polynucleotides, e.g., mRNA.

### Nucleic acid analysis

Mutant p53 detection and/or PDGFRb detection at the nucleic acid level is encompassed by embodiments of the present disclosure. Nucleic acid analyses can be performed on genomic DNA, messenger RNAs, and/or cDNA. In many embodiments, nucleic acids are extracted from a biological sample, e.g. a tumor sample. In some embodiments, nucleic acids are analyzed without having been amplified. In some embodiments, nucleic acids are amplified using techniques known in the art (such as polymerase chain reaction (PCR)) and amplified nucleic acids are used in subsequent analyses. Multiplex PCR, in which several amplicons (e.g., from different genomic regions) are amplified at once using multiple sets of primer pairs, may be employed. (see generally, Bustin, S.A., 2000 J. Molecular Endocrinology 25:169-193.) Additional techniques include, but are not limited to, in situ hybridization, Northern blot, and various nucleic acid amplification techniques such as PCR, rtPCR, quantitative rtPCR, and the ligase chain reaction. PCR and considerations for primer design are well known in the art and are described, for example, in Newton, et al. (eds.) PCR: Essential data Series, John Wiley & Sons; PCR Primer: A Laboratory Manual, Cold Spring Harbor Laboratory Press, Cold Spring Harbor, NY, 1995; White, et al.. (eds.) PCR Protocols: Current methods and Applications, Methods in Molecular Biology, The Humana Press, Totowa, NJ, 1993.

### Polypeptide analysis

Mutant p53 polypeptides and/or PDGFRb polypeptides can be detected using any of a variety of techniques and binding agents. In certain embodiments, a binding agent is an antibody that binds specifically to a mutant p53 polypeptide. The disclosure also encompasses the use of protein arrays, including antibody arrays, for detection of a mutant p53 polypeptide and/or a PDGFRb polypeptide. The use of antibody arrays is described, for example, in Haab et al., Genome Biol. 2(2):2001 (2001). Other types of protein arrays known in the art are useful in the disclosed methods. In general, antibodies that bind specifically to mutant p53 polypeptides and/or PDGFRb polypeptides can be generated by methods well known in the art and described, for example, in Harlow, E, Lane, E, and Harlow, E, (eds.) Using Antibodies: A Laboratory Manual, Cold Spring Harbor Laboratory Press, Cold Spring Harbor, 1998. Details and references for the production of antibodies may also be found in U.S. Patent No. 6,008,337. Antibodies may include, but are not limited to, polyclonal, monoclonal, chimeric (e.g., "humanized"), single chain antibodies, Fab fragments, antibodies generated using phage display technology, etc. The invention encompasses the use of "fully human" antibodies produced using the XenoMouse™ technology (AbGenix Corp., Fremont, CA) according to the techniques described in U.S. Patent No. 6,075,181. Antibody detection methods are well known in the art including, but are not limited to, enzyme-linked immunosorbent assays (ELISAs) and Western blots. Some such methods are amenable to being performed in an array format.

In addition, in certain embodiments of the disclosure, mutant p53 polypeptides and/or PDGFRb polypeptides are detected using other specific binding agents known in the art for the detection of polypeptides, such as aptamers (Aptamers, Molecular Diagnosis, Vol. 4, No. 4, 1999), reagents derived from combinatorial libraries for specific detection of proteins in complex mixtures, random peptide affinity reagents, etc. In general, any appropriate binding agent for detecting a polypeptide may be used in conjunction with the present invention, although antibodies may represent a particularly appropriate modality.

In certain embodiments of the disclosure, a single binding agent (e.g., antibody) is used whereas in other embodiments of the invention multiple binding agents, directed either against the same or against different p53 mutant polypeptides and/or PDGFRb polypeptides can be used to increase the sensitivity or specificity of the detection technique or to provide more detailed information than that provided by a single binding agent. Thus the disclosure encompasses the use of a battery of binding agents that bind to polypeptides encoded by the mutant p53 genes (and/or PDGFRb polypeptides) described herein. In general, polypeptides are detected within a tumor sample that has been obtained from a subject, e.g., a tissue sample, cell sample, cell extract, body fluid sample, etc.

In certain embodiments, binding can be detected by adding a detectable label to a binding agent. In other embodiments, binding can be detected by using a labeled secondary binding agent that associates specifically with a primary binding agent, e.g., as is well known in the art of antigen/antibody detection. A detectable label may be directly detectable or indirectly detectable, e.g., through combined action with one or more additional members of a signal producing system. Examples of directly detectable labels include radioactive, paramagnetic, fluorescent, light scattering, absorptive and colorimetric labels. Indirectly detectable labels include chemiluminescent labels, e.g., enzymes that are capable of converting a substrate to a chromogenic product such as alkaline phosphatase, horseradish peroxidase and the like.

Once a labeled binding agent has bound a mutant p53 polypeptide, the complex may be visualized or detected in a variety of ways, with the particular manner of detection being chosen based on the particular detectable label. Representative detection means include, e.g., scintillation counting, autoradiography, measurement of paramagnetism, fluorescence measurement, light absorption measurement, measurement of light scattering and the like. Depending upon the nature of the sample, appropriate detection techniques include, but are not limited to, immunohistochemistry (IHC), radioimmunoassay, ELISA, immunoblotting and fluorescence activated cell sorting (FACS). In the case where the polypeptide is to be detected in a tissue sample, e.g., a biopsy sample, IHC is a particularly appropriate detection technique.

In certain embodiments, detection techniques of the present disclosure include a negative control, which can involve applying assaying a control sample (e.g., from a normal non-cancerous tissue) so that a signal obtained thereby can be compared with a signal obtained from a tumor sample being tested. In tests in which a secondary binding agent is used to detect a primary binding agent that binds to the polypeptide of interest, an appropriate negative control can involve performing the test on a portion of the sample with the omission of the primary binding agent.

In general, results of detection techniques can be presented in any of a variety of formats. The results can be presented in a qualitative fashion. For example, a test report may indicate only whether or not a particular p53 mutant polypeptide was detected, perhaps also with an indication of the limits of detection. Results may be presented in a semi-quantitative fashion. For example, various ranges may be defined, and the ranges may be assigned a score (e.g., 0 to 3 where 0 means no binding detected and 3 means strong binding detected) that provides a certain degree of quantitative information. Such a score may reflect various factors, e.g., the number of cells in which a polypeptide is detected, the intensity of the signal (which may indicate the level of expression of a polypeptide), etc. The results may be presented in a quantitative fashion, e.g., as a percentage of cells in which a polypeptide is detected, as a protein concentration, etc.

### Detectable moieties

In certain embodiments, certain molecules (e.g., nucleic acid probes, antibodies, etc.) used in accordance with and/or provided by the disclosure comprise one or more detectable entities or moieties, i.e., such molecules are "labeled" with such entities or moieties. Nonlimiting examples of detectable moieties include, without limitation, fluorescent compounds, various enzymes, prosthetic groups, luminescent materials, bioluminescent materials, fluorescent emitting metal atoms, (e.g., europium (Eu)), radioactive isotopes (described below), quantum dots, electron-dense reagents, and haptens. The detectable moieties can be detected using various means including, but are not limited to, spectroscopic, photochemical, radiochemical, biochemical, immunochemical, or chemical means.

Nonlimiting exemplary fluorescent detectable moieties include fluorescein, fluorescein isothiocyanate, rhodamine, 5-dimethylamine-1-napthalenesulfonyl chloride, phycoerythrin, and the like. A detectable moiety can also be a detectable enzyme, such as alkaline phosphatase, horseradish peroxidase, beta-galactosidase, acetylcholinesterase, glucose oxidase and the like. When a nanopolymer is derivatized with a detectable enzyme, it can be detected by adding additional reagents that the enzyme uses to produce a detectable reaction product. For example, when the detectable moiety is horseradish peroxidase, the addition of hydrogen peroxide and diaminobenzidine leads to a detectable colored reaction product. A polypeptide can also be derivatized with a prosthetic group (e.g., streptavidin/biotin and avidin/biotin). For example, a polypeptide can be derivatized with biotin and detected through indirect measurement of avidin or streptavidin binding. Nonlimiting examples of fluorescent compounds that can be used as detectable moieties include umbelliferone, fluorescein, fluorescein isothiocyanate, rhodamine, dichlorotriazinylamine fluorescein, dansyl chloride, and phycoerythrin. Luminescent materials include, e.g., luminol, and bioluminescent materials include, e.g., luciferase, luciferin, and aequorin.

A detectable moiety can also be a radioactive isotope, such as, but not limited to, alpha-, beta-, or gamma-emitters; or beta- and gamma-emitters. Radioactive isotopes can be used in diagnostic or therapeutic applications. Such radioactive isotopes include, but are not limited to, iodine (¹³¹I or ¹²⁵I), yttrium (⁹⁰Y), lutetium (¹⁷⁷Lu), actinium (²²⁵Ac), praseodymium (¹⁴²Pr or ¹⁴³Pr), astatine (²¹¹At), rhenium (¹⁸⁶Re or ¹⁸⁷Re), bismuth (²¹²Bi or ²¹³Bi), indium (¹¹¹In), technetium (^{99m}Tc), phosphorus (³²P), rhodium (¹⁸⁸Rh), sulfur (³⁵S), carbon (¹⁴C), tritium (³H), chromium (⁵¹Cr), chlorine (³⁶Cl), cobalt (⁵⁷Co or ⁵⁸Co), iron (⁵⁹Fe), selenium (⁷⁵Se), and gallium (⁶⁷Ga).

### Detection of p53 mutations

The disclosure also encompasses the detection of mutations of a p53 gene. Mutations may include, but are not limited to, deletions, additions, substitutions, and amplification of regions of genomic DNA that include all or part of a gene. Methods for detecting such mutations are well known in the art and include direct sequencing, denaturing HPLC and combinations thereof (see, e.g., Segal, N. H. et al., 2003, Am J Pathol, 163: 691-700; Visel, A. et al., 2009, Nature, 457: 854-858). Mutations may result in overexpression or inappropriate expression of p53 (i.e., aberrant expression). Additionally or alternatively mutations may result in an overly activated gene product (e.g., polypeptide). p53 mutations may be analyzed by the exemplary methods below.

### i. Allele-specific amplification

In some embodiments, gene mutations are detected using an allele-specific amplification assay. This approach is variously referred to as PCR amplification of specific allele (PASA) (Sarkar, et al., 1990 Anal. Biochem. 186:64-68), allele-specific amplification (ASA) (Okayama, et al., 1989 J. Lab. Clin. Med. 114:105-113), allele-specific PCR (ASPCR) (Wu, et al. 1989 Proc. Natl. Acad. Sci. USA. 86:2757-2760), and amplification-refractory mutation system (ARMS) (Newton, et al., 1989 Nucleic Acids Res. 17:2503-2516). This method can be used to detect single base substitutions as well as micro deletions/insertions.

For example, for PCR-based amplification methods, amplification primers may be designed such that they can distinguish between different alleles (e.g., between a wild-type allele and a mutant allele). Thus, the presence or absence of amplification product can be used to determine whether a gene mutation is present in a given nucleic acid sample. In some embodiments, allele specific primers can be designed such that the presence of amplification product is indicative of a gene mutation. In some embodiments, allele specific primers can be designed such that the absence of amplification product is indicative of a gene mutation.

In some embodiments, two complementary reactions are used. One reaction employs a primer specific for the wild type allele ("wild-type-specific reaction") and the other reaction employs a primer for the mutant allele ("mutant-specific reaction"). The two reactions may employ a common second primer. PCR primers specific for a particular allele (e.g., the wild-type allele or mutant allele) generally perfectly match one allelic variant of the target, but are mismatched to other allelic variant (e.g., the mutant allele or wild-type allele). The mismatch may be located at/near the 3' end of the primer, leading to preferential amplification of the perfectly matched allele. Whether an amplification product can be detected from one or in both reactions indicates the absence or presence of the mutant allele. Detection of an amplification product only from the wild-type-specific reaction indicates presence of the wild-type allele only (e.g., homozygosity of the wild-type allele). Detection of an amplification product in the mutant-specific reaction only indicates presence of the mutant allele only (e.g., homozygosity of the mutant allele). Detection of amplification products from both reactions indicate (e.g., a heterozygote). As used herein, this approach will be referred to as "allele specific amplification (ASA)."

Allele-specific amplification can also be used to detect duplications, insertions, or inversions by using a primer that hybridizes partially across the junction. The extent of junction overlap can be varied to allow specific amplification.

Amplification products can be examined by methods known in the art, including by visualizing (e.g., with one or more dyes) bands of nucleic acids that have been migrated (e.g., by electrophoresis) through a gel to separate nucleic acids by size.

### ii. Allele-specific primer extension

In some embodiments, an allele-specific primer extension (ASPE) approach is used to detect gene mutations. ASPE employs allele-specific primers that can distinguish between alleles (e.g., between a mutant allele and a wild-type allele) in an extension reaction such that an extension product is obtained only in the presence of a particular allele (e.g., mutant allele or wild-type allele). Extension products may be detectable or made detectable, e.g., by employing a labeled deoxynucleotide in the extension reaction. Any of a variety of labels are compatible for use in these methods, including, but not limited to, radioactive labels, fluorescent labels, chemiluminescent labels, enzymatic labels, etc. In some embodiments, a nucleotide is labeled with an entity that can then be bound (directly or indirectly) by a detectable label, e.g., a biotin molecule that can be bound by streptavidin-conjugated fluorescent dyes. In some embodiments, reactions are done in multiplex, e.g., using many allele-specific primers in the same extension reaction.

In some embodiments, extension products are hybridized to a solid or semi-solid support, such as beads, matrix, gel, among others. For example, the extension products may be tagged with a particular nucleic acid sequence (e.g., included as part of the allele-specific primer) and the solid support may be attached to an "anti-tag" (e.g., a nucleic acid sequence complementary to the tag in the extension product). Extension products can be captured and detected on the solid support. For example, beads may be sorted and detected. One such system that can be employed in this manner is the LUMINEX™ MAP system, which can be adapted for cystic fibrosis mutation detection by TM Bioscience and is sold commercially as a universal bead array (TAG-IT™). Additional methods and reagents are described in, e.g., U.S. Patent Publication No. 2008/0138803 A1.

### iii. Single nucleotide primer extension

In some embodiments, a single nucleotide primer extension (SNuPE) assay is used, in which the primer is designed to be extended by only one nucleotide. In some such methods, the identity of the nucleotide just downstream (e.g., 3') of the 3' end of the primer is known and differs in the mutant allele as compared to the wild-type allele. SNuPE can be performed using an extension reaction in which the only one particular kind of deoxynucleotide is labeled (e.g., labeled dATP, labeled dCTP, labeled dGTP, or labeled dTTP). Thus, the presence of a detectable extension product can be used as an indication of the identity of the nucleotide at the position of interest (e.g., the position just downstream of the 3' end of the primer), and thus as an indication of the presence or absence of a mutation at that position. SNuPE can be performed as described in U.S. Pat. No. 5,888,819; U.S. Pat. No. 5,846,710; U.S. Pat. No. 6,280,947; U.S. Pat. No. 6,482,595; U.S. Pat. No. 6,503,718; U.S. Pat. No. 6,919,174; Piggee et al., Journal of Chromatography A 781 (1997), p. 367-375; Hoogendoom et al., Human Genetics (1999) 104:89-93).

In some embodiments, primer extension can be combined with mass spectrometry for accurate and fast detection of the presence or absence of a mutation. See, U.S. Pat. No. 5,885,775; U.S. Pat. No. 7,501,251. Suitable mass spectrometric format includes, but is not limited to, Matrix-Assisted Laser Desorption/Ionization, Time-of-Flight (MALDI-TOF), Electrospray (ES), IR-MALDI, Ion Cyclotron Resonance (ICR), Fourier Transform, and combinations thereof.

### iv. Oligonucleotide ligation assay

In some embodiments, an oligonucleotide ligation assay ("OLA" or "OL") is used. OLA employs two oligonucleotides that are designed to be capable of hybridizing to abutting sequences of a single strand of a target molecules. Typically, one of the oligonucleotides is biotinylated, and the other is detectably labeled, e.g., with a streptavidin-conjugated fluorescent moiety. If the precise complementary sequence is found in a target molecule, the oligonucleotides will hybridize such that their termini abut, and create a ligation substrate that can be captured and detected. See e.g., Nickerson et al. (1990) Proc. Natl. Acad. Sci. U.S.A. 87:8923-8927; Landegren et al. (1988) Science 241:1077-1080; and U.S. Pat. No. 4,998,617.

### v. Hybridization approach

In some embodiments, nucleic acids are analyzed by hybridization using one or more oligonucleotide probes specific for a region in the gene corresponding to one or more p53 mutations, and under conditions sufficiently stringent to disallow a single nucleotide mismatch. In certain embodiments, suitable nucleic acid probes can distinguish between a normal gene and a mutant gene containing one or more mutations. For example, suitable nucleic acid probes specifically bind to a normal gene but not to a mutant gene. Alternatively, nucleic acid probes specifically bind to a p53 mutant gene containing one or more mutations but not to a normal gene. Probes of the present invention include those that are capable of specifically hybridizing a mutant p53 allele containing one or more mutations. Probes of the present invention also include those that are capable of specifically hybridizing a normal allele in a particular region of the p53 gene and therefore capable of distinguishing a normal allele from a mutant p53 allele. Thus, for example, one of ordinary skill in the art could use probes of the invention to determine whether an individual is homozygous or heterozygous for a particular allele.

Nucleic acid hybridization techniques are well known in the art. See, e.g., Sambrook, et al., 1989, Molecular Cloning: A Laboratory Manual, Second Edition, Cold Spring Harbor Press, Plainview, N.Y. Those skilled in the art understand how to estimate and adjust the stringency of hybridization conditions such that sequences having at least a desired level of complementary will stably hybridize, while those having lower complementary will not. For examples of hybridization conditions and parameters, see, e.g., Sambrook, et al., 1989, Molecular Cloning: A Laboratory Manual, Second Edition, Cold Spring Harbor Press, Plainview, N.Y.; Ausubel, F. M. et al. 1994, Current Protocols in Molecular Biology. John Wiley & Sons, Secaucus, N.J.

Nucleic acid probes may comprise ribonucleic acids and/or deoxyribonucleic acids. In some embodiments, nucleic acid probes are oligonucleotides (i.e., "oligonucleotide probes"). Generally, oligonucleotide probes are long enough to bind specifically to a homologous region of the gene, but short enough such that a difference of one nucleotide between the probe and the nucleic acid sample being tested disrupts hybridization. Typically, the sizes of oligonucleotide probes vary from approximately 10 to 100 nucleotides. In some embodiments, oligonucleotide probes vary from 15 to 90, 15 to 80, 15 to 70, 15 to 60, 15 to 50, 15 to 40, 15 to 35, 15 to 30, 18 to 30, or 18 to 26 nucleotides in length. As appreciated by those of ordinary skill in the art, the optimal length of an oligonucleotide probe may depend on the particular methods and/or conditions in which the oligonucleotide probe may be employed.

In some embodiments, nucleic acid probes are useful as primers, e.g., for nucleic acid amplification and/or extension reactions. In some embodiments, nucleic acid probes are labeled with a detectable moiety as described herein.

### Therapeutic Methods

Another aspect of the disclosure makes use of therapeutic agents, e.g., PDGFRb inhibitors, to inhibit or reduce PDGFRb activity and/or levels. For example, by preventing transcription, repressing mRNA transcript levels, inhibiting translation, or otherwise directly or indirectly interfering with the activity of PDGFRb, it is possible to treat and/or inhibit tumor cells, e.g., tumor cells having and/or expressing a mutant p53 gene and/or having an increased level of activity and/or expression of PDGFRb relative to control. PDGFRb expression or activity may be modulated either directly through interaction with a PDGFRb gene, mRNA or protein itself, or indirectly through one or more upstream regulator or downstream targets of PDGFRb. Any inhibitor of a PDGFRb can be used in the methods of the disclosure, including nucleic acid, polypeptide, and small molecule inhibitors. Various PDGFR inhibitors are known and/or commercially available. For example, PDGFR inhibitors useful in connection with methods described herein include, but are not limited to, imatinib, sunitinib, axitinib, BIBF1120 (Vargatef), pazopanib, ponatinib, MK-2461, dovitinib, crenolanib, PP-121, telatinib, CP 673451, TSU-68, Ki8751, tivozanib, masitinib, motesanib, regorafenib, and MEDI-575. Additional therapeutic agents, e.g., PDGFRb inhibitors, are described below.

### Antibodies

The present disclosure encompass the use of antibodies in the treatment of mutant p53-mediated cancers, e.g., pancreatic cancer. Such antibodies include, but are not limited to, polyclonal antibodies; monoclonal antibodies or antigen binding fragments thereof; modified antibodies such as chimeric antibodies, reshaped antibodies, humanized antibodies, or fragments thereof (e.g., Fv, Fab', Fab, F(ab')₂); or biosynthetic antibodies, e.g., single chain antibodies, single domain antibodies (DAB), Fvs, or single chain Fvs (scFv). Methods of making and using polyclonal and monoclonal antibodies are well known in the art, e.g., in Harlow et al., Using Antibodies: A Laboratory Manual: Portable Protocol I. Cold Spring Harbor Laboratory (Dec. 1, 1998). Methods for making modified antibodies and antibody fragments (e.g., chimeric antibodies, reshaped antibodies, humanized antibodies, or fragments thereof, e.g., Fab', Fab, F(ab')₂ fragments); or biosynthetic antibodies (e.g., single chain antibodies, single domain antibodies (DABs), Fv, single chain Fv (scFv), and the like), are known in the art and can be found, e.g., in Zola, Monoclonal Antibodies: Preparation and Use of Monoclonal Antibodies and Engineered Antibody Derivatives, Springer Verlag (Dec. 15, 2000; 1st edition).

### Aptamers

Aptamers are macromolecules composed of nucleic acid (e.g., RNA, DNA) that bind tightly to a specific molecular target (e.g., PDGFRb polypeptide or an epitope thereof). A particular aptamer may be described by a linear nucleotide sequence and is typically about 15-60 nucleotides in length. In some embodiments, aptamers are modified to dramatically reduce their sensitivity to degradation by enzymes in the blood for use in in vivo applications. In addition, aptamers can be modified to alter their biodistribution or plasma residence time.

Selection of aptamers that can bind PDGFRb or a fragment thereof can be achieved through methods known in the art. For example, aptamers can be selected using the SELEX (Systematic Evolution of Ligands by Exponential Enrichment) method (Tuerk, C., and Gold, L., Science 249:505-510 (1990); Jayasena, S. D. Clin. Chem. 45:1628-1650 (1999)).

### Antisense and ribozymes

Other agents that are useful in the methods described herein are nucleic acids, including antisense molecules or catalytic nucleic acid molecules (e.g., ribozymes) that specifically hybridize mRNA encoding a target polypeptide. An antisense construct includes the reverse complement of at least part of the cDNA coding sequence or mRNA of a target polypeptide, the target polypeptide cDNA, or gene sequence or flanking regions thereof, and thus it can hybridize to the mRNA.

The introduced sequence need not be the full-length cDNA or gene or reverse complement thereof, and need not be exactly homologous to the equivalent sequence found in the cell type to be transformed. Antisense molecules can be made using known techniques in the art (see, e.g., Agrawal, Methods in Molecular Biology, Humana Press Inc., 1993, Vol. 20 ("Protocols for Oligonucleotides and Analogs")).

The antisense molecule may be conjugated to another molecule, e.g., a peptide, hybridization triggered cross-linking agent, transport agent, aptamer, or hybridization-triggered cleavage agent. A targeting moiety can also be included that enhances uptake of the molecule by cells. The targeting moiety can be a specific binding molecule, such as an antibody or fragment thereof that recognizes a molecule present on the surface of the cell.

Alternatively, the agent is a catalytic nucleic acid, such as a ribozyme (a synthetic RNA molecule that possesses highly specific endoribonuclease activity). The production and use of ribozymes are disclosed in, e.g., U.S. Pat. No. 4,987,071 and U.S. Pat. No. 5,543,508. Ribozymes can be synthesized and administered to a cell or a subject, or can be encoded on an expression vector, from which the ribozyme is synthesized in the targeted cell (see, e.g., WO 9523225, and Beigelman et al., Nucl. Acids Res. 23:4434-42, 1995). Examples of oligonucleotides with catalytic activity are described in, e.g., WO 9506764 and WO 9011364, and Sarver et al., Science 247:1222-1225, 1990. The inclusion of ribozyme sequences within antisense RNAs can be used to confer RNA cleaving activity on the antisense RNA, such that endogenous mRNA molecules that bind to the antisense RNA are cleaved, which, in turn, leads to an enhanced antisense inhibition of endogenous gene expression.

### RNA interference

Double-stranded nucleic acid molecules that can silence a gene encoding a target polypeptide can also be used as agents in the methods described herein. RNA interference (RNAi) is a mechanism of post-transcriptional gene silencing in which double-stranded RNA (dsRNA) corresponding to a gene (or coding region) of interest is introduced into a cell or an organism, resulting in degradation of the corresponding mRNA. The RNAi effect persists for multiple cell divisions before gene expression is regained. RNAi is therefore an effective method for making targeted knockouts or "knockdowns" at the RNA level. RNAi has proven successful in human cells, including human embryonic kidney and HeLa cells (see, e.g., Elbashir et al., Nature 411:494-498, 2001). For example, gene silencing can be induced in mammalian cells by the endogenous expression of RNA hairpins (see Paddison et al., PNAS (USA) 99:1443-1448, 2002). In another instances, transfection of small (21-23 nt) dsRNA specifically inhibits gene expression (reviewed in Caplen, Trends Biotechnol. 20:49-51, 2002).

Briefly, RNAi is thought to work as follows. miRNA, pre-miRNA, pri-miRNA, or dsRNA corresponding to a portion of a gene to be silenced is introduced into a cell. The dsRNA is digested into 21-23 nucleotide siRNAs, or short interfering RNAs. The siRNA duplexes bind to a nuclease complex to form what is known as the RNA-induced silencing complex, or RISC. The RISC targets the homologous transcript by base pairing interactions between one of the siRNA strands and the endogenous mRNA. It then cleaves the mRNA approximately 12 nucleotides from the 3' terminus of the siRNA (reviewed in Sharp et al., Genes Dev. 15: 485-490, 2001; and Hammond et al., Nature Rev. Gen. 2: 110-119, 2001).

RNAi technology in gene silencing utilizes standard molecular biology methods. dsRNA corresponding to the sequence from a target gene to be inactivated can be produced by standard methods, e.g., by simultaneous transcription of both strands of a template DNA (corresponding to the target sequence) with T7 RNA polymerase. Kits for production of dsRNA for use in RNAi are available commercially, e.g., from New England Biolabs, Inc. Methods of transfection of dsRNA or plasmids engineered to make dsRNA are routine in the art.

Gene silencing effects similar to those of RNAi have been reported in mammalian cells with transfection of a mRNA-cDNA hybrid construct (Lin et al., Biochem. Biophys. Res. Commun. 281:639-644, 2001), and can be used as another method for gene silencing. Therapeutic applications of RNAi are described, e.g., in Shuey, Drug Discov. Today 7:1040-1046, 2002.

In addition, siRNA may also be delivered by a gene therapy approach, e.g., using a DNA vector from which siRNA compounds in, e.g., small hairpin form (shRNA), can be transcribed directly. Numerous studies have demonstrated that while double-stranded siRNAs are very effective at mediating RNAi, short, single-stranded, hairpin-shaped RNAs can also mediate RNAi, presumably because they fold into intramolecular duplexes that are processed into double-stranded siRNAs by cellular enzymes (see, e.g., Sui et al., Proc Natl Acad Sci USA, 99:5515-5520 (2002); Yu et al., Proc Natl Acad Sci USA, 99:6047-6052 (2002); Paul et al., Nature Biotech., 20:505-508 (2002); Hannon, Nature. 418:244-251 (2002); Shi, Trends Genet, 19:9-12 (2003); see also Xia et al., Nature Biotech., 20:1006-1010 (2002)).

### Small molecules

Small molecule modulators (e.g., inhibitors or activators) of gene expression are also within the scope of the invention and may be detected by screening libraries of compounds using, for example, cell lines that express a PDGFRb polypeptide or a version of a PDGFRb polypeptide that has been modified to include a readily detectable moiety. Methods for identifying compounds capable of modulating gene expression are described, for example, in U.S. Patent No. 5,976,793.

### Therapeutic Administration

The route and/or mode of administration of a therapeutic agent described herein can vary depending upon the desired results. Dosage regimens can be adjusted to provide the desired response, e.g., a therapeutic response.

Methods of administration include, but are not limited to, intradermal, intramuscular, intraperitoneal, intravenous, subcutaneous, intranasal, epidural, oral, sublingual, intracerebral, intravaginal, transdermal, rectal, by inhalation, or topical, particularly to the ears, nose, eyes, or skin. The mode of administration is left to the discretion of the practitioner.

In some instances, a therapeutic agent described herein is administered locally. This is achieved, for example, by local infusion during surgery, topical application (e.g., in a cream or lotion), by injection, by means of a catheter, by means of a suppository or enema, or by means of an implant, said implant being of a porous, non-porous, or gelatinous material, including membranes, such as sialastic membranes, or fibers. In some situations, a therapeutic agent described herein is introduced into the central nervous system, circulatory system or gastrointestinal tract by any suitable route, including intraventricular, intrathecal injection, paraspinal injection, epidural injection, enema, and by injection adjacent to the peripheral nerve. Intraventricular injection can be facilitated by an intraventricular catheter, for example, attached to a reservoir, such as an Ommaya reservoir.

This disclosure also features a device for administering a therapeutic agent described herein. The device can include, e.g., one or more housings for storing pharmaceutical compositions, and can be configured to deliver unit doses of a therapeutic agent described herein.

Pulmonary administration can also be employed, e.g., by use of an inhaler or nebulizer, and formulation with an aerosolizing agent, or via perfusion in a fluorocarbon or synthetic pulmonary surfactant.

In some instances, a therapeutic agent described herein can be delivered in a vesicle, in particular, a liposome (see Langer, Science 249:1527-1533 (1990) and Treat et al., Liposomes in the Therapy of Infectious Disease and Cancer, pp. 317-327 and pp. 353-365 (1989)).

In yet other situations, a therapeutic agent described herein can be delivered in a controlled-release system or sustained-release system (see, e.g., Goodson, in Medical Applications of Controlled Release, 2:115-138 (1984)). Other controlled or sustained-release systems discussed in the review by Langer, Science 249:1527-1533 (1990) can be used. In one case, a pump can be used (Langer, Science 249:1527-1533 (1990); Sefton, CRC Crit. Ref Biomed. Eng. 14:201 (1987); Buchwald et al., Surgery 88:507 (1980); and Saudek et al., N. Engl. J. Med. 321:574 (1989)). In yet other situations, a controlled- or sustained-release system can be placed in proximity of a target of a therapeutic agent described herein, reducing the dose to a fraction of the systemic dose.

A therapeutic agent described herein can be formulated as a pharmaceutical composition that includes a suitable amount of a physiologically acceptable excipient (see, e.g., Remington's Pharmaceutical Sciences pp. 1447-1676 (Alfonso R. Gennaro, ed., 19th ed. 1995)). Such physiologically acceptable excipients can be, e.g., liquids, such as water and oils, including those of petroleum, animal, vegetable, or synthetic origin, such as peanut oil, soybean oil, mineral oil, sesame oil and the like. The physiologically acceptable excipients can be saline, gum acacia, gelatin, starch paste, talc, keratin, colloidal silica, urea and the like. In addition, auxiliary, stabilizing, thickening, lubricating, and coloring agents can be used. In one situation, the physiologically acceptable excipients are sterile when administered to an animal. The physiologically acceptable excipient should be stable under the conditions of manufacture and storage and should be preserved against the contaminating action of microorganisms. Water is a particularly useful excipient when a nanoparticle described herein is administered intravenously. Saline solutions and aqueous dextrose and glycerol solutions can also be employed as liquid excipients, particularly for injectable solutions. Suitable physiologically acceptable excipients also include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. Other examples of suitable physiologically acceptable excipients are described in Remington's Pharmaceutical Sciences pp. 1447-1676 (Alfonso R. Gennaro, ed., 19th ed. 1995). The pharmaceutical compositions, if desired, can also contain minor amounts of wetting or emulsifying agents, or pH buffering agents.

Liquid carriers can be used in preparing solutions, suspensions, emulsions, syrups, and elixirs. A therapeutic agent described herein can be suspended in a pharmaceutically acceptable liquid carrier such as water, an organic solvent, a mixture of both, or pharmaceutically acceptable oils or fat. The liquid carrier can contain other suitable pharmaceutical additives including solubilizers, emulsifiers, buffers, preservatives, sweeteners, flavoring agents, suspending agents, thickening agents, colors, viscosity regulators, stabilizers, or osmo-regulators. Suitable examples of liquid carriers for oral and parenteral administration include water (particular containing additives described herein, e.g., cellulose derivatives, including sodium carboxymethyl cellulose solution), alcohols (including monohydric alcohols and polyhydric alcohols, e.g., glycols) and their derivatives, and oils (e.g., fractionated coconut oil and arachis oil). For parenteral administration the carrier can also be an oily ester such as ethyl oleate and isopropyl myristate. The liquid carriers can be in sterile liquid form for administration. The liquid carrier for pressurized compositions can be halogenated hydrocarbon or other pharmaceutically acceptable propellant.

In other instances, a therapeutic agent described herein is formulated for intravenous administration. Compositions for intravenous administration can comprise a sterile isotonic aqueous buffer. The compositions can also include a solubilizing agent. Compositions for intravenous administration can optionally include a local anesthetic such as lignocaine to lessen pain at the site of the injection. The ingredients can be supplied either separately or mixed together in unit dosage form, for example, as a dry lyophilized powder or water-free concentrate in a hermetically sealed container such as an ampule or sachette indicating the quantity of active agent. Where a therapeutic agent described herein is administered by infusion, it can be dispensed, for example, with an infusion bottle containing sterile pharmaceutical grade water or saline. Where a therapeutic agent described herein is administered by injection, an ampule of sterile water for injection or saline can be provided so that the ingredients can be mixed prior to administration.

In other circumstances, a therapeutic agent described herein can be administered across the surface of the body and the inner linings of the bodily passages, including epithelial and mucosal tissues. Such administrations can be carried out using a therapeutic agent described herein in lotions, creams, foams, patches, suspensions, solutions, and suppositories (e.g., rectal or vaginal). In some instances, a transdermal patch can be used that contains a therapeutic agent described herein and a carrier that is inert to the therapeutic agent described herein, is non-toxic to the skin, and that allows delivery of the agent for systemic absorption into the blood stream via the skin. The carrier can take any number of forms such as creams or ointments, pastes, gels, or occlusive devices. The creams or ointments can be viscous liquid or semisolid emulsions of either the oil-in-water or water-in-oil type. Pastes of absorptive powders dispersed in petroleum or hydrophilic petroleum containing a therapeutic agent described herein can also be used. A variety of occlusive devices can be used to release a therapeutic agent described herein into the blood stream, such as a semi-permeable membrane covering a reservoir containing the therapeutic agent described herein with or without a carrier, or a matrix containing the therapeutic agent described herein.

A therapeutic agent described herein can be administered rectally or vaginally in the form of a conventional suppository. Suppository formulations can be made using methods known to those in the art from traditional materials, including cocoa butter, with or without the addition of waxes to alter the suppository's melting point, and glycerin. Water-soluble suppository bases, such as polyethylene glycols of various molecular weights, can also be used.

The amount of a therapeutic agent described herein that is effective for treating disorder or disease is determined using standard clinical techniques known to those with skill in the art. In addition, in vitro or in vivo assays can optionally be employed to help identify optimal dosage ranges. The precise dose to be employed can also depend on the route of administration, the condition, the seriousness of the condition being treated, as well as various physical factors related to the individual being treated, and can be decided according to the judgment of a health-care practitioner. For example, the dose of a therapeutic agent described herein can each range from about 0.001 mg/kg to about 250 mg/kg of body weight per day, from about 1 mg/kg to about 250 mg/kg body weight per day, from about 1 mg/kg to about 50 mg/kg body weight per day, or from about 1 mg/kg to about 20 mg/kg of body weight per day. Equivalent dosages can be administered over various time periods including, but not limited to, about every 2 hr, about every 6 hr, about every 8 hr, about every 12 hr, about every 24 hr, about every 36 hr, about every 48 hr, about every 72 hr, about every week, about every 2 weeks, about every 3 weeks, about every month, and about every 2 months. The number and frequency of dosages corresponding to a completed course of therapy can be determined according to the judgment of a health-care practitioner.

In some instances, a pharmaceutical composition described herein is in unit dosage form, e.g., as a tablet, capsule, powder, solution, suspension, emulsion, granule, or suppository. In such form, the pharmaceutical composition can be sub-divided into unit doses containing appropriate quantities of a therapeutic agent described herein. The unit dosage form can be a packaged pharmaceutical composition, for example, packeted powders, vials, ampoules, pre-filled syringes or sachets containing liquids. The unit dosage form can be, for example, a capsule or tablet itself, or it can be the appropriate number of any such compositions in package form. Such unit dosage form can contain from about 1 mg/kg to about 250 mg/kg, and can be given in a single dose or in two or more divided doses.

### Kits

Another aspect of the disclosure provides a kit to test for the presence of a mutant p53, e.g., in a tumor sample. The kit can comprise, for example, an antibody for detection of a polypeptide or a probe for detection of a polynucleotide. In addition, the kit can include a reference or control sample, instructions for processing samples, performing the test and interpreting the results, buffers and other reagents necessary for performing the test. In one embodiment the kit comprises one or more PDGFRb inhibitors described herein. In certain embodiments, the kit includes instructions for administering one or more PDGFRb inhibitors if a mutant p53 is detected in the tumor sample.

### Cancers

The disclosure includes treatment methods for various cancers, including, but not limited to, solid tumors and blood born tumors. In some embodiments, the cancer is a cancer of skin tissues, organs, bone, cartilage, blood and vessels. In some embodiments, the cancer is a cancer of the head, neck, eye, mouth, throat, esophagus, chest, bone, lung, colon, rectum, stomach, prostate, breast, ovaries, kidney, liver, pancreas and brain. In some embodiments, a cancer encompasses primary and/or metastatic cancers.

In some embodiments, methods described herein are used to treat and/or prevent pancreatic cancer, e.g., treat and/or prevent metastasis of a pancreatic tumor, e.g., a pancreatic tumor having or expressing a mutant p53 gene. In some embodiments, the pancreatic cancer is exocrine pancreatic cancer or endocrine pancreatic cancer. Exocrine pancreatic cancer includes, but not limited to, adenocarcinomas, acinar cell carcinomas, adenosquamous carcinomas, colloid carcinomas, undifferentiated carcinomas with osteoclast-like giant cells, hepatoid carcinomas, intraductal papillary-mucinous neoplasms, mucinous cystic neoplasms, pancreatoblastomas, serous cystadenomas, signet ring cell carcinomas, solid and pseuodpapillary tumors, pancreatic ductal carcinomas, and undifferentiated carcinomas. In some embodiments, the exocrine pancreatic cancer is pancreatic ductal carcinoma. Endocrine pancreatic cancer includes, but is not limited to, insulinomas and glucagonomas.

In some embodiments, the pancreatic cancer is early stage pancreatic cancer, non-metastatic pancreatic cancer, primary pancreatic cancer, advanced pancreatic cancer, locally advanced pancreatic cancer, metastatic pancreatic cancer, unresectable pancreatic cancer, pancreatic cancer in remission, or recurrent pancreatic cancer. In some embodiments, the pancreatic cancer is resistant to one or more known therapies. In some embodiments, the pancreatic cancer is resectable (i.e., tumors that are confined to a portion of the pancreas or has spread just beyond it that allows for complete surgical removal), or locally advanced (unresectable) (i.e., the localized tumors may be unresectable because of local vessel impingement or invasion by tumor). In some embodiments, the pancreatic cancer is, according to American Joint Committee on Cancer (AJCC) TNM classifications, a stage 0 tumor (the tumor is confined to the top layers of pancreatic duct cells and has not invaded deeper tissues, and it has not spread outside of the pancreas (e.g., pancreatic carcinoma in situ or pancreatic intraepithelial neoplasia III), a stage IA tumor (the tumor is confined to the pancreas and is less than 2 cm in size, and it has not spread to nearby lymph nodes or distinct sites), a stage IB tumor (the tumor is confined to the pancreas and is larger than 2 cm in size, and it has not spread to nearby lymph nodes or distant sites), a stage IIA tumor (the tumor is growing outside the pancreas but not into large blood vessels, and it has not spread to nearby lymph nodes or distant sites), stage IIB (the tumor is either confined to the pancreas or growing outside the pancreas but not into nearby large blood vessels or major nerves, and it has spread to nearby lymph nodes but not distant sites), stage III (the tumor is growing outside the pancreas into nearby large blood vessels or major nerves, and it may or may not have spread to nearby lymph nodes) or stage IV tumor (the cancer has spread to distant sites).

The methods provided herein can be used to treat a subject who has been diagnosed with pancreatic cancer and has been treated with a prior therapy (e.g., gemcitabine-based, erlotinib-based, or 5-fluorouracil-based therapy). In some embodiments, a subject is a human. In some embodiments, a human subject is at least about 30, 35, 40, 45, 50, 55, 60, 65, 70, 75, 80, or 85 years old. In some embodiments, a subject has a family history of pancreatic cancer (e.g., at least 2 first-degree relatives affected with pancreatic cancer without accumulation of other cancers or familial diseases). In some embodiments, a subject has one or more hereditary pancreatic cancer syndromes, including, but not limited to, BRCA2 mutation, familial atypical multiple mole melanoma (FAMMM), peutz-jeghers syndrome, and hereditary pancreatitis. In some embodiments, a subject is a long-time smoker (e.g., more than 10, 15, or 20 years). In some embodiments, a subject has ECOG/WHO/Zubrod score of 0 (asymptomatic), 1 (symptomatic but completely ambulatory), 2 (symptomatic, <50% in bed during the day), 3 (symptomatic, >50% in bed, but not bedbound), or 4 (bedbound). In some embodiments, a subject has a single lesion at presentation. In some embodiments, a subject has multiple lesions at presentation. In some embodiments, a subject is a human who exhibits one or more symptoms associated with pancreatic cancer.

The methods described herein are useful for various aspects of pancreatic cancer treatment. In some embodiments, the disclosure provides methods of inhibiting pancreatic cancer cell proliferation (such as pancreatic cancer tumor growth) in a subject, comprising administering to the subject an effective amount of a PDGFRb inhibitor described herein. In some embodiments, at least about 10%, 20%, 30%, 40%, 60%, 70%, 80%, 90%, or 100% cell proliferation is inhibited.

In some embodiments, the disclosure provides methods of inhibiting tumor metastasis, e.g., pancreatic cancer tumor metastasis, in a subject, comprising administering to the subject an effective amount of a PDGFRb inhibitor described herein. In some embodiments, at least about 10%, 20%, 30%, 40%, 60%, 70%, 80%, 90%, or 100% metastasis is inhibited. In some embodiments, a method of inhibiting metastasis to one or more lymph nodes is provided.

In some embodiments, the disclosure provides methods of reducing (such as eradiating) pre-existing tumor metastasis, e.g., pancreatic cancer tumor metastasis (such as metastasis to the lymph node) in a subject, comprising administering to the subject an effective amount of a PDGFRb inhibitor described herein. In some embodiments, metastasis is reduced by at least about 10%, 20%, 30%, 40%, 60%, 70%, 80%, 90%, or 100%.

In some embodiments, the disclosure provides methods of reducing tumor size, e.g., pancreatic cancer tumor size, in a subject, comprising administering to the individual an effective amount of a PDGFRb inhibitor described herein. In some embodiments, tumor size is reduced at least about 10%, 20%, 30%, 40%, 60%, 70%, 80%, 90%, or 100%. In some embodiments, the subject has non-metastatic pancreatic cancer. In some embodiments, the subject has primary pancreatic cancer.

In some embodiments, the disclosure provides methods of prolonging time to disease progression of cancer, e.g., pancreatic cancer (e.g., progression-free survival) in a subject, comprising administering to the subject an effective amount of a PDGFRb inhibitor described herein. In some embodiments, administration of a PDGFRb inhibitor prolongs the time to disease progression by at least 1, 2, 3, 4, 5, 6, 7, 8, 9, 10, 11, 12 weeks. In some embodiments, administration of a PDGFRb inhibitor prolongs the time to disease progression by at least 1.0, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0, 10.2, 10.4, 10.6, 10.8, 11.0, 11.2, 11.4, 11.6, 11.8, 12.0, 13, 14, 15, 16, 17, 18, 19, 20, 21, 22, 23, 24, 30, 36, 42, 48, 54, 60, 66, or 72 months.

In some embodiments, the disclosure provides methods of prolonging overall survival of a subject having cancer, e.g., pancreatic cancer, comprising administering to the subject an effective amount of a PDGFRb inhibitor described herein. In some embodiments, administration of a PDGFRb inhibitor prolongs the survival of the subject by at least 1.0, 1.2, 1.4, 1.6, 1.8, 2.0, 2.2, 2.4, 2.6, 2.8, 3.0, 3.2, 3.4, 3.6, 3.8, 4.0, 4.2, 4.4, 4.6, 4.8, 5.0, 5.2, 5.4, 5.6, 5.8, 6.0, 6.2, 6.4, 6.6, 6.8, 7.0, 7.2, 7.4, 7.6, 7.8, 8.0, 8.2, 8.4, 8.6, 8.8, 9.0, 9.2, 9.4, 9.6, 9.8, 10.0, 10.2, 10.4, 10.6, 10.8, 11.0, 11.2, 11.4, 11.6, 11.8, 12.0, 13, 14, 15, 16, 17, 18, 19, 20, 21, 22, 23, 24, 30, 36, 42, 48, 54, 60, 66, or 72 months.

In some embodiments, the disclosure provides methods of disrupting (such as destroying) pancreatic cancer stroma of a subject having pancreatic cancer, comprising administering to the subject an effective amount of a PDGFRb inhibitor described herein. In some embodiments, the pancreatic cancer stroma is disrupted or destroyed by at least about 10%, 20%, 30%, 40%, 60%, 70%, 80%, 90%, or 100%. In some embodiments, the subject has non-metastatic pancreatic cancer. In some embodiments, the subject has primary pancreatic cancer.

In some embodiments, the disclosure provides methods of improving one or more clinical benefits of a subject having cancer, e.g., pancreatic cancer, comprising administering to the subject an effective amount of a PDGFRb inhibitor described herein. Clinical benefits include, but are not limited to, improved/better quality of life, improved/better symptom control of cancer, e.g., pancreatic cancer, and increased weight gain.

### Combination Therapy

In some embodiments, a PDGFRb inhibitor described herein is administered to a subject in combination with one or more additional therapies, e.g., to treat a tumor and/or one or more symptoms of a tumor. For example, a PDGFRb inhibitor can be administered in combination with one or more additional anticancer ingredients or drugs. Such anticancer ingredients include known anticancer therapies including, but are not limited to, an antifolate, a 5-fluoropyrimidine (including 5-fluorouracil), a cytidine analogue such as β-L-1,3-dioxolanyl cytidine or β-L-1,3-dioxolanyl 5-fluorocytidine, an antimetabolite (including purine antimetabolites, cytarabine, fudarabine, floxuridine, 6-mercaptopurine, methotrexate, and 6-thioguanine), hydroxyurea, a mitotic inhibitor (including CPT-11, Etoposide (VP-21), taxol, and vinca alkaloids such as vincristine and vinblastine), an alkylating agent (including but not limited to busulfan, chlorambucil, cyclophosphamide, ifofamide, mechlorethamine, melphalan, and thiotepa), a nonclassical akylating agent, a platinum containing compound, bleomycin, an anti-tumor antibiotic, an anthracycline such as doxorubicin and dannomycin, an anthracenedione, a topoisomerase II inhibitor, a hormonal agent (including but not limited to a corticosteriod (dexamethasone, prednisone, and methylprednisone), an androgen such as fluoxymesterone and methyltestosterone), an estrogen such as diethylstilbesterol, an antiestrogen such as tamoxifen, an LHRH analogue such as leuprolide, an antiandrogen such as flutamdie, aminogluetethimide, megestrol acetate, and medroxyprogesterone, asparaginase, carmustine, lomustine, hexamethyl-melamine, dacarbazine, mitotane, streptozocin, cisplatin, carboplatin, levamasole, and leucovorin. The compounds of the present invention can also be used in combination with an enzyme therapy agent and/or immune system modulator such as an interferon, interleukin, tumor necrosis factor, macrophage colony-stimulating factor and colony stimulating factor. In some embodiments, a PDGFRb inhibitor is administered in combination with one or more of gemcitabine (e.g., GEMZAR®), erlotinib (e.g., TARCEVA®), gefitinib, a taxane (e.g., paclitaxel, docetaxel), capecitabine, mitomycin and/or irinotecan.

In some embodiments, combined administration of a PDGFRb inhibitor and a second agent results in an improvement in a disease or disorder described herein or a symptom thereof to an extent that is greater than one produced by either the PDGFRb inhibitor or the second agent alone. The difference between the combined effect and the effect of each agent alone can be a statistically significant difference.

In some embodiments, combined administration of a PDGFRb inhibitor and a second agent allows administration of the second agent at a reduced dose, at a reduced number of doses, and/or at a reduced frequency of dosage compared to a standard dosing regimen approved for the second agent.

### Identifying Modulators of PDGFRb Expression or Activity

PDGFRb described herein are useful for identifying agents that can be potentially used to treat a disorder described herein, e.g., a cancer. For example, an agent that decreases expression or activity of a PDGFRb can be identified as an agent that can be used to treat cancer. Numerous methods exist for evaluating whether an agent alters a PDGFRb polypeptide expression activity or level. In one embodiment, the ability of a test agent to modulate (e.g., increase or decrease) (e.g., permanently or temporarily) expression from a PDGFRb polynucleotide promoter is evaluated by e.g., routine reporter (e.g., LacZ, luciferase, or GFP) transcription assay. For example, a cell or transgenic animal whose genome comprises a reporter gene operably linked to a PDGFRb polynucleotide promoter, can be contacted with a test agent, and the ability of the test agent to increase or decrease reporter activity is indicative of the ability of the agent to modulate a PDGFRb polypeptide.

In some embodiments, effects of a test agent on a PDGFRb polypeptide expression activity or level can be evaluated in a cell, cell lysate, or subject, preferably a non-human experimental mammal, and more preferably a rodent (e.g., a rat, mouse, rabbit), or explant thereof. Methods of assessing a PDGFRb polypeptide expression are well known in the art, e.g., Northern analysis, ribonuclease protection assay, reverse transcription-polymerase chain reaction (RT-PCR) or RNA in situ hybridization (see, e.g., Sambrook et al. Molecular Cloning: A Laboratory Manual (3rd ed. 2001)). The level of a PDGFRb polypeptide can be monitored by, e.g., Western analysis, immunoassay, or in situ hybridization. In some embodiments, a DNA construct encoding a PDGFRb polypeptide/GFP fusion protein is transfected into cells, and level of GFP fluorescence in the presence or absence of a test agent is determined. A decrease in fluorescence in the presence of the test agent is indicative of the ability of the test agent to decrease a PDGFRb polypeptide level.

Agents and test agents to be used in the methods described herein include crude or partially or substantially purified extracts of organic sources, e.g., botanical (e.g., herbal) and algal extracts, inorganic elements or compounds, as well as partially or substantially purified or synthetic agents, e.g., small molecules, polypeptides, antibodies, and polynucleotides, and libraries of these.

In one example, combinatorial chemical libraries can be produced or obtained that sample chemical compounds that are structurally or chemically related or unrelated. Preparation and screening of combinatorial chemical libraries is well known to those of skill in the art. Such combinatorial chemical libraries include, but are not limited to, peptide libraries (see, e.g., U.S. Pat. No. 5,010,175; Furka, Int. J. Pept. Prot. Res. 37:487-493 (1991); and Houghton et al., Nature 354:84-88 (1991)). Other chemistries for generating chemical diversity libraries can also be used. Such chemistries include, but are not limited to: peptoids (e.g., PCT Publication No. WO 91/19735), encoded peptides (e.g., PCT Publication No. WO 93/20242), random bio-oligomers (e.g., PCT Publication No. WO 92/00091), benzodiazepines (e.g., U.S. Pat. No. 5,288,514), diversomers such as hydantoins, benzodiazepines and dipeptides (Hobbs et al., Proc. Nat. Acad. Sci. USA 90:6909-6913 (1993)), vinylogous polypeptides (Hagihara et al., J. Amer. Chem. Soc. 114:6568 (1992)), nonpeptidal peptidomimetics with glucose scaffolding (Hirschmann et al., J. Amer. Chem. Soc. 114:9217-9218 (1992)), analogous organic syntheses of small compound libraries (Chen et al., J. Amer. Chem. Soc. 116:2661 (1994)), oligocarbamates (Cho et al., Science 261:1303 (1993)), and/or peptidyl phosphonates (Campbell et al., J. Org. Chem. 59:658 (1994)), nucleic acid libraries, peptide nucleic acid libraries (see, e.g., U.S. Pat. No. 5,539,083), antibody libraries (see, e.g., Vaughn et al., Nature Biotechnology, 14(3):309-314 (1996) and PCT/US96/10287), carbohydrate libraries (see, e.g., Liang et al., Science, 274:1520-1522 (1996) and U.S. Pat. No. 5,593,853), and small organic molecule libraries (see, e.g., benzodiazepines, Baum C&EN, January 18, page 33 (1993); isoprenoids, U.S. Pat. No. 5,569,588; thiazolidinones and metathiazanones, U.S. Pat. No. 5,549,974; pyrrolidines, U.S. Pat. Nos. 5,525,735 and 5,519,134; morpholino compounds, U.S. Pat. No. 5,506,337; benzodiazepines, U.S. Pat. No. 5,288,514, and the like).

The materials, methods, and examples are illustrative only and not intended to be limiting. Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described herein.

The disclosure is further illustrated by the following examples. The examples are provided for illustrative purposes only. They are not to be construed as limiting the scope or content of the disclosure in any way.

### EXAMPLES

In the Examples described herein, several orthogonal approaches and models were combined to systematically explore the molecular basis whereby mutant p53 promotes invasion and metastasis in PDAC and the clinical implications of its effects. These studies identified the platelet-derived growth factor receptor beta (PDGFRb) as necessary and sufficient to mediate the effects of mutant p53 on invasion and metastasis in both a murine model and human PDAC cells. Further, elevated PDGFRb expression was identified as an indicator of poor metastasis-free survival in human PDAC patients. Taken together, these data identify a key mediator of mutant p53 activity and indicate that PDGFRb inhibitors can act as anti-metastatic agents in patients with tumors expressing mutant p53.

### Methods and Materials

### Retroviral Constructs, Antibodies and Reagents

All vectors were derived from the Murine Stem Cell Virus (MSCV, Clontech) retroviral vector backbone. PDGFRb cDNA (Addgene, #23893) was subcloned into MSCV-PGK-Puro-IRES-GFP (MSCV-PIG) (Hemann et al. 2003). miR30-based shRNAs were designed and cloned as previously described (Zuber et al. 2011), and the sequences are provided below:

| **target gene** | **name** | **organism** | **sequence** | | | | | | | | | |
|---|---|---|---|---|---|---|---|---|---|---|---|---|
| p53 | 1224 | mouse | TGCTGTTGACAGTGAGCGCCCACTACAAGTACATGTGTAATAGTGAAGCCACAGATGTATTACACATGTACTTGTAGTGGATGCCTACTGCCTCGGA | | | | | | | | | |
| p53 | 703 | mouse | TGCTGTTGACAGTGAGCGCCCGGGTGGAAGGAAATTTGTATAGTGAAGCCACAGATGTATACAAATTTCCTTCCACCCGGATGCCTACTGCCTCGGA | | | | | | | | | |
| p53 | 393 | mouse | TGCTGTTGACAGTGAGCGACCCTGTCATCTTTTGTCCCTTTAGTGAAGCCACAGATGTAAAGGGACAAAAGATGACAGGGGTGCCTACTGCCTCGGA | | | | | | | | | |
| p53 | 996 | human | Tgctgttgacagtgagcgcctggaagactccagtggtaattagtgaagccacagatgtaattaccactggagtcttccagttgcctactgcctcgga | | | | | | | | | |
| p53 | 2057 | human | tgctgttgacagtgagcgcggaggatttcatctcttgtattagtgaagccacagatgtaatacaagagatgaaatcctccatgcctactgcctcgga | | | | | | | | | |
| | | | | | | | | | | | | |
| Pdgfrb | 544 | mouse | TGCTGTTGACAGTGAGCGATGGGCCAGAGTTTGTTCTCAATAGTGAAGCCACAGATGTATTGAGAACAAACTCTGGCCCAGTGCCTACTGCCTCGGA | | | | | | | | | |
| Pdgfrb | 5241 | mouse | TGCTGTTGACAGTGAGCGCCACAATGGTACCAAAGATAGATAGTGAAGCCACAGATGTATCTATCTTTGGTACCATTGTGATGCCTACTGCCTCGGA | | | | | | | | | |
| Pdgfrb | 4778 | human | TGCTGTTGACAGTGAGCGACAGCACTAACATTCTAGAGTATAGTGAAGCCACAGATGTATACTCTAGAATGTTAGTGCTGGTGCCTACTGCCTCGGA | | | | | | | | | |
| Pdgfrb | 3204 | human | TGCTGTTGACAGTGAGCGACAGTTCTACAATGCCATCAAATAGTGAAGCCACAGATGTATTTGATGGCATTGTAGAACTGCTGCCTACTGCCTCGGA | | | | | | | | | |
| | | | | | | | | | | | | |
| p73 | 254 | mouse | TGCTGTTGACAGTGAGCGACCCACTCTTGAAGAAGTTGTATAGTGAAGCCACAGATGTATACAACTTCTTCAAGAGTGGGGTGCCTACTGCCTCGGA | | | | | | | | | |
| p73 | 3827 | mouse | TGCTGTTGACAGTGAGCGACAGCTTTGTGCATCCAATTAATAGTGAAGCCACAGATGTATTAATTGGATGCACAAAGCTGGTGCCTACTGCCTCGGA | | | | | | | | | |
| | | | | | | | | | | | | |
| NF-YB | 1 | mouse | TGCTGTTGACAGTGAGCGACTAGGGATTTCTGCAGACTAATAGTGAAGCCACAGATGTATTAGTCTGCAGAAATCCCTAGCTGCCTACTGCCTCGGA | | | | | | | | | |
| NF-YB | 2 | mouse | TGCTGTTGACAGTGAGCGCCAGGAGAAGCGGAAGACAATATAGTGAAGCCACAGATGTATATTGTCTTCCGCTTCTCCTGATGCCTACTGCCTCGGA | | | | | | | | | |
| | | | | | | | | | | | | |
| Ctrl | 1 | - | TGCTGTTGACAGTGAGCGCAGGAATTATAATGCTTATCTATAGTGAAGCCACAGATGTATAGATAAGCATTATAATTCCTATGCCTACTGCCTCGGA | | | | | | | | | |
| Ctrl | 2 | - | tgctgttgacagtgagcgcccgcctgaagtctctgattaatagtgaagccacagatgtattaatcagagacttcaggcggttgcctactgcctcgga | | | | | | | | | |

shRNAs were cloned into the MLP vector (MSCV-mir30-PGK-Puro-IRES-GFP) for constitutive expression, and inducible shRNAs were cloned into the TRMPV-Neo vector (pSIN-TRE-dsRed-miR30-PGK-Venus-IRES-NeoR) as previously described (Zuber et al. 2011). All constructs were verified by sequencing.

p53 was detected using mAb NCL-P53-505 (Novocastra) and hAB OP43 (Calbiochem). Anti-Actin (A3854) was purchased from Sigma. Anti-PDGFRa (3164) and -PDGFRb (3169) antibodies were purchased from Cell Signaling. Alexa Fluor 568-Phalloidin (A12380) was purchased from Invitrogen. Crenolanib (CP-868596) and imatinib (I-5508) were purchased from Selleckchem and LC Laboratories, respectively.

### Cell Culture and Drug Treatments

All cell lines were maintained in DMEM + 10% FBS, at 37° C in 5% CO₂. Stable cell lines expressing shRNAs were generated by retroviral mediated gene transfer. Briefly, Phoenix or Ampho packaging cells (for murine or human cell lines, respectively) were transfected by the calcium phosphate method with vectors expressing NF-YA, NF-YB, p53, p73, PDGFRa, PDGFRb or Renilla-control shRNAs, and the generated viruses were harvested to infect KPC, KP_{fl}C, or a series of human pancreatic, breast, lung and colon cancer cell lines. Infected cells were selected with puromycin and experiments were carried out on derived cell populations. To generate cells stably expressing mutant p53, p73 or PDGFRb, KP_{fl}C cells were infected with MSCV-p53-R175H, -R273H, PDGFRb or pcDNA3-HA-p73α and selected in puromycin and G418 respectively to yield stable pools. Cultured cells were treated with 300 nm crenolanib and 3 µM imatinib. These concentrations were selected after determining the concentration required to fully inhibit the phosphorylation of PDGFRb without any effects on cell proliferation.

### Wound healing and Invasion Assays

Wound healing assays were conducted as previously described (Goulimari et al., 2005). Briefly, cells were seeded in 6-well plates, grown until confluent, and wounding was performed with a 10-µl microtiter tip that was cut longitudinally. Three-dimensional invasion assays were carried out as previously described (Kitzing et al., 2007). In brief, 24-well transwell inserts (Greiner bio-one) were lined with collagen type 1 (BD Biosciences), and cells were seeded on the inverted inserts. The lower chambers were filled with medium containing 0.5% FBS and upper chambers containing the collagen matrix were filled with medium containing 20% FBS and the chemoattractant HGF.

### Immunostaining and Microscopy

Live cell recordings were performed immediately after wounding for 18 h at 37 °C using a Zeiss observer Microscope. Pictures were acquired every 10 min with a motor-controlled Leica DC 350 FX camera, which enables simultaneous recordings from multiple wells. For statistical analysis, the wound distance from each well was measured in duplicate at three randomly defined wound gap locations per frame recorded per experiment. Invasion assay inserts were fixed using 4% formaldehyde and stained using DAPI and Alexa568 phalloidin (Invitrogen) before confocal microscopy (Perkin Elmer Spinning Disk) was conducted. Images were analyzed using Imaris software.

### RT-qPCR

Total RNA was isolated using TRIZOL (Invitrogen), and cDNA was obtained using the TaqMan reverse transcription reagents (Applied Biosystems). Real-time PCR was carried out in triplicate using SYBR Green PCR Master Mix (Applied Biosystems) on the ViiA™ 7 Real-Time PCR System (Invitrogen). GAPDH or β-actin served as endogenous normalization controls. Primer sequences are listed below:

**Primer sequences for RT-qPCR**

| gene symbol | FWD primer | RVS primer |
|---|---|---|
| mPDGFRB.1 | TTCCAGGAGTGATACCAGCTT | AGGGGGCGTGATGACTAGG |
| mPDGFRB.2 | CGCCAGGGCGAGAGCATCAC | CGCCCACTCTTCATGCGGGG |
| mPDGFRA.1 | TCCATGCTAGACTCAGAAGTCA | TCCCGGTGGACACAATTTTTC |
| mPDGFRA.2 | ATGAGAGTGAGATCGAAGGCA | CGGCAAGGTATGATGGCAGAG |
| hPDGFRB.1 | AGACACGGGAGAATACTTTTGC | AGTTCCTCGGCATCATTAGGG |
| hPDGFRB.2 | GCAAAACCACCATTGGGGAC | GGGTGATGTTCTCACCCTGG |
| Trp73.1 | CCACCTGCAGAACCTTACCA | TCATGGCTCTGCTTCAGGTC |
| Trp73.2 | AAAATGGAGCTGCCAGCAAAC | TGGCGTCTCTTCTTCACGTTG |
| Actin | | |
| Gapdh | | |

### RNA sequencing and Data Analysis

Nucleotide sequencing of RNAs of >200 nt length was carried out us previously described (Djebali et al., 2012). Directional (stranded) libraries for Paired End (PE) sequencing on the Illumina platform were generated as described previously (Parkhomchuck et al., 2009). Primary data processing and library mapping was completed using the Spliced Transcripts Alignment to a Reference (STAR) software (Dobin et al., 2013). Differential expression analysis for sequence count data (FPKM values) was conducted using DESeq as described previously (Anders and Huber, 2010).

### PDGFRb Luciferase Reporter Assay

The promoter assay was performed as previously described (Hackzell et al., 2002). In brief, cells were seeded in 24-well plates and transiently transfected with 0.5 ug of expression plasmid, 2.0 ug of reporter plasmid, and 20 ng of renilla-luciferase vector (PGL4.74, Promega). After 36 h, cells were lysed (Dual-Luciferase Reporter Assay System, Promega), and firefly luciferase and renilla activities were measured (Varioskan Flash Multimode Reader, Thermo Scientific). Results shown were normalized to renilla activity and are representative of at least three independent replicates.

### Immunoprecipitation

To detect p53/p73 and p73/NF-Y binding, sub-confluent KP_{fl}C cells were infected with mutant p53 or an empty control construct and transiently transfected with HA.p73 (Addgene) using Lipofectamine 2000 (Invitrogen). Thirty-six hours post-transfection, cells were lysed in RIPA Buffer (20 mM Tris pH 7.4, 1% TritonX-100, 37 mM NaCl, 2 mM EDTA, 1% SDS, 0.5% NP-40, 10% Glycerol, phosphatase inhibitors [2.5 mM Sodium pyrophosphate, 1 mM β-Glycerophosphate, 1 mM Na₃VO₄], and protease inhibitors [Roche]). Whole cell lysates (0.5 mg protein) were pre-cleared with A/G Sepharose beads (Invitrogen), incubated at 4° C with either anti-HA (Covance, 16B12) or anti-p53 antibody (Calbiochem, OP43) overnight and subsequently with protein A/G beads for 2 hr. The bead pellet was extensively washed in lysis buffer three times and then electrophoresed on 10% SDS-PAGE gels followed by immunoblotting using anti-p53, anti-HA, anti-GFP (Cell Signaling, 2555) and anti-NF-YB (Santa Cruz, FL-207) antibodies.

To measure levels of endogenously phosphorylated PDGFRb, KPC cells were treated with the drug as described and harvested in phospho-lysis buffer (50 mM Tris pH 7.5, 1% Tween-20, 200 mM NaCl, 0.2% NP-40, phosphatase inhibitors [2.5 mM Sodium pyrophosphate, 1 mM β-Glycerophosphate, 1 mM Na₃VO₄], and protease inhibitors [Roche]). Anti-PDGFRb (Santa Cruz, 958) was used to immunoprecipitate PDGFRb from whole cell lysate samples containing 0.5 mg protein. Washed immunoprecipitates were subjected to SDS-Page and immunoblotted with anti-PTyr-100 antibody (Cell Signaling, 9411).

### Immunohistochemistry and Immunofluorescence

Tissues were fixed overnight in formalin, embedded in paraffin, and cut into 5-µm thick sections. Sections were subjected to hematoxylin and eosin staining, and immunohistochemical and immunofluorescent staining following standard protocols. The following primary antibodies were used: mouse anti-GFP (Cell Signaling), rabbit anti-p-PDGFR-β (Tyr 1021) (Santa Cruz Biotechnology) and rat anti-CK8 (DSHB). For immunofluorescence Alexa Fluor 488 goat anti-rabbit and Alexa Fluor 568 goat anti-rat were used as secondary antibodies, and DAPI was used as a chromogen. Images were acquired using a Zeiss Axio Imager D1 scope.

### Mouse Studies

All animal experiments were performed in accordance with a protocol approved by the Memorial Sloan-Kettering Institutional Animal Care and Use Committee. For colonization studies, KPC cells (1x10⁵) were resuspended in 200 µl PBS and injected intravenously into the tail vein of 8 week-old female athymic nude mice. Lungs were harvested 7 days post injection and analyzed for colonization by GFP positivity (Nikon SMZ1500) and histology.

*Pdx1-Cre* (Hingorani et al., 2003), *LSL-Kras^{G12D}* (Jackson et al., 2001) and *LSL-p53^{R172H}* (Olive et al, 2004) mouse strains were previously described: Male *Pdx1-Cre*^{+/+}, *LSL-p53*^{*R172H*/*R172H*} mice were bred with female *LSL-Kras*^{*G12D*/+} mice to generate KPC mice. Strains were maintained on mixed background. Mice were genotyped by polymerase chain reaction analysis as described previously (Hingorani et al., 2006). Mice were dosed twice daily by oral gavage with 50 mg/kg Imatinib in ddH₂O or with ddH₂O only. Organs and tumors were removed and fixed in 10% buffered formalin and tumor and metastatic burden was assessed by gross pathology and histology.

### Human Data Sets

The gene expression data and survival analyses of PDAC patients is from The International Cancer Genome Consortium (ICGC) pancreatic cancer project, which is stored at Gene Expression Omnibus (GEO) with accession number GSE50827. It includes gene expression data from 103 primary tumor samples, 89 of which contain disease-free survival and clinicopathological annotations, and used in the survival analysis according to previously described methods (Biankin et al., 2012).

Gene expression data of ovarian, colorectal, and pancreatic cancer patients with annotated clinical outcomes were downloaded from the Gene Expression Omnibus database (GSE9899 (Tothill et al., 2008), GSE17537 (Smith et al., 2010), and GSE28735 (Zhang et al., 2012)). Preprocessed data were downloaded as provided in the data matrix files (GCRMA/RMA normalized Affymetrix expression microarray data) and gene set enrichment analysis (GSEA) was performed on each set after mean-centering across samples. Median of probes per gene was used to account for differential representation on the chip. The GSEA scores were then clustered into two groups ("High", "Low") using kmeans, and a Kaplan-Meier analysis was performed to obtain survival outcome plots. Significance for these plots was determined using the logrank test.

### Results

### Sustained Expression of Mutant p53 is Required for the Invasive Phenotype of Pancreatic Cancer Cells

Genetically engineered mouse models of pancreas cancer harboring a latent oncogenic *Kras* allele (*lox-stop-lox Kras^{G12D}*), a latent mutant *p53^{R172H}*, and a tissue specific *Cre* recombinase (*Pdx1-*Cre), also known as KPC mice, develop highly metastatic pancreatic cancer that faithfully mimics the human disease (Hingorani et al., 2005). To understand the impact of p53 mutations on cell invasion and metastasis in this well-defined genetic system, a murine KPC pancreatic cancer cell line was employed that lost the remaining p53 wild type allele during disease progression (Morton et al., 2010). The behavior of KPC cell lines stably expressing shRNAs targeting (mutant) p53, or control shRNAs targeting *Renilla* (KPC+sh.Ctrl), were compared to one another and to a p53-null KPflC cell line (from a *Pdx-Cre, LSL-Kras*^{*G12D*/+}, *LSL-p53*^{*loxP*/+}) expressing the control shRNA (KP_{fl}C+sh.Ctrl).

KPC+sh.Ctrl cells expressing mutant p53 efficiently migrated to close the wound using a scratch-wound assay. This motility depended on the mutant p53, as mutant p53 knockdown in KPC+sh.p53 cells reduced motility similarly to *p53*^{*-*/*-*} KP_{fl}C+sh.Ctrl cells (Figure 1A). Next, the invasive capacity of KPC cells into collagen gels in an inverted invasion assay was examined. The presence of mutant p53 in KPC+sh.Ctrl cells enhanced invasiveness, which was significantly abrogated upon p53 knockdown (Figure 1B). The ability of mutant p53 to drive cell invasion was also evident following enforced expression of p53^{R175H} and p53^{R273H}, two mutants frequently found in human PDAC, in KP_{fl}C cells. This result indicates that the differences in invasiveness of KPC and KP_{fl}C cells depends on mutant p53 and were not acquired during generation and selection of cell populations expressing sh.RNAs or through RNAi off-target effects.

To test whether mutant p53 expression was required to sustain the metastatic potential of KPC cells, KPC+sh.Ctrl and KPC+sh.p53 cells were orthotopically injected into the pancreata of athymic mice, and the number of metastases formed in both lung and liver, the most common sites of pancreatic cancer spread in patients, were scored. Although the primary tumor burden was independent of the p53 status, tumors originating from KPC+sh.Ctrl cells expressing mutant p53 were significantly more metastatic than those in which mutant p53 was silenced, and metastases in the lung and liver were detected to a greater extent in mice that had been injected with cells expressing mutant p53 (Figure 1C). Together, these results demonstrate that mutant p53 can contribute to PDAC invasion and metastasis and that inhibiting its activity can have an anti-metastatic effect.

### Transcriptional Profiling and Functional Screening Identifies PDGFRb as a Downstream Mediator of Mutant p53 in Murine Pancreatic Cancer

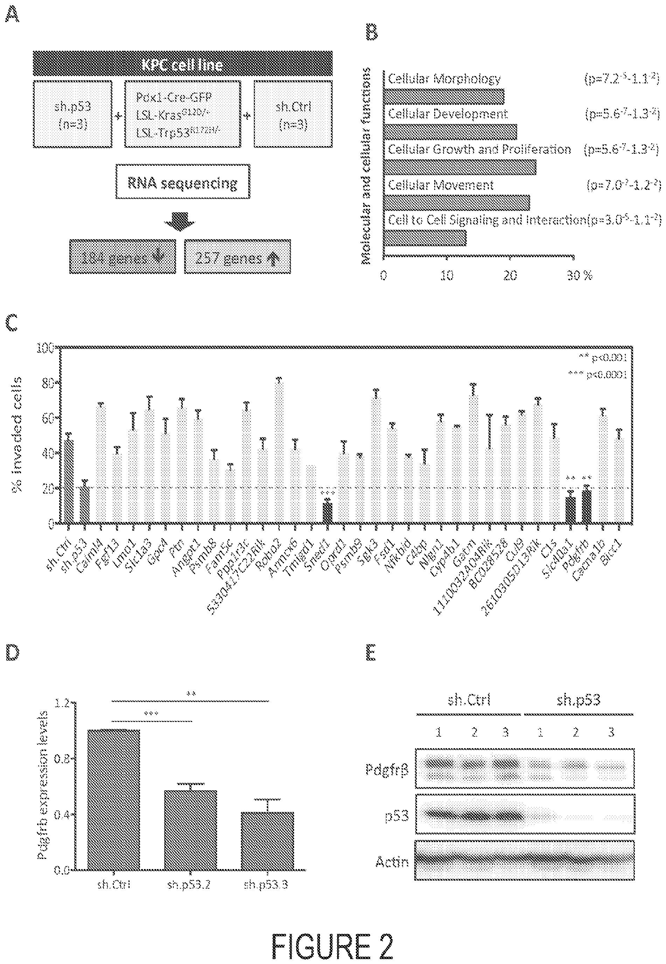

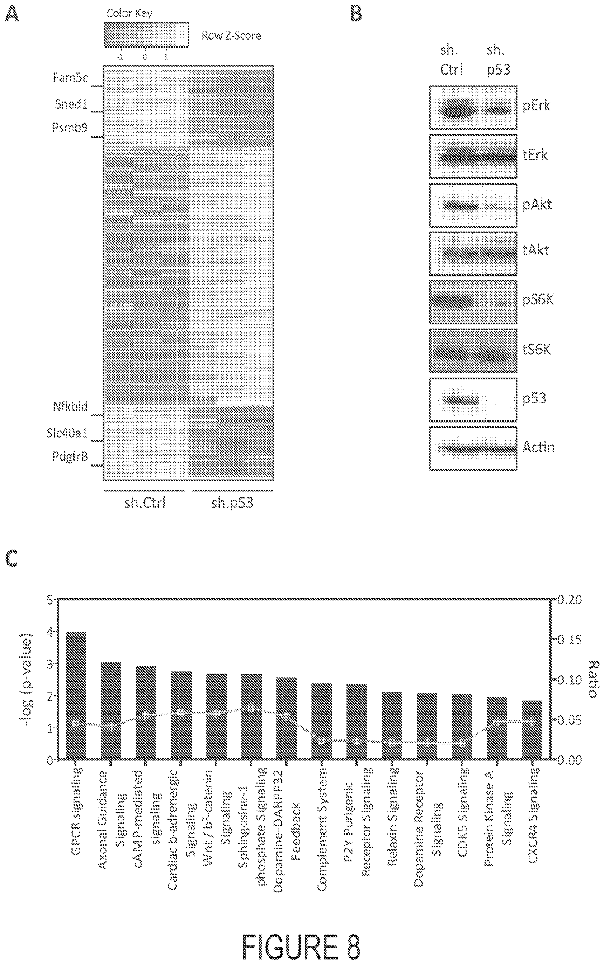

To gain insight into how mutant p53 mediates the invasive phenotype of PDAC, genome-wide transcriptome profiling of KPC cells was performed by RNA sequencing (RNAseq). Four days following knockdown of mutant p53 in three clonal populations resulted in a complex pattern of gene expression changes compared to those observed in three independent KPC+sh.Ctrl cell lines. 441 genes were identified as either significantly up- or down regulated upon shRNA-mediated depletion of endogenous mutant p53 (Figures 2A and 8A). The table below lists the top 40 deregulated genes upon mutant p53 knock down:

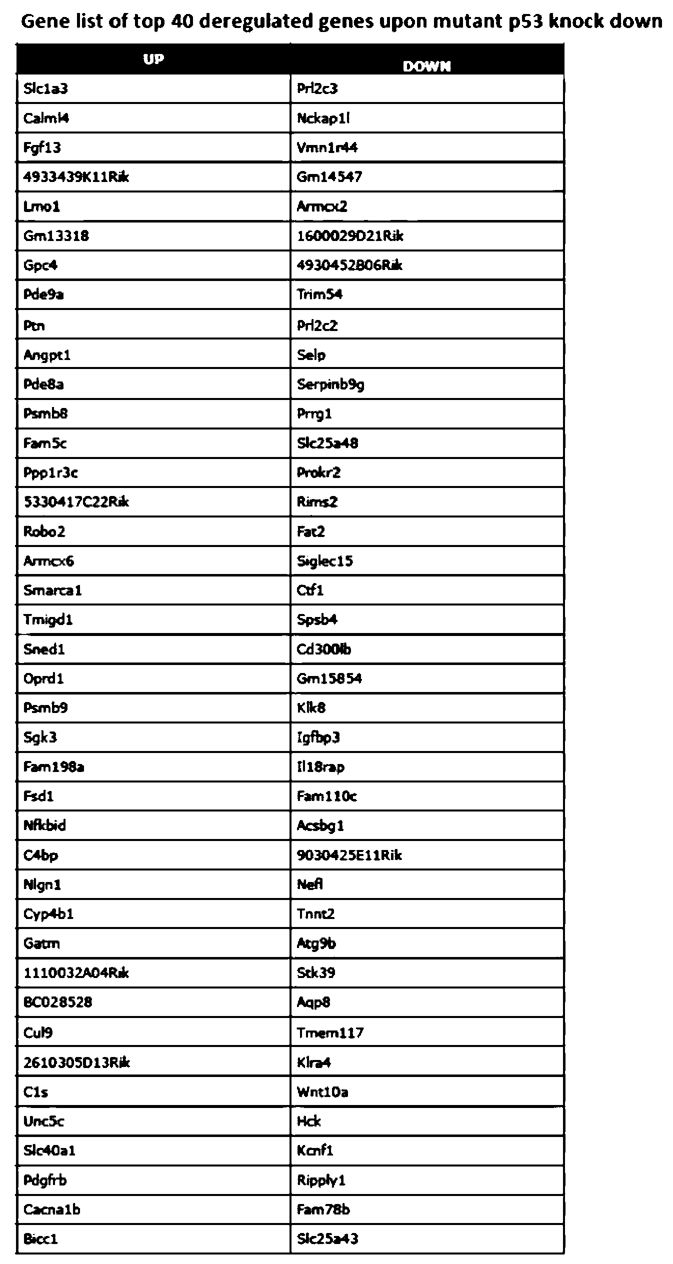

Ingenuity pathway analysis revealed that -20% of the affected genes were involved in the "cellular movement" program, confirming experimental observations that mutant p53 can govern the invasive phenotype of pancreatic cancer cells (Figures 2B and 8C).

To facilitate the identification of mediators of mutant p53 activity, genes whose expression was positively regulated by mutant p53 were analyzed, as such molecules might both mediate effects of mutant p53 and be targets for pharmacological inhibition. Therefore, pools of 3-6 shRNAs targeting individual upregulated genes were generated and screened one-by-one to identify those that phenocopied the decreased invasion seen upon downregulation of mutant p53. Three genes were identified whose knock down abrogated invasion driven by mutant p53 (Figure 2C). SLC40A1 is a cell membrane protein that mediates cellular iron efflux (Montalbetti et al., 2013); SNED1 is a stromal marker that induces cisplatin-resistance in head and neck squamous carcinoma (Longatic et al., 2013); and PDGFRb is a receptor tyrosine kinase that mediates PDGF-regulated proliferation, survival and chemotaxis (Dai, 2010).

Oncogenic properties of mutated or amplified PDGFRa have been studied in several tumor types, whereas PDGFRb has been linked to tumor angiogenesis via paracrine effects (Pietras et al., 2003; Cao et al., 2004). The result of the screening described herein led to the hypothesis for a cell-autonomous role of PDGFRb on cell invasion in pancreatic cancer. First, a reduction in PDGFRb mRNA and protein upon knockdown of mutant p53 was verified by RT-qPCR and western blotting (Figures 2D and 2E). Expression of mutant p53 correlated with high PDGFRb expression levels and also with the expression of key downstream mediators of the PDGFRb signaling cascade (Figure 8B).

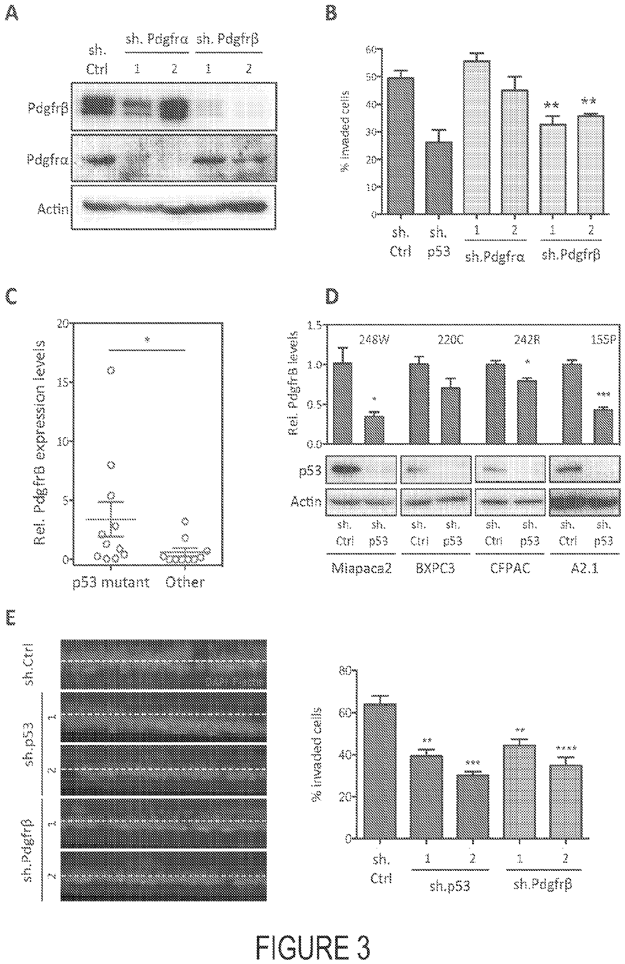

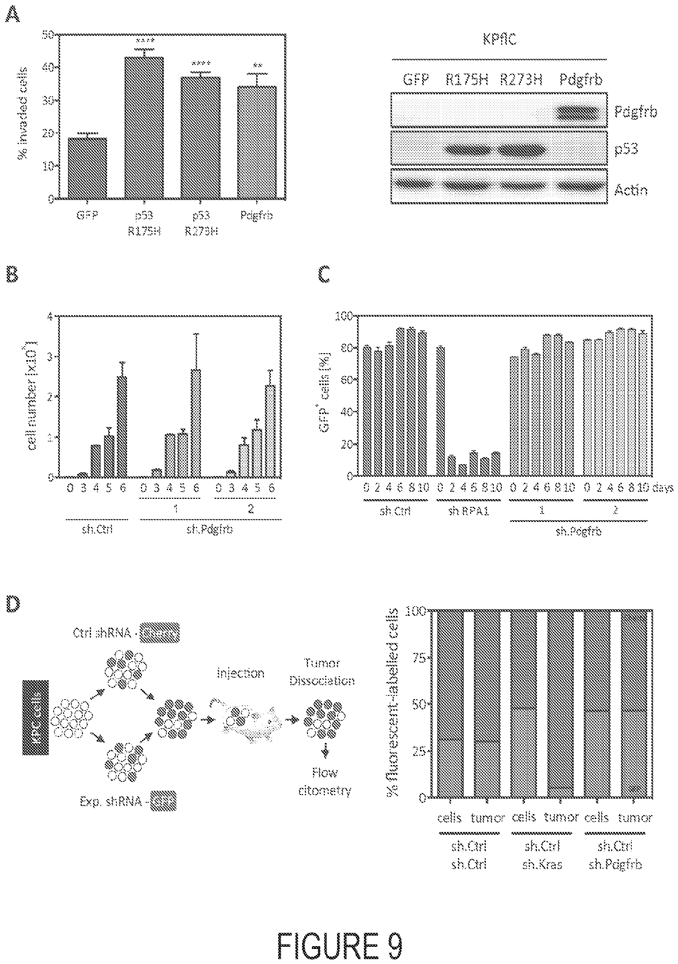

Next, the effects of depleting each PDGFR isoform on the invasive potential of KPC cells were examined in vitro (Figure 3A). Although knock down of PDGFRa had no effect, depletion of PDGFRb decreased the ability of KPC cells to invade (Figure 3B). Conversely, overexpression of PDGFRb in *p53*^{*-*/*-*} KP_{fl}C enhanced cell migration and invasion, similarly to cells over-expressing mutant forms of p53 (Figure 9A). Reduced levels of PDGFRb in KPC cells neither altered the rate of cell proliferation (Figure 9B) nor led to a competitive proliferative disadvantage over KPC cells expressing high PDGFRb levels (Figure 9C). When GFP-positive KPC+sh.PDGFRb cells were mixed with cherry-positive KPC+sh.Ctrl cells and injected subcutaneously into athymic mice, the GFP:cherry ratio of pre-injected cells was maintained in the established tumors, indicating that increased PDGFRb levels did not confer a selective advantage to tumor cell proliferation at the site of injection (Figure 9D). Thus, cell-autonomous activity of PDGFRb is not required for the proliferation and tumorigenic potential of p53 mutant murine cancer cells but specifically impacts their invasive potential.

### PDGFRb Mediates Mutant p53 Action in Human Cancer Cells

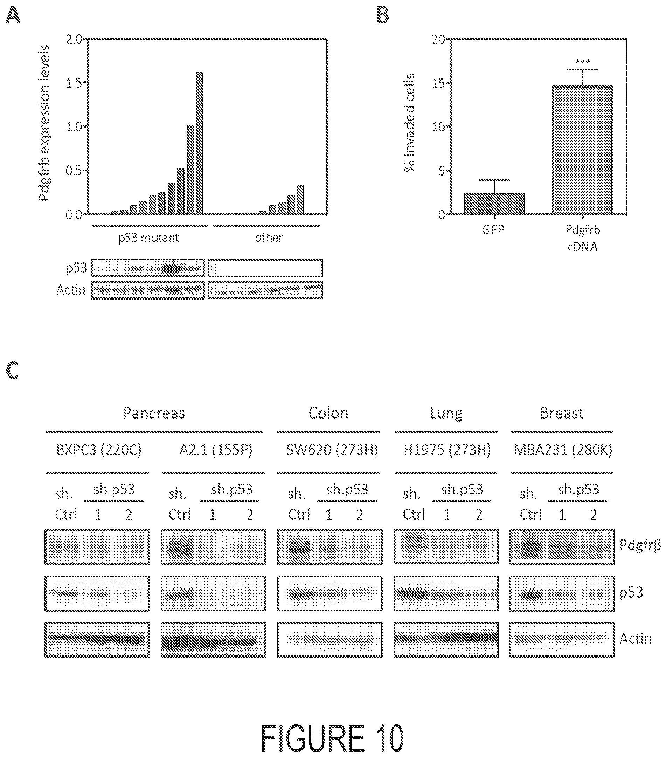

To determine if the mutant p53-PDGFRb signaling axis acts in human cancer cells, PDGFRb expression levels were analyzed in a panel of human pancreatic cancer cell lines. As in our model, PDGFRb mRNA levels were significantly higher in cells expressing mutant p53 compared to those in cell lines that maintained or lost the wild type p53 allele (Figures 3C and 10A). Furthermore, knock down of mutant p53 in Miapaca2, BXPC3, CFPAC and A2.1 cell lines (carrying the 248W, 220C, 242R, and 155P alleles, respectively) decreased PDGFRb mRNA levels to varying degrees (Figure 3D). Knockdown of mutant p53 also decreased PDGFRb in several human colon (SW620, p53^{273H/P309S}), lung (H1975, p53^{273H}), and breast (MDA-MB-231, p53^{280K}) cancer cell lines (Figure 10C). Thus, the ability of mutant p53 to induce PDGFRb levels is not strictly related to a particular p53 allele or tumor type.

The functional connection between mutant p53 and PDGFRb in promoting the invasiveness of human PDAC lines was further analyzed. Consistent with the studies in mice, knockdown of either mutant p53 or PDGFRb reduced invasiveness of the A2.1 pancreatic cancer cell line (Figure 3E). Conversely, overexpression of PDGFRb in the human *p53*^{*-*/*-*} ASPC pancreatic cancer cell line enhanced invasion compared to cells infected with a GFP control vector (Figure 10B). Collectively, these results confirmed that upregulation of the PDGFRb receptor is important for the action of mutant p53 in PDAC and possibly other tumor types.

### Mutant p53 disrupts the p73/NF-Y Complex to Mediate PDGFRb Expression and Tumor Cell Invasion

Several pro-oncogenic properties of mutant p53 depend on its ability to physically interact with and inhibit the p53 family members, p63 and p73 (Li and Prives, 2007). Despite its similarity to p63, the role of p73 in invasion and metastasis has not been directly examined. Since previous reports indicated that p73 can repress the transcription of *PDGFRB* (Hackzell et al., 2002), and because our KPC cells expressed p73 but not p63 as determined by RNA-seq (data not shown), whether the physical interaction of mutant p53 with p73 impairs the ability of p73 to negatively regulate the expression of PDGFRb was examined.

First, the interaction between p73 and mutant p53 proteins was verified by reciprocal co-immunoprecipitation (Figure 4A) and, consistent with previous reports, p73 binding to a "conformation" p53 mutant (R175H) appeared stronger than to a "DNA-binding" p53 mutant (R273H) (Gaiddon et al., 2001; Muller et al., 2009). Next, using a luciferase reporter driven from the *PDGFRB* promoter, overexpression of p73 in KP_{fl}C cells decreased transcriptional activity of *PDGFRB* and, conversely, knockdown of endogenous p73 increased luciferase expression (Figures 4C and 11A). A similar increase in luciferase signal upon overexpression of two distinct forms of mutant p53 in KP_{fl}C cells was demonstrated (Figure 4C) as well as a significant decrease upon depletion of mutant p53 or overexpression of p73 in KPC cells (Figure 11C). Importantly, depletion of endogenous p73 in KPC cells expressing mutant p53 did not enhance the transcription of *PDGFRB* (Figure 11C), indicating that the repressing activity of p73 is regulated by its interaction with mutant p53. Hence, mutant p53 cancels the ability of p73 to repress PDGFRb transcription, leading to an increase in its expression.

To better understand how p73 represses *PDGFRB* transcription, chromatin immunoprecipitation (ChIP) analysis in KP_{fl}C cells was performed, but no direct binding of p73 to the *PDGFRB* promoter was detected (data not shown), a result consistent with previous reports (Matys et al., 2006). Nevertheless, promoter analysis of *PDGFRB* (Transfec®, Biobase) identified a conserved CCAAT binding motif for NF-Y, a well-characterized transcriptional activator of *PDGFRB* that physically interacts with p73 (Ballagi et al., 1995; Ishisaki et al., 1997). NF-Y binding to the *PDGFRB* promoter was demonstrated using ChIP analysis (Figure 4B). Remarkably, this binding was prevented by p73 overexpression, suggesting that the p73/NF-Y interaction hampers its ability to bind and activate the *PDGFRB* promoter (Figure 4B). Indeed, when p73 was immunoprecipitated in KP_{fl}C cells, direct binding of NF-Y to p73 was detected (Figure 4A). This interaction was abrogated upon expression of mutant p53, indicating that it disrupts or interferes with the formation of the repressive p73/NF-Y complex (Figure 4A).

Next, whether the repressive action of p73 on *PDGFRB* transcription was mediated by NF-Y and modulated by mutant p53, and the implications of this regulatory circuit for invasion, was tested. Interestingly, the ability of p73 overexpression to inhibit the PDGFRB-luc reporter was abolished by depletion of NF-Y subunits A and B (Figures 4D and 11B), and NF-Y knockdown suppressed the ability of mutant p53 to enhance PDGFRb expression in KP_{fl}C cells (Figure 11D). p73 overexpression reduced the invasive potential of KP_{fl}C cells, whereas knockdown of p73 or overexpression of mutant p53 significantly increased invasiveness (Figure 4E). Conversely, as occured in mutant p53 cells following p73 overexpression, depletion of p53 also reduced the invasive behavior in KPC cells (Figure 11E). Finally, NF-Y knockdown restored the invasive potential of KP_{fl}C cells that overexpressed p73 (Figure 4F) and suppressed the ability of mutant p53 to enhance cell invasion (Figure 11F). Together these results support a model in which mutant p53 promotes invasion in pancreas cancer cells via an indirect mechanism that depends on its ability to enhance PDGFRb expression through the disruption of the repressive p73/NF-Y complex (Figure 4G).

### Modulation of PDGFRb Expression Levels Mediates the Phenotypic Effects of Mutant p53 Depletion in vivo

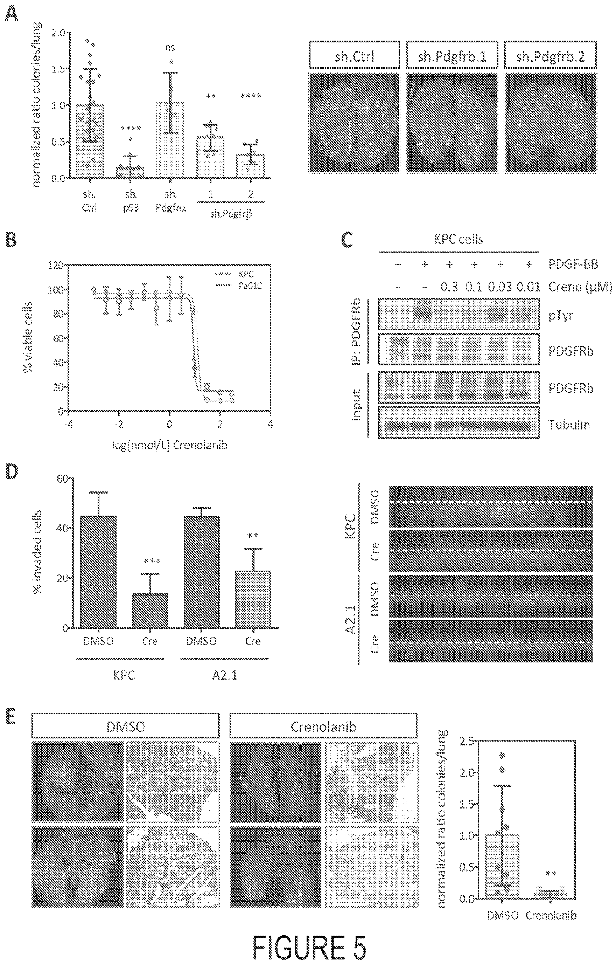

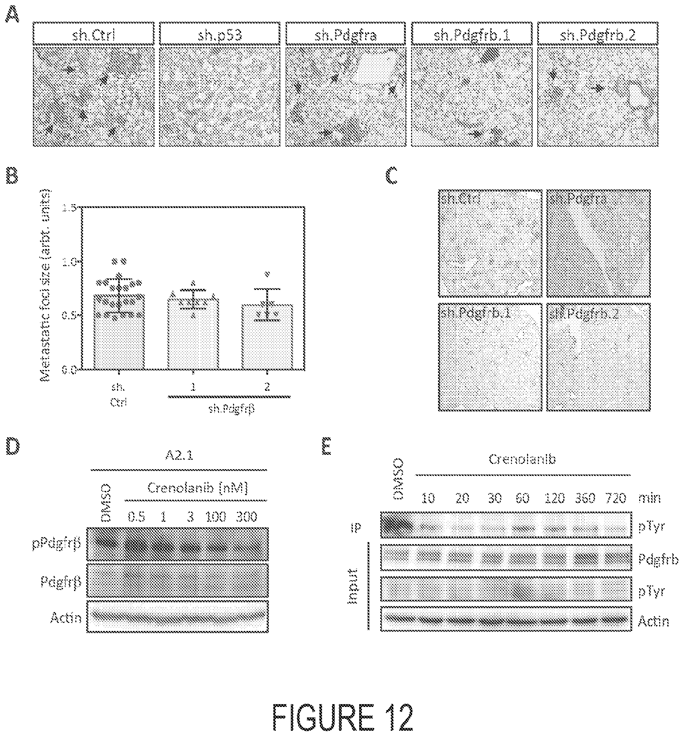

Following the observation that p53 mutants induce the expression of PDGFRb to promote cell invasion in PDAC cultures, whether PDGFRb levels regulate metastatic behavior of PDAC cells in mice was investigated. To this end, a lung colonization assay was performed by injecting KPC+sh.p53, KPC+sh.PDGFRB, KPC+sh.*PDGFRA* or KPC+sh.*Ctrl*-cells intravenously via the tail vein into athymic mice and scoring the number of colonies formed in the lungs. Whereas KPC+sh.*Ctrl* and KPC+sh.PDGFRa cells expressing mutant p53 formed tumor nodules in the lungs at high frequency, PDGFRb depletion significantly reduced the number of lung colonies, phenocopying the anti-metastatic effect observed upon knocking down mutant p53 (Figures 5A and 12A). However, depletion of PDGFRb did not affect the size of the metastatic foci, suggesting that PDGFRb does not alter their capacity to grow and proliferate in a new environment (Figure 12B). In whole lung sections, GFP-positive signals coincided with metastatic nodules, indicating that metastases formed from cells expressing shRNAs, and likely not from the proliferation of tumor cells that lost shRNA expression (Figure 12C).

Whether pharmacologic inhibition of the PDGFRb pathway recapitulates the effects of PDGFRb or mutant p53 depletion was examined (Figure 5A and 12A). The compound crenolanib was used, which is a small molecule inhibitor of type III tyrosine kinases, potent against PDGFRa, PDGFRb, and FLT3 but not other known receptor tyrosine kinases (VEGFR, FGFR), serine/threonine (RAF) or tyrosine kinases (ABL1) (Lewis et al., 2009). The potency and efficacy of crenolanib on inhibiting the viability of murine KPC and human A2.1 pancreatic cancer cells were assessed. The dose-response patterns were comparable between the two cell lines, with IC₅₀ values of 13.1 µM and 8.5 µM, respectively (Figure 5B). Strong inhibition of PDGFRb activity, as measured by phospho-PDGFRb, was achieved in both cell lines at 0.3 µM, a dose at which no toxicity was observed (Figures 5C and 12D). Time course experiments revealed that strong target inhibition was achieved within 10 min of drug treatment (Figure 12E). Accordingly, crenolanib treatment of KPC and A2.1 cells substantially reduced invasion relative to that seen with cells treated with DMSO (Figure 5D).

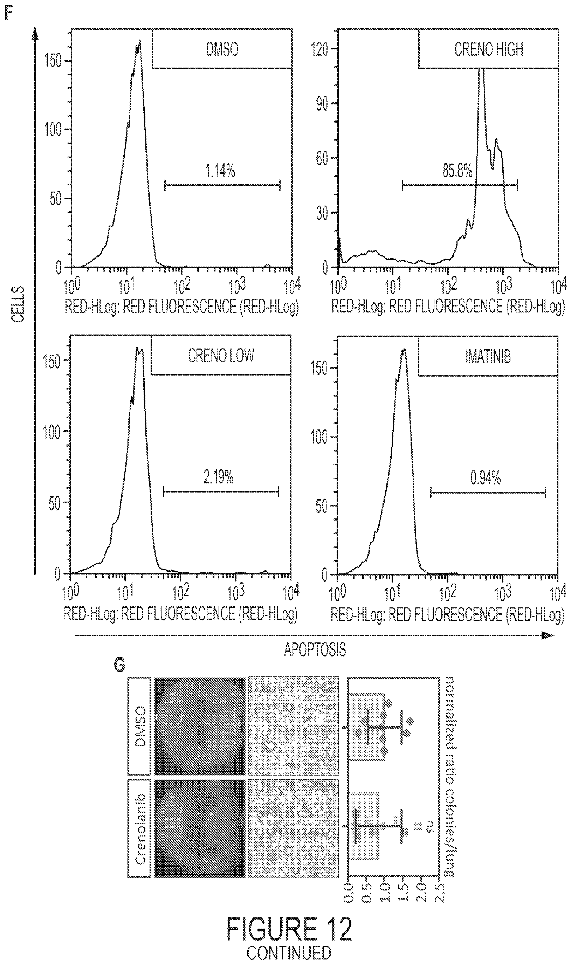

To test whether crenolanib can suppress metastasis, KPC cells were pretreated with the drug overnight and injected intravenously into recipient mice that were subsequently assessed for colony formation in the lung. Although drug treatment had no effect on the viability of the injected cell population, mice injected with drug-treated KPC cells showed significantly fewer lung nodules compared to controls pre-treated with DMSO (Figures 5E and 12F). Conversely, the same concentration of crenolanib did not reduce the metastatic potential of KP_{fl}C cells in a lung colonization assay, suggesting that PDGFRb acts autonomously in KPC cells to potentiate cell invasion and metastasis (Figure 12G). Therefore, small molecule inhibition of PDGFRb or depletion by RNAi leads to a significant reduction of invasion and metastasis driven by mutant p53 both in vitro and in vivo.

### Imatinib Inhibits the Development of Metastases in a PDAC Mouse Model by targeting PDGFRb

The results described above imply that pharmacologic inhibition of PDGFRb could have anti-metastatic effects. Therefore, whether PDGFRb inhibition prevented metastasis in KPC mice that develop metastatic disease with a variable latency of 3 to 10 months in 50 - 80% of animals (Hingorani et al., 2005) was determined. FDA-approved imatinib, a potent inhibitor of PDGFRb, c-KIT and BCR-ABL activity, was used. Notably, the c-KIT and BCR-ABL kinases have not been linked to PDAC development (Jones et al., 2008).

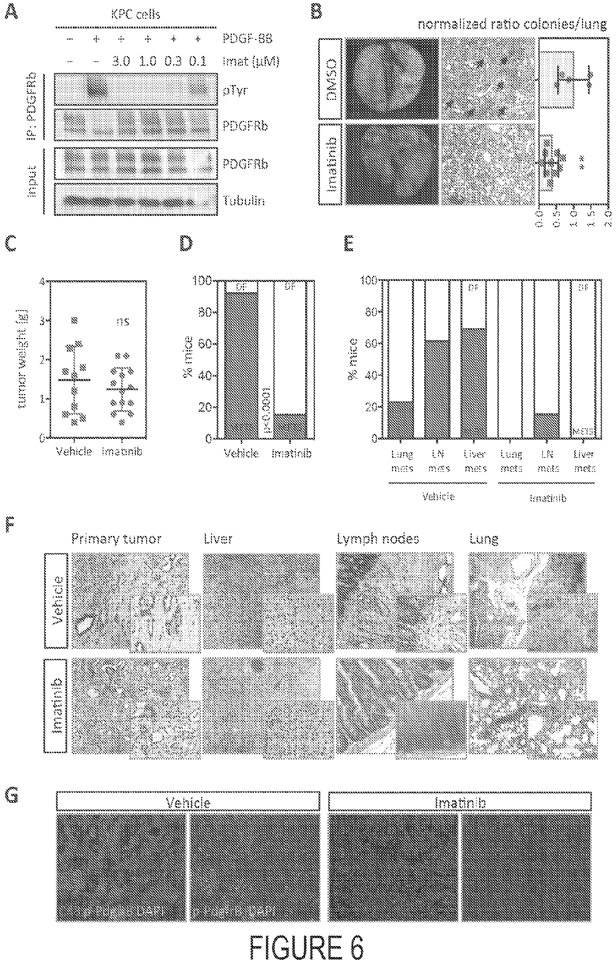

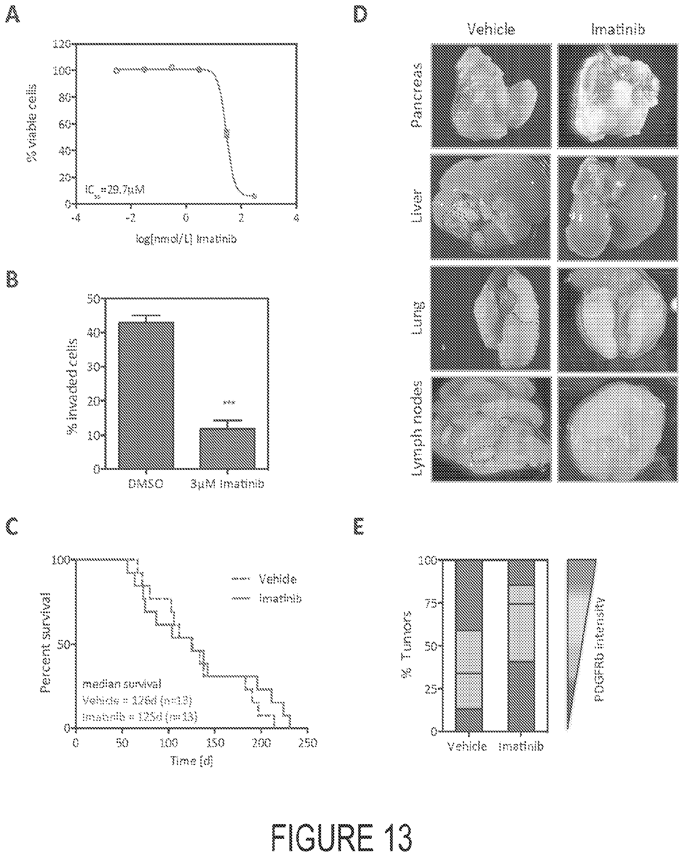

Inhibition of PDGFRb by imatinib in KPC cells strongly reduced PDGFRb tyrosine phosphorylation at 3 uM (Figure 6A), a dose of the drug that was significantly lower than that required to inhibit cell proliferation (IC₅₀ of 29.7 µM) (Figure 13A). Nonetheless, imatinib treatment significantly reduced the invasive potential of KPC cells in vitro (Figure 13B). More importantly, pre-treatment of KPC cells with imatinib decreased their potential to colonize the lungs of recipient athymic mice to a similar extent as that seen upon crenolanib treatment (Figure 6B).

Next, KPC mice were treated with imatinib to assess its effects on metastases. To this end, mice were treated with a dose of 50 mg/kg imatinib by oral gavage twice daily, a regimen that produces therapeutic concentrations of imatinib in mice (Wolff et al., 2003; Beppu et al., 2004). Treatment was initiated in mice of 8 weeks of age, a time at which KPC mice have developed preneoplastic lesions (Hingorani et al., 2005), and mice were monitored until they became symptomatic. Imatinib had no impact on tumor volume in the pancreas or overall survival, suggesting that the high disease burden in the pancreas was the primary cause of cell death (Figures 6C and 13C).

However, imatinib induced a striking reduction in the occurrence of metastasis. The incidence of metastasis was 92% in vehicle-treated animals compared to 15% in mice treated with imatinib, as assessed by GFP fluorescence and confirmed by histopathological analyses (χ² test, p<0.0001) (Figures 6D and 13D). The anti-metastatic effect was observed across several organs such as liver, lymph nodes and lung (Figures 6E and 6F). Imatinib effectively inhibited PDGFRb activity in primary tumors based on reduced levels of phospho-PDGFRb in the tumor cells (Figures 6G and 13E). Together, these data suggest that by inhibiting the kinase activity of PDGFRb, imatinib significantly diminishes the metastatic potential of cancer cells, such as pancreatic cancer cells.

### PDGFRb Expression Correlates with Disease Free-Survival in Human Pancreatic, Colorectal and Ovarian Cancer Patients

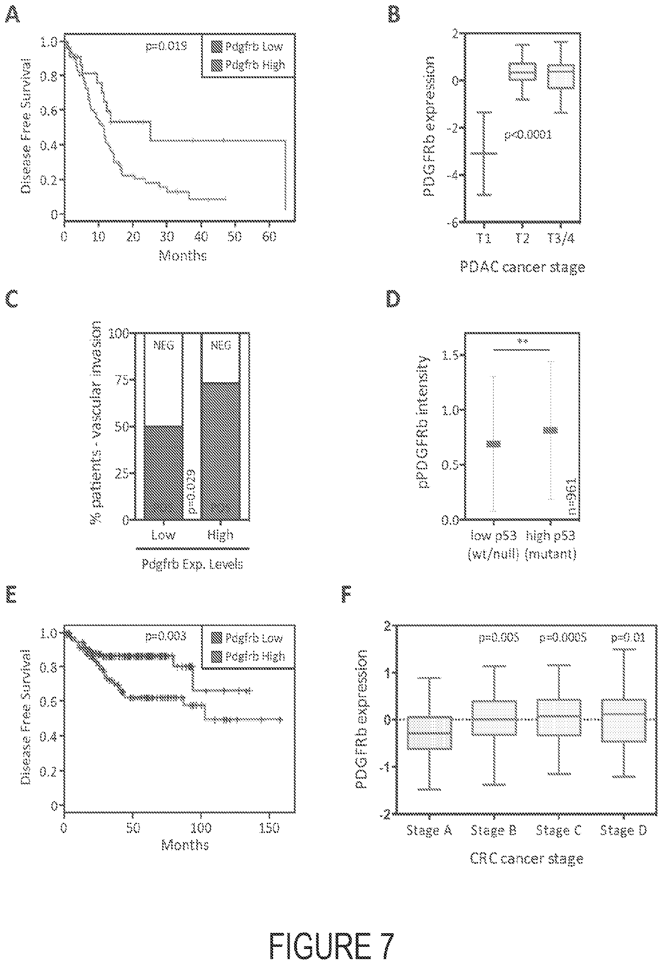

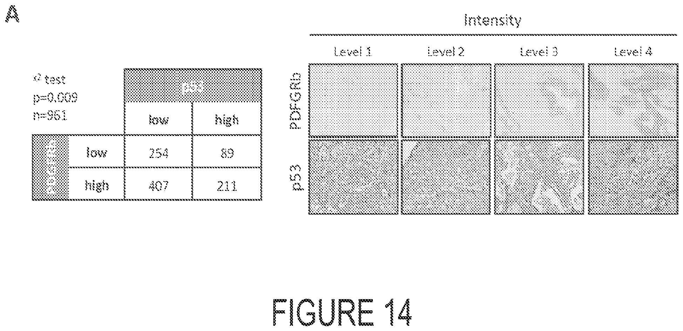

To investigate the clinical significance of PDGFRb expression, whether upregulation of this gene was correlated with prognosis or with the clinicopathological characteristics of PDACs in patients was examined. To avoid confounding signals from the tumor stroma, which can be substantial (data not shown), PDGFRb mRNA levels were assessed in microdissected tumor samples by RT-qPCR. Strikingly, pancreatic cancer patients with tumors expressing high levels of PDGFRb showed a poor disease-free survival and, hence, shorter time to relapse including metastases in distant organs (p=0.019) (Figure 7A). Additionally, PDGFRb expression levels were significantly elevated in late-stage PDAC as compared to the earlier stages (Figure 7B). Patients with high PDGFRb levels also displayed an increase in tumor cells invading the vascular space, another clinicopathological characteristic of tumor dissemination (Figure 7C). Next, whether PDGFRb levels correlated with the status of p53 was tested by analyzing a panel of PDAC tissue microarrays (TMAs). Of importance, significantly higher levels of activated PDGFRb were observed in those tumors that showed an accumulation of p53 (p=0.004), which generally represents tumors with p53 mutation (Cooks et al., 2013) (Figures 7D and 14A). These results confirmed results obtained with mice and underscore a role of mutant p53 in regulating the PDGF signaling in human pancreatic cancer.

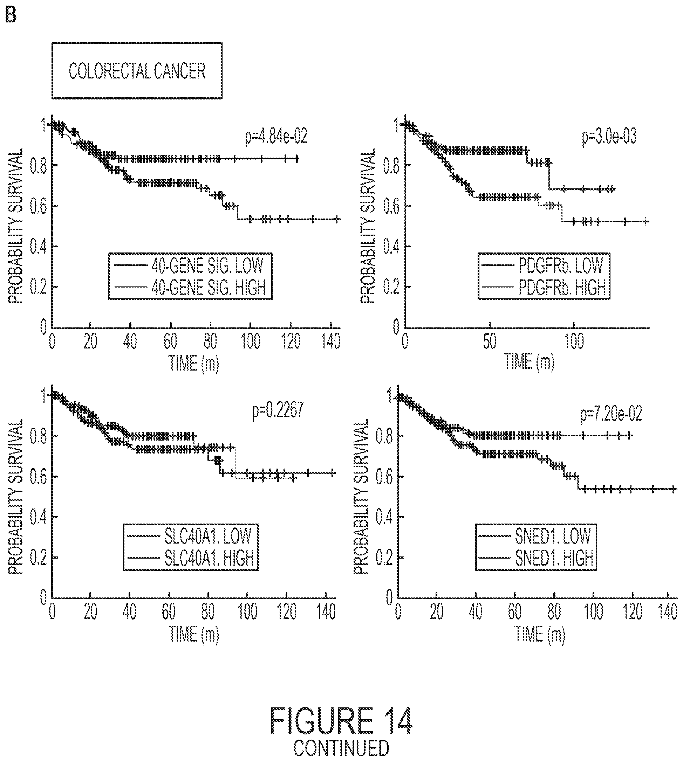

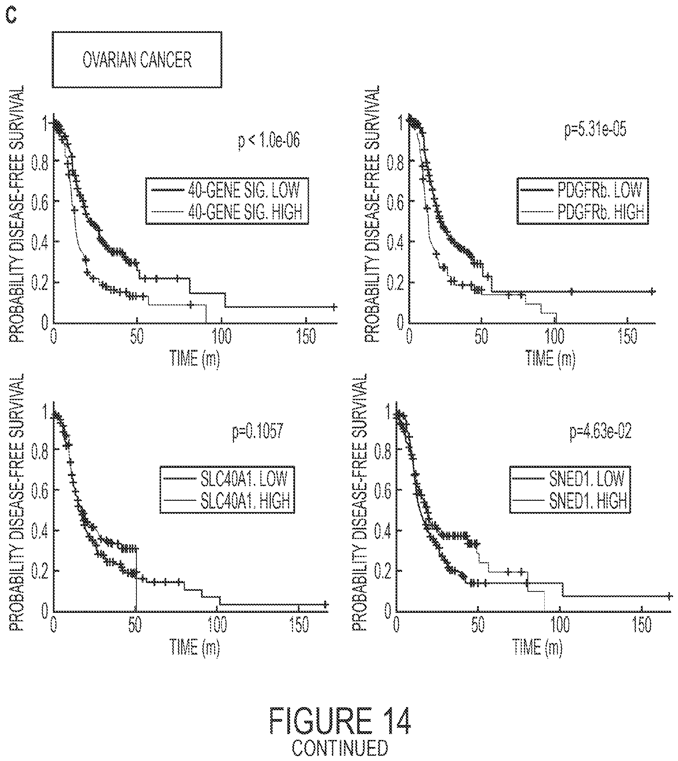

The clinical significance of PDGFRb in colorectal and ovarian cancer was also analyzed, and levels of PDGFRb significantly stratified colorectal and ovarian tumor patients into two distinct cohorts. Patients with tumors expressing low PDGFRb levels exhibited a lower probability to form metastases compared to patients with PDGFRb high-expressing tumors (p=0.003 and p<0.0001) (Figures 7E and 14C). As in PDAC, a significant increase in PDGFRb expression was observed in higher stage colorectal cancers (Figure 7F). In addition to PDGFRb, mutant p53 gene signature significantly scored as a prognostic marker in colorectal and ovarian tumor patients (top 40 genes upregulated in KPC cells; Figure 2C). When the three genes that scored in the invasion assay screen of the disclosure were analyzed, the *PDGFRB* gene was the strongest predictor for the probability to develop metastasis in colorectal and ovarian cancer patients (Figures 14B and 14C). In summary, consistent with functional studies described herein, elevated PDGFRb expression correlated significantly with the status of p53, higher tumor stage, and a poorer disease-free survival rate in pancreatic, colorectal and ovarian cancer patients.

### Discussion

Mutations that occur in the p53 tumor suppressor can inactivate wild-type p53 functions but can also produce "gain-of-function" oncogenic properties that can contribute to cell proliferation, survival and metastasis. Described herein are results of analysis of the phenotypic effects of mutant p53 in pancreatic cancer. As demonstrated herein, the sustained expression of the mutant p53 allele is necessary to maintain the invasive phenotype of PDAC cells by increasing the expression of PDGFRb. These results have several ramifications for understanding of mutant p53 action as well as the behavior and potential treatment of pancreatic cancer.

Signaling through PDGFRb can contribute to multiple tumor-associated processes including cell invasion and metastasis. Given the generally restricted expression of PDGFRb to mesenchymal cell types, most of its oncogenic properties are thought to reflect paracrine effects of tumor cell-secreted PDGF. Indeed, previous work on the role of PDGFRb in carcinoma progression and metastasis suggest that it mainly elicits responses in the tumor stroma by promoting tumor angiogenesis (Pietras et al., 2003; Cao et al., 2004). In contrast, the results described herein provide new evidence for tumor cell-specific expression of PDGFRb in promoting metastasis. As demonstrated herein, genetic or pharmacological inhibition of PDGFRb in the pancreas cancer cells themselves dramatically reduced their invasive and metastatic potential, and that treatment of mice harboring genetic and histologically relevant tumors prevented metastatic spread in vivo. Together, these data suggest that pancreatic tumor cells expressing mutant p53 not only synthesize PDGF but also up-regulate PDGFRb, leading to a tumor cell-autonomous effect.

Though increases in PDGFRb expression were necessary and sufficient to mediate mutant p53 effects in the model described herein, at least two additional genes (*SNED1* and *SLC40A1*) were identified that also contribute to the invasive phenotype through as yet unknown mechanisms. Studies in other systems, primarily breast cancer, have suggested that CXCR4, cyclin-G2, and the mevalonate pathway are mediators of the pro-metastatic activities of mutant p53 (Mehta et al., 2007; Adorno et al., 2009; Freed-Pastor et al., 2012). In addition, mutant p53 has also been reported to drive invasion by regulating several miRNAs such as miR155 and miR130b (Neilsen et al., 2013; Dong et al., 2013). In agreement, miR155 was found to be positively regulated by mutant p53 in KPC cells and promoted metastasis in the model described herein (data not shown); however, in contrast to a previous report (Su et al., 2010), no effects on DICER expression were observed, arguing that in pancreas cancer the mutant p53-associated changes in microRNA expression and metastasis are DICER independent. Regardless, as for wild type p53, mutant p53 exerts effects through the regulation of multiple genes rather than by modulating a single signaling pathway.

Most of the prior understanding of how mutant p53 mediates its oncogenic activity involved exploring the consequences of the physical interaction between the mutant protein and the p53 family members, p63 and p73. Whereas the mutant p53-p63 interaction can modulate the expression of p63 targets genes to enhance invasion and metastasis (Adorno et al., 2009; Muller et al., 2009; Girardini et al., 2011), the role of p73 in these oncogenic properties was unknown. Previous studies have shown that (1) loss of p73 in a p53-null background might be functionally equivalent to the expression of mutant p53 (Flores et al., 2005; Lang et al., 2004); (2) aberrant transcriptional regulation by mutant p53 is mediated through the transcriptional activator NF-Y (Di Agostino et al., 2006); and (3) mutant p53 promotes recycling of receptor tyrosine kinases to initiate invasion (Muller et al., 2009). Demonstrated herein, among other things, is a mechanism where mutant p53 enhances the metastatic potential of pancreatic cancer cells by modulating p73 and its interaction with the transcriptional activator NF-Y.

Mutations in *KRAS, p53, CDKN2A, BRCA2* and *SMAD4* define the genetic landscape of PDAC. Provided herein is evidence for a crucial role of mutant p53 in metastasis formation, which supports the attractive concept of targeting its gain-of-function activities to limit cancer cell dissemination and metastasis. However, mutant p53 has been neither a targetable cell surface protein nor a druggable enzyme (Levine and Oren, 2009), and therapeutic modalities such as RNAi or restoring wild type p53 conformations have failed to show efficacy in clinical studies (Lehmann and Pietenpol, 2012). Hence, targeting downstream pathways or genes that mediate the activity of mutant p53, such as PDGFRb as disclosed herein, pose an alternative treatment strategy. For example, methods of the disclosure include treating and/or inhibiting early metastatic spread of pancreas cancer by targeting PDGFRb following early detection, such as a preventative approach in patients with familial predisposition to cancer development. In some embodiments, targeting PDGFRb is used in combination with one or more additional therapies, such as after surgical resection of localized disease.

In addition, the data disclosed herein demonstrated correlations between elevated PDGFRb levels, more advanced tumor stage, and poorer disease-free survival in pancreatic as well as colorectal and ovarian cancer patients. Thus, in some embodiments, the disclosure includes the use of PDGFRb levels as a prognostic biomarker for pancreatic cancer progression. For example, PDGFRb levels can be used in conjunction with p53 to identify patient cohorts likely to respond to therapies targeting this axis.

### REFERENCES

Adorno, M., Cordenonsi, M., Montagner, M., Dupont, S., Wong, C., Hann, B., Solari, A., Bobisse, S., Rondina, M.B., Guzzardo, V., et al. (2009). A Mutant p53/Smad complex opposes p63 to empower TGFbeta-induced metastasis. Cell 137, 87-98.
Ballagi, AE., Ishizaki, A., Nehlin JO., and Funa, K. (1995). Isolation and characterization of the mouse PDGF beta-receptor promoter. Biochem Biophys Res Commun. 210, 165-173.
Beppu, K., Jaboine, J., Merchant, MS., Mackall, CL., and Thiele, CJ. (2004). Effect of imatinib mesylate on neuroblastoma tumorigenesis and vascular endothelial growth factor expression. J Natl Cancer Inst. 96, 46-55.
Biankin, A.V., Waddell, N., Kassahn, K.S., Gingras, M.C., Muthuswamy, L.B., Johns, A.L., Miller, D.K., Wilson, P.J., Patch, A.M., Wu, J., et al. (2012). Pancreatic cancer genomes reveal aberrations in axon guidance pathway genes. Nature 491, 399-405.
Brosh, R., and Rotter, V. (2009). When mutants gain new powers: news from the mutant p53 field." Nat Rev Cancer 9, 701-713.
Cao, R., Björndahl, MA., Religa, P., Clasper, S., Garvin, S., Gaiter, D., Meister, B., Ikomi, F., Tritsaris, K., Dissing, S., et al. (2004). PDGF-BB induces intratumoral lymphangiogenesis and promotes lymphatic metastasis. Cancer Cell 6, 333-345.
Dai, C., Celestino, J.C., Okada, Y., Louis, D.N., Fuller, G.N., and Holland, E.C. (2001). PDGF autocrine stimulation dedifferentiates cultured astrocytes and induces oligodendrogliomas and oligoastrocytomas from neural progenitors and astrocytes in vivo. Genes Dev 15, 1913-1925
Dai, Y. (2010). Platelet-derived growth factor receptor tyrosine kinase inhibitors: a review of the recent patent literature. Expert Opin Ther Pat 20, 885-897.
Di Agostino, S., Strano, S., Emiliozzi, V., Zerbini, V., Mottolese, M., Sacchi, A., Blandino, G., and Piaggio, G. (2006). Gain of function of mutant p53: the mutant p53/NF-Y protein complex reveals an aberrant transcriptional mechanism of cell cycle regulation. Cancer Cell 10, 191-202.
Djebali, S., Davis, C., Merkel, A., Dobin, A., Lassmann, T., Mortazavi, A., Tanzer, A., Lagarde, J., Lin, W., Schlesinger, F., Xue, C., et al. (2012). Landscape of transcription in human cells. Nature 489, 101-108.
Dobin, A., Davis. C., Schlesinger, F., Drenkow, J., Zaleski, C., Jha, S., Batut, P., Chaisson, M., Gingeras, TR. (2013). STAR: ultrafast universal RNA-seq aligner. Bioinformatics 29, 15-21.
Dong, P., Karaayvaz, M., Jia, N., Kaneuchi, M., Hamada, J., Watari, H., Sudo, S., Ju, J., and Sakuragi, N. (2013). Mutant p53 gain-of-function induces epithelial-mesenchymal transition through modulation of the miR-130b-ZEB1 axis. Oncogene 32, 3286-3295.
Freed-Pastor, WA., Mizuno, H., Zhao, X., Langerød, A., Moon, SH., Rodriguez-Barrueco, R., Barsotti, A., Chicas, A., Li, W., Polotskaia, A., et al. (2012). Mutant p53 Disrupts Mammary Tissue Architecture via the Mevalonate Pathway. Cell 148, 244-258.
Gaiddon, C., Lokshin, M., Ahn, J., Zhang, T., and Prives, C. (2001). A subset of tumor-derived mutant forms of p53 down-regulate p63 and p73 through a direct interaction with the p53 core domain. Mol Cell Biol. 21, 1874-1887.
Girardini, JE., Napoli, M., Piazza, S., Rustighi, A., Marotta, C., Radaelli, E., Capaci, V., Jordan, L., Quinlan, P., Thompson, A., et al. (2011). A Pin1/mutant p53 axis promotes aggressiveness in breast cancer. Cancer Cell 12, 79-91.
Goulimari, P., Kitzing, T., Knieling, H., Brandt, DT., Offermanns, S., and Grosse, R. (2005). Galpha12/13 is essential for directed cell migration and localized Rho-Dia1 function. J Biol Chem. 280, 42242-42251.
Gril, B., Palmirie, D., Qian, Y., Anwar, T., Liewehr, DJ., Steinberg, SM., Andreu, Z., Masana, D., Fernández, P., Steeg, PS., et al. (2013). Pazopanib inhibits the activation of PDGFRβ-expressing astrocytes in the brain metastatic microenvironment of breast cancer cells. Am J Pathol. 182, 2368-2379
Hackzell, A., Uramoto, H., Izumi, H., Kohno, K., and Funa, K. (2002). p73 independent of c-Myc represses transcription of platelet-derived growth factor beta-receptor through interaction with NF-Y. J Biol Chem. 277, 39769-39776.
Hemann, MT., Fridman, J., Zilfou, JT., Hernando, E., Paddison, PJ., Cordon-Cardo, C., Hannon, GJ., and Lowe, SW. (2003). An epi-allelic series of p53 hypomorphs created by stable RNAi produces distinct tumor phenotypes in vivo. Nat Genet. 33, 396-400.
Hidalgo, M. (2010). Pancreatic cancer. N Engl J Med 362: 1605-1617.
Hingorani, SR., Petricoin, E., Maitra, A., Rajapakse, V., King, C., Jacobetz, MA., Ross, S., Conrads, TP., Veenstra, TD., Hitt, BA., et al. (2003). Preinvasive and invasive ductal pancreatic cancer and its early detection in the mouse. Cancer Cell 4, 437-450.
Hingorani, SR., Wang, L., Multani, AS., Combs, C., Deramaudt, TB., Hruban, RH., Rustgi, AK., Chang, S., and Tuveson, DA. (2005). Trp53R172H and KrasG12D cooperate to promote chromosomal instability and widely metastatic pancreatic ductal adenocarcinoma in mice. Cancer Cell 7, 469-483.
Hoehler, T., von Wichert, G., Schimanski, C., Kanzler, S., Moehler, MH., Hinke, A., Seufferlein, T., Siebler, J., Hochhaus, A., Arnold, D., et al. (2013). Phase I/II trial of capecitabine and oxaliplatin in combination with bevacizumab and imatinib in patients with metastatic colorectal cancer: AIO KRK 0205. Br J Cancer. Sep 17, 1408-1413.
Ishisaki, A., Muramaya, T., Ballagi, AE., and Funa, K. (1997). Nuclear factor Y controls the basal transcription activity of the mouse platelet-derived-growth-factor beta-receptor gene. Eur J Biochem 246, 142-146.
Jackson, EL., Willis, N., Mercer, K., Bronson, RT., Crowley, D., Montoya, R., Jacks, T., and Tuveson, DA. (2001). Analysis of lung tumor initiation and progression using conditional expression of oncogenic K-ras. Genes Dev. 15, 3243-3248.
Joerger, AC., Ang, H., and Fersht, AR. (2006). Structural basis for understanding oncogenic p53 mutations and designing rescue drugs. PNAS 103, 15056-15061.
Jones, S., Zhang, X., Parsons, DW., Lin, JC., Leary, RJ., Angenendt, P., Mankoo, P., Carter, H., Kamiyama, H., and Jimeno, A., (2008). Core signaling pathways in human pancreatic cancers revealed by global genomic analyses. Science 321, 1801-1806.
Kim, Y., Kim, E., Wu, Q., Guryanova, O., Hitomi, M., Lathia, JD., Serwanski, D., Sloan, AE., Weil, RJ., Lee, J., et al. (2012). Platelet-derived growth factor receptors differentially inform intertumoral and intratumoral heterogeneity. Genes Dev. 26, 1247-1262.
Lehmann, BD., and Pietenpol, JA. (2012). Targeting Mutant p53 in Human Tumors." J Clin Oncol. 30, 3648-3650.
Levesque, MA., Katsaros, D., Yu, H., Zola, P., Sismondi, P., Giardina, G., Diamandis, EP. (1995). Mutant p53 protein overexpression is associated with poor outcome in patients with well or moderately differentiated ovarian carcinoma. Cancer. 15, 1327-38.
Levin, AR., and Oren, M. (2009). The first 30 years of p53: growing ever more complex. Nature Reviews Cancer 9, 749-758.
Lewis, NL., Lewis LD., Eder, JP., Reddy, NJ., Guo, F., Pierce, KJ., Olszanski, AJ., and Cohen, RB. (2009). Phase I study of the safety, tolerability, and pharmacokinetics of oral CP-868,596, a highly specific platelet-derived growth factor receptor tyrosine kinase inhibitor in patients with advanced cancers. J Clin Oncol. 27, 5262-5269.
Li, D., Xie, K., Wolff, R., and Abbruzzese, JL. (2004). Pancreatic Cancer. Lancet 363, 1049-1057.
Li, Y., and Prives, C. (2007). "Are interactions with p63 and p73 involved in mutant p53 gain of oncogenic function?" Oncogene 26, 2220-2225.
Liu, K., Ling, S., and Lin, WC. (2011). TopBP1 mediates mutant p53 gain of function through NF-Y and p63/p73. Mol Cell Biol. 31, 4464-4481.
Longati, P., Jia, X., Eimer, J., Wagman, A., Witt, MR., Rehnmark, S., Verbeke, C., Toftgård, R., Löhr, M., and Heuchel, RL. (2013). 3D pancreatic carcinoma spheroids induce a matrix-rich, chemoresistant phenotype offering a better model for drug testing. BMC Cancer 13, 95.
Maitra, A., and Hruban, RH. (2008). Pancreatic cancer. Annu Rev Pathol. 3, 157-188.
Masciarelli, S., Fontemaggi, G., Di Agostino, S., Donzelli, S., Carcarino, E., Strano, S., and Blandino, G. (2013). Gain-of-function mutant p53 downregulates miR-223 contributing to chemoresistance of cultured tumor cells. Oncogene, 15 April 2013; doi:10.1038/onc.2013.106.
Mathey, S., Graeser, M., Zu Eulenburg, C., Woelber, L., Trillsch, F., Jaenicke, F., Müller, V., Milde-Langosch, K., and Mahner, S. (2013). Platelet-Derived Growth Factor Receptor Beta Serum Concentrations during First-Line Therapy in Ovarian Cancer. Oncology 85, 69-77.
Matys, V., Kel-Margoulis, OV., Fricke, E., Liebich, I., Land, S., Barre-Dirrie, A., Reuter, I., Chekmenev, D., Krull, M., Hornischer, K., et al. (2006). TRANSFAC® and its module TRANSCompel®: transcriptional gene regulation in eukaryotes. Nucleic Acids Res. 34, D108-110.
Mehta, SA., Christopherson, KW., Bhat-Nakshatri, P., Goulet, RJ Jr., Broxmeyer, HE., Kopelovich, L., and Nakshatri, H. (2007). Negative regulation of chemokine receptor CXCR4 by tumor suppressor p53 in breast cancer cells: implications of p53 mutation or isoform expression on breast cancer cell invasion. Oncogene. 17, 3329-3337.
Montalbetti, N., Simonin, A., Kovacs, G., and Hediger, MA. (2013) Mammalian iron transporters: families SLC11 and SLC40. Mol Aspects 34, 270-287.
Morton, JP., Timpson, P., Karim, SA., Ridgway, RA., Athineos, D., Doyle, B., Jamieson, NB., Oien, KA., Lowy, AM., Brunton, VG., et al. (2010). Mutant p53 drives metastasis and overcomes growth arrest/senescence in pancreatic cancer. PNAS 107, 246-251.
Muller, PA., Caswell, PT., Doyle, B., Iwanicki, MP., Tan, EH., Karim, S., Lukashchuk, N., Gillespie, DA., Ludwig, RL., Gosselin, P., et al. (2009). Mutant p53 Drives Invasion by Promoting Integrin Recycling. Cell 139, 1327-1341.
Neilsen, PM., Noll, JE., Mattiske, S., Bracken, CP., Gregory, PA., Schulz, RB., Lim, SP., Kumar, R., Suetani, RJ., Goodall, GJ., et al. (2013). Mutant p53 drives invasion in breast tumors through up-regulation of miR-155. Oncogene 13, 2992-3000.
Olive, KP., Tuveson, D., Ruhe, ZC., Yin, B., Willis, NA., Bronson, RT., Crowley, D., and Jacks, T. (2004). Mutant p53 gain of function in two mouse models of Li-Fraumeni syndrome. Cell 119, 847-860.
Oren, M., and Rotter, V. (2010). Mutant p53 gain-of-function in cancer. Cold Spring Harb Perspect Biol. 2:a001107.
Parkhomchuk, D., Borodina, T., Amstislavskiy, V., Banaru, M., Hallen, L., Krobitsch, S., Lehrach, H., and Soldatov, A. (2009). Transcriptome analysis by strand-specific sequencing of complementary DNA. Nucleic Acids Res. 37, e123.
Pietras, K., Sjoblom, T., Rubin, K., Heldin, C.H., and Ostman, A. (2003). PDGF receptors as cancer drug targets. Cancer Cell 3, 439-443.
Rolley, N., Butcher, S., and Milner, J. (1995). Specific DNA binding by different classes of human p53 mutants. Oncogene. 11, 763-770.
Su, X., Chakravarti, D., Cho, MS., Liu, L., Gi, YJ., Lin, YL., Leung, ML., El-Naggar, A., Creighton, CJ., Suraokar, MB., et al. (2010). TAp63 suppresses metastasis through coordinate regulation of Dicer and miRNAs. Nature 467, 986-990.
Uramoto, H., Wetterskog, D., Hackzell, A., Matsumoto, and Y., Funa, K. (2004). p73 competes with co-activators and recruits histone deacetylase to NF-Y in the repression of PDGF beta-receptor. J Cell Sci. 117, 5323-5331.
Vogelstein, B., Lane, D., and Levine, A. J. (2000). Surfing the p53 network. Nature 408, 307-310.
Wang, P., Reed, M., Wang, Y., Mayr, G., Stenger, J.E., Anderson, M.E., Schwedes, J.F., Tegtmeyer, P. (1994). p53 domains: structure, oligomerization, and transformation. Mol Cell Biol. 8:5182-5191.
Weisz, L., Oren, M., and Rotter, V. (2007). Transcription regulation by mutant p53. Oncogene 26, 2202-2211.
Wolff, NC., Richardson, JA., Egorin, M., and Ilaria, RL Jr (2003). The CNS is a sanctuary for leukemic cells in mice receiving imatinib mesylate for Bcr/Abl-induced leukemia. Blood 101, 5010-5013.
Yachida, S., Jones, S., Bozic, I., Antal, T., Leary, R., Fu, B., Kamiyama, M., Hruban, RH., Eshleman, JR., Nowak, MA., et al. (2010). Distant metastasis occurs late during the genetic evolution of pancreatic cancer. Nature 467, 1114-1117.
Zuber, J., McJunkin, K., Fellmann, C., Dow, LE., Taylor, MJ., Hannon, GJ., and Lowe, SW. (2011). Toolkit for evaluating genes required for proliferation and survival using tetracycline-regulated RNAi. Nat Biotechnol. 29, 79-83.

### Sequences

SEQ ID NO:1 (Human p53 nucleotide sequence)
1 cgtgctttcc acgacggtga cacgcttccc tggattggcc agactgcctt ccgggtcact
61 gccatggagg agccgcagtc agatcctagc gtcgagcccc ctctgagtca ggaaacattt
121 tcagacctat ggaaactact tcctgaaaac aacgttctgt cccccttgcc gtcccaagca
181 atggatgatt tgatgctgtc cccggacgat attgaacaat ggttcactga agacccaggt
241 ccagatgaag ctcccagaat gccagaggct gctccccgcg tggcccctgc accagcagct
301 cctacaccgg cggcccctgc accagccccc tcctggcccc tgtcatcttc tgtcccttcc
361 cagaaaacct accagggcag ctacggtttc cgtctgggct tcttgcattc tgggacagcc
421 aagtctgtga cttgcacgta ctcccctgcc ctcaacaaga tgttttgcca actggccaag
481 acctgccctg tgcagctgtg ggttgattcc acacccccgc ccggcacccg cgtccgcgcc
541 atggccatct acaagcagtc acagcacatg acggaggttg tgaggcgctg cccccaccat
601 gagcgctgct cagatagcga tggtctggcc cctcctcagc atcttatccg agtggaagga
661 aatttgcgtg tggagtattt ggatgacaga aacacttttc gacatagtgt ggtggtgccc
721 tatgagccgc ctgaggttgg ctctgactgt accaccatcc actacaacta catgtgtaac
781 agttcctgca tgggcggcat gaaccggagg cccatcctca ccatcatcac actggaagac
841 tccagtggta atctactggg acggaacagc tttgaggtgc atgtttgtgc ctgtcctggg
901 agagaccggc gcacagagga agagaatctc cgcaagaaag gggagcctca ccacgagctg
961 cccccaggga gcactaagcg agcactgtcc aacaacacca gctcctctcc ccagccaaag
1021 aagaaaccac tggatggaga atatttcacc cttcagatcc gtgggcgtga gcgcttcgag
1081 atgttccgag agctgaatga ggccttggaa ctcaaggatg cccaggctgg gaaggagcca
1141 ggggggagca gggctcactc cagccacctg aagtccaaaa agggtcagtc tacctcccgc
1201 cataaaaaac tcatgttcaa gacagaaggg cctgactcag actgacattc tccacttctt
1261 gttccccact gacagcctcc cacccccatc tctccctccc ctgccatttt gggttttggg
1321 tctttgaacc cttgcttgca ataggtgtgc gtcagaagca cccaggactt ccatttgctt
1381 tgtcccgggg ctccactgaa caagttggcc tgcactggtg ttttgttgtg gggaggagga
1441 tggggagtag gacataccag cttagatttt aaggttttta ctgtgaggga tgtttgggag
1501 atgtaagaaa tgttcttgca gttaagggtt agtttacaat cagccacatt ctaggtaggg
1561 gcccacttca ccgtactaac cagggaagct gtccctcact gttgaatttt ctctaacttc
1621 aaggcccata tctgtgaaat gctggcattt gcacctacct cacagagtgc attgtgaggg
1681 ttaatgaaat aatgtacatc tggccttgaa accacctttt attacatggg gtctagaact
1741 tgaccccctt gagggtgctt gttccctctc cctgttggtc ggtgggttgg tagtttctac
1801 agttgggcag ctggttaggt agagggagtt gtcaagtctc tgctggccca gccaaaccct
1861 gtctgacaac ctcttggtga accttagtac ctaaaaggaa atctcacccc atcccacacc
1921 ctggaggatt tcatctcttg tatatgatga tctggatcca ccaagacttg ttttatgctc
1981 agggtcaatt tcttttttct tttttttttt tttttctttt ttctttgaga ctgggtctcg
2041 ctttgttgcc caggctggag tggagtggcg tgatcttggc ttactgcagc ctttgcctcc
2101 ccggctcgag cagtcctgcc tcagcctccg gagtagctgg gaccacaggt tcatgccacc
2161 atggccagcc aacttttgca tgttttgtag agatggggtc tcacagtgtt gcccaggctg
2221 gtctcaaact cctgggctca ggcgatccac ctgtctcagc ctcccagagt gctgggatta
2281 caattgtgag ccaccacgtc cagctggaag ggtcaacatc ttttacattc tgcaagcaca
2341 tctgcatttt caccccaccc ttcccctcct tctccctttt tatatcccat ttttatatcg
2401 atctcttatt ttacaataaa actttgctgc caaaaaaaaa aaaaaaaaaa a

SEQ ID NO:2 (Human p53 amino acid sequence)
10 20 30 40 50 60


70 80 90 100 110 120


130 140 150 160 170 180


190 200 210 220 230 240


250 260 270 280 290 300


310 320 330 340 350 360


370 380 390


SEQ ID NO:3 (human PDGFRb nucleotide sequence)
1 ctcctgaggc tgccagcagc cagcagtgac tgcccgccct atctgggacc caggatcgct
61 ctgtgagcaa cttggagcca gagaggagat caacaaggag gaggagagag ccggcccctc
121 agccctgctg cccagcagca gcctgtgctc gccctgccca acgcagacag ccagacccag
181 ggcggcccct ctggcggctc tgctcctccc gaaggatgct tggggagtga ggcgaagctg
241 ggccgctcct ctcccctaca gcagccccct tcctccatcc ctctgttctc ctgagccttc
301 aggagcctgc accagtcctg cctgtccttc tactcagctg ttacccactc tgggaccagc
361 agtctttctg ataactggga gagggcagta aggaggactt cctggagggg gtgactgtcc
421 agagcctgga actgtgccca caccagaagc catcagcagc aaggacacca tgcggcttcc
481 gggtgcgatg ccagctctgg ccctcaaagg cgagctgctg ttgctgtctc tcctgttact
541 tctggaacca cagatctctc agggcctggt cgtcacaccc ccggggccag agcttgtcct
601 caatgtctcc agcaccttcg ttctgacctg ctcgggttca gctccggtgg tgtgggaacg
661 gatgtcccag gagcccccac aggaaatggc caaggcccag gatggcacct tctccagcgt
721 gctcacactg accaacctca ctgggctaga cacgggagaa tacttttgca cccacaatga
781 ctcccgtgga ctggagaccg atgagcggaa acggctctac atctttgtgc cagatcccac
841 cgtgggcttc ctccctaatg atgccgagga actattcatc tttctcacgg aaataactga
901 gatcaccatt ccatgccgag taacagaccc acagctggtg gtgacactgc acgagaagaa
961 aggggacgtt gcactgcctg tcccctatga tcaccaacgt ggcttttctg gtatctttga
1021 ggacagaagc tacatctgca aaaccaccat tggggacagg gaggtggatt ctgatgccta
1081 ctatgtctac agactccagg tgtcatccat caacgtctct gtgaacgcag tgcagactgt
1141 ggtccgccag ggtgagaaca tcaccctcat gtgcattgtg atcgggaatg aggtggtcaa
1201 cttcgagtgg acataccccc gcaaagaaag tgggcggctg gtggagccgg tgactgactt
1261 cctcttggat atgccttacc acatccgctc catcctgcac atccccagtg ccgagttaga
1321 agactcgggg acctacacct gcaatgtgac ggagagtgtg aatgaccatc aggatgaaaa
1381 ggccatcaac atcaccgtgg ttgagagcgg ctacgtgcgg ctcctgggag aggtgggcac
1441 actacaattt gctgagctgc atcggagccg gacactgcag gtagtgttcg aggcctaccc
1501 accgcccact gtcctgtggt tcaaagacaa ccgcaccctg ggcgactcca gcgctggcga
1561 aatcgccctg tccacgcgca acgtgtcgga gacccggtat gtgtcagagc tgacactggt
1621 tcgcgtgaag gtggcagagg ctggccacta caccatgcgg gccttccatg aggatgctga
1681 ggtccagctc tccttccagc tacagatcaa tgtccctgtc cgagtgctgg agctaagtga
1741 gagccacccc gacagtgggg aacagacagt ccgctgtcgt ggccggggca tgccccagcc
1801 gaacatcatc tggtctgcct gcagagacct caaaaggtgt ccacgtgagc tgccgcccac
1861 gctgctgggg aacagttccg aagaggagag ccagctggag actaacgtga cgtactggga
1921 ggaggagcag gagtttgagg tggtgagcac actgcgtctg cagcacgtgg atcggccact
1981 gtcggtgcgc tgcacgctgc gcaacgctgt gggccaggac acgcaggagg tcatcgtggt
2041 gccacactcc ttgcccttta aggtggtggt gatctcagcc atcctggccc tggtggtgct
2101 caccatcatc tcccttatca tcctcatcat gctttggcag aagaagccac gttacgagat
2161 ccgatggaag gtgattgagt ctgtgagctc tgacggccat gagtacatct acgtggaccc
2221 catgcagctg ccctatgact ccacgtggga gctgccgcgg gaccagcttg tgctgggacg
2281 caccctcggc tctggggcct ttgggcaggt ggtggaggcc acggctcatg gcctgagcca
2341 ttctcaggcc acgatgaaag tggccgtcaa gatgcttaaa tccacagccc gcagcagtga
2401 gaagcaagcc cttatgtcgg agctgaagat catgagtcac cttgggcccc acctgaacgt
2461 ggtcaacctg ttgggggcct gcaccaaagg aggacccatc tatatcatca ctgagtactg
2521 ccgctacgga gacctggtgg actacctgca ccgcaacaaa cacaccttcc tgcagcacca
2581 ctccgacaag cgccgcccgc ccagcgcgga gctctacagc aatgctctgc ccgttgggct
2641 ccccctgccc agccatgtgt ccttgaccgg ggagagcgac ggtggctaca tggacatgag
2701 caaggacgag tcggtggact atgtgcccat gctggacatg aaaggagacg tcaaatatgc
2761 agacatcgag tcctccaact acatggcccc ttacgataac tacgttccct ctgcccctga

2821 gaggacctgc cgagcaactt tgatcaacga gtctccagtg ctaagctaca tggacctcgt
2881 gggcttcagc taccaggtgg ccaatggcat ggagtttctg gcctccaaga actgcgtcca
2941 cagagacctg gcggctagga acgtgctcat ctgtgaaggc aagctggtca agatctgtga
3001 ctttggcctg gctcgagaca tcatgcggga ctcgaattac atctccaaag gcagcacctt
3061 tttgccttta aagtggatgg ctccggagag catcttcaac agcctctaca ccaccctgag
3121 cgacgtgtgg tccttcggga tcctgctctg ggagatcttc accttgggtg gcacccctta
3181 cccagagctg cccatgaacg agcagttcta caatgccatc aaacggggtt accgcatggc
3241 ccagcctgcc catgcctccg acgagatcta tgagatcatg cagaagtgct gggaagagaa
3301 gtttgagatt cggcccccct tctcccagct ggtgctgctt ctcgagagac tgttgggcga
3361 aggttacaaa aagaagtacc agcaggtgga tgaggagttt ctgaggagtg accacccagc
3421 catccttcgg tcccaggccc gcttgcctgg gttccatggc ctccgatctc ccctggacac
3481 cagctccgtc ctctatactg ccgtgcagcc caatgagggt gacaacgact atatcatccc
3541 cctgcctgac cccaaacccg aggttgctga cgagggccca ctggagggtt cccccagcct
3601 agccagctcc accctgaatg aagtcaacac ctcctcaacc atctcctgtg acagcccct
3661 ggagccccag gacgaaccag agccagagcc ccagcttgag ctccaggtgg agccggagcc
3721 agagctggaa cagttgccgg attcggggtg ccctgcgcct cgggcggaag cagaggatag
3781 cttcctgtag ggggctggcc cctaccctgc cctgcctgaa gctccccccc tgccagcacc
3841 cagcatctcc tggcctggcc tgaccgggct tcctgtcagc caggctgccc ttatcagctg
3901 tccccttctg gaagctttct gctcctgacg tgttgtgccc caaaccctgg ggctggctta
3961 ggaggcaaga aaactgcagg ggccgtgacc agccctctgc ctccagggag gccaactgac
4021 tctgagccag ggttccccca gggaactcag ttttcccata tgtaagatgg gaaagttagg
4081 cttgatgacc cagaatctag gattctctcc ctggctgaca ggtggggaga ccgaatccct
4141 ccctgggaag attcttggag ttactgaggt ggtaaattaa cttttttctg ttcagccagc
4201 tacccctcaa ggaatcatag ctctctcctc gcacttttat ccacccagga gctagggaag
4261 agaccctagc ctccctggct gctggctgag ctagggccta gccttgagca gtgttgcctc
4321 atccagaaga aagccagtct cctccctatg atgccagtcc ctgcgttccc tggcccgagc
4381 tggtctgggg ccattaggca gcctaattaa tgctggaggc tgagccaagt acaggacacc
4441 cccagcctgc agcccttgcc cagggcactt ggagcacacg cagccatagc aagtgcctgt
4501 gtccctgtcc ttcaggccca tcagtcctgg ggctttttct ttatcaccct cagtcttaat
4561 ccatccacca gagtctagaa ggccagacgg gccccgcatc tgtgatgaga atgtaaatgt
4621 gccagtgtgg agtggccacg tgtgtgtgcc agtatatggc cctggctctg cattggacct
4681 gctatgaggc tttggaggaa tccctcaccc tctctgggcc tcagtttccc cttcaaaaaa
4741 tgaataagtc ggacttatta actctgagtg ccttgccagc actaacattc tagagtattc
4801 caggtggttg cacatttgtc cagatgaagc aaggccatat accctaaact tccatcctgg
4861 gggtcagctg ggctcctggg agattccaga tcacacatca cactctgggg actcaggaac
4921 catgcccctt ccccaggccc ccagcaagtc tcaagaacac agctgcacag gccttgactt
4981 agagtgacag ccggtgtcct ggaaagcccc cagcagctgc cccaggacat tgggaagacc
5041 acgggacctc tttcactacc cacgatgacc tccgggggta tcctgggcaa aagggacaaa
5101 gagggcaaat gagatcacct cctgcagccc accactccag cacctgtgcc gaggtctgcg
5161 tcgaagacag aatggacagt gaggacagtt atgtcttgta aaagacaaga agcttcagat
5221 gggtacccca agaaggatgt gagaggtggg cgctttggag gtttgcccct cacccaccag
5281 ctgccccatc cctgaggcag cgctccatgg gggtatggtt ttgtcactgc ccagacctag
5341 cagtgacatc tcattgtccc cagcccagtg ggcattggag gtgccagggg agtcaggtt
5401 gtagccaaga cgcccccgca cggggagggt tgggaagggg gtgcaggaag ctcaacccct
5461 ctgggcacca accctgcatt gcaggttggc accttacttc cctgggatcc ccagagttgg
5521 tccaaggagg gagagtgggt tctcaatacg gtaccaaaga tataatcacc taggtttaca
5581 aatattttta ggactcacgt taactcacat ttatacagca gaaatgctat tttgtatgct
5641 gttaagtttt tctatctgtg tacttttttt taagggaaag attttaatat taaacctggt
5701 gcttctcact cacaaaaa

SEQ ID NO:4 (human PDGFRb amino acid sequence)







































## Claims

1. A therapeutic agent that inhibits a platelet derived growth factor receptor beta (PDGFRβ) for use in a method of treating or preventing tumor metastasis, in a subject having a gain of function p53 mutation that increases its oncogenic properties relative to wild type, wherein the tumor is a pancreatic cancer tumor, a colorectal cancer tumor, or an ovarian cancer tumor.

2. The therapeutic agent for use as claimed in claim 1, wherein the therapeutic agent reduces expression or activity of the PDGFRβ.

3. A therapeutic agent that reduces expression or activity of a PDGFRβ, for use in a method of treating a subject having a tumor comprising a gain of function p53 mutation, wherein the tumor is a pancreatic cancer tumor, a colorectal cancer tumor, or an ovarian cancer tumor.

4. The therapeutic agent for use as claimed in any one of claims 1-3, wherein the therapeutic agent reduces tumor metastasis.

5. The therapeutic agent for use as claimed in any one of claims 1-4, wherein the therapeutic agent is an antibody, a nucleic acid, or a small molecule.

6. The therapeutic agent for use as claimed in any one of claims 1-5, wherein the therapeutic agent is imatinib.

7. The therapeutic agent for use as claimed in any one of claims 1-5, wherein the therapeutic agent is a nucleic acid that binds to SEQ ID NO:3 or a portion thereof.

8. The therapeutic agent for use as claimed in any one of claims 1-5, wherein the therapeutic agent is an antibody that binds to SEQ ID NO:4 or a portion thereof.

9. The therapeutic agent for use as claimed in any one of claims 1-8, further comprising detecting a gain of function p53 mutation in the tumor.

10. The therapeutic agent for use as claimed in claim 9, wherein the gain of function p53 mutation is detected in a sample of the tumor.

11. The therapeutic agent for use as claimed in claim 9, wherein the gain of function p53 mutation is detected in the tumor *in vivo.*

12. A method of identifying a subject for treatment with a therapeutic agent that targets a PDGFRβ, the method comprising:
detecting in a sample from the subject a tumor comprising a gain of function p53 mutation; and
selecting the subject for treatment with a therapeutic agent that targets a PDGFRβ, wherein the tumor is a pancreatic cancer tumor, a colorectal cancer tumor, or an ovarian cancer tumor.

13. The therapeutic agent for use as claimed in any one of claims 1-6, wherein the oncogenic properties include tumor cell proliferation, tumor cell survival, and tumor cell invasion and metastasis.

## Patentansprüche

1. Therapeutikum, das einen Thrombozyten-abgeleiteten Wachstumsfaktor-Rezeptor β (Platelet Derived Growth Factor Receptor Beta, PDGFRβ) inhibiert, zur Verwendung in einem Verfahren zur Behandlung oder Prävention der Tumormetastase in einem Individuum mit einer P53-Funktionsgewinn-Mutation, die seine onkogenen Eigenschaften im Vergleich zu dem Wildtyp verstärkt, wobei es sich bei dem Tumor um einen Bauchspeicheldrüsenkrebs-, einen Kolorektalkrebs- oder einen Eierstockkrebs-Tumor handelt.

2. Therapeutikum zur Verwendung nach Anspruch 1, wobei das Therapeutikum die Expression oder die Aktivität von PDGFRβ reduziert.

3. Therapeutikum, das die Expression oder die Aktivität eines PDGFRβ reduziert, zur Verwendung in einem Verfahren, bei dem man ein Individuum mit einem Tumor, der eine einen Funktionsgewinn aufweisende P53-Mutation aufweist, behandelt, wobei es sich bei dem Tumor um einen Bauchspeicheldrüsenkrebs-, einen Kolorektalkrebs- oder einen Eierstockkrebs-Tumor handelt.

4. Therapeutikum zur Verwendung nach einem der Ansprüche 1 bis 3, wobei das Therapeutikum die Tumormetastase reduziert.

5. Therapeutikum zur Verwendung nach einem der Ansprüche 1 bis 4, wobei es sich bei dem Therapeutikum um einen Antikörper, eine Nukleinsäure oder eine wieder molekulare Substant handelt.

6. Therapeutikum zur Verwendung nach einem der Ansprüche 1 bis 5, wobei es sich bei dem Therapeutikum um Imatinib handelt.

7. Therapeutikum zur Verwendung nach einem der Ansprüche 1 bis 5, wobei es sich bei dem Therapeutikum um eine Nukleinsäure handelt, die an SEQ ID NO:3 oder einen Teilabschnitt davon bindet.

8. Therapeutikum zur Verwendung nach einem der Ansprüche 1 bis 5, wobei es sich bei dem Therapeutikum um einen Antikörper handelt, der an SEQ ID NO:4 oder einen Teilabschnitt davon bindet.

9. Therapeutikum zur Verwendung nach einem der Ansprüche 1 bis 8, das weiterhin den Nachweis einer P53-Funktionsgewinn-Mutation in dem Tumor umfasst.

10. Therapeutikum zur Verwendung nach Anspruch 9, wobei die P53-Funktionsgewinn-Mutation in einer Probe des Tumors nachgewiesen wird.

11. Therapeutikum zur Verwendung nach Anspruch 9, wobei die P53-Funktionsgewinn-Mutation in dem Tumor *in vivo* nachgewiesen wird.

12. Verfahren zur Ermittlung eines Individuums für die Behandlung mit einem auf PDGFRβ abzielenden Therapeutikum, wobei das Verfahren
den Nachweis eines Tumors mit P53-Funktionsgewinn-Mutation in einer von dem Individuum stammenden Probe und
die Auswahl des Individuums für die Behandlung mit einem auf PDGFRβ abzielenden Therapeutikum, wobei es sich bei dem Tumor um einen Bauchspeicheldrüsenkrebs-, einen Kolorektalkrebs- oder einen Eierstockkrebs-Tumor handelt,
umfasst.

13. Therapeutikum zur Verwendung nach einem der Ansprüche 1 bis 6, wobei die onkogenen Eigenschaften Tumorzellenproliferation, das Überleben von Tumorzellen und Tumorzellinvasion und -metastase beinhalten.

## Revendications

1. Agent thérapeutique qui inhibe un récepteur bêta du facteur de croissance dérivé des plaquettes (PDGFRβ), pour une utilisation dans une méthode de traitement ou de prévention d'une métastase tumorale, chez un sujet présentant une mutation de p53 à gain de fonction qui augmente ses propriétés oncogènes par rapport au type sauvage, où la tumeur est une tumeur cancéreuse du pancréas, une tumeur cancéreuse colorectale, ou une tumeur cancéreuse de l'ovaire.

2. Agent thérapeutique pour une utilisation selon la revendication 1, l'agent thérapeutique réduisant l'expression ou l'activité du PDGFRβ.

3. Agent thérapeutique réduisant l'expression ou l'activité d'un PDGFRβ, pour une utilisation dans une méthode de traitement d'un sujet possédant une tumeur comprenant une mutation de p53 à gain de fonction, où la tumeur est une tumeur cancéreuse du pancréas, une tumeur cancéreuse colorectale, ou une tumeur cancéreuse de l'ovaire.

4. Agent thérapeutique pour une utilisation selon l'une quelconque des revendications 1-3, l'agent thérapeutique réduisant la métastase tumorale.

5. Agent thérapeutique pour une utilisation selon l'une quelconque des revendications 1-4, l'agent thérapeutique étant un anticorps, un acide nucléique ou une petite molécule.

6. Agent thérapeutique pour une utilisation selon l'une quelconque des revendications 1-5, l'agent thérapeutique étant l'imatinib.

7. Agent thérapeutique pour une utilisation selon l'une quelconque des revendications 1-5, l'agent thérapeutique étant un acide nucléique qui se fixe à SEQ ID n° 3 ou une portion de celle-ci.

8. Agent thérapeutique pour une utilisation selon l'une quelconque des revendications 1-5, l'agent thérapeutique étant un anticorps qui se fixe à SEQ ID n° 4 ou une portion de celle-ci.

9. Agent thérapeutique pour une utilisation selon l'une quelconque des revendications 1-8, comprenant en outre la détection d'une mutation de p53 à gain de fonction dans la tumeur.

10. Agent thérapeutique pour une utilisation selon la revendication 9, où la mutation de p53 à gain de fonction est détectée dans un échantillon de la tumeur.

11. Agent thérapeutique pour une utilisation selon la revendication 9, où la mutation de p53 à gain de fonction est détectée dans la tumeur *in vivo.*

12. Méthode d'identification d'un sujet pour un traitement par un agent thérapeutique qui cible un PDGFRβ, la méthode comprenant :
la détection, dans un échantillon issu du sujet, d'une tumeur comprenant une mutation de p53 à gain de fonction ; et
la sélection du sujet pour un traitement par un agent thérapeutique qui cible un PDGFRβ, où la tumeur est une tumeur cancéreuse du pancréas, une tumeur cancéreuse colorectale, ou une tumeur cancéreuse de l'ovaire.

13. Agent thérapeutique pour une utilisation selon l'une quelconque des revendications 1-6, où les propriétés oncogènes comportent la prolifération de cellules tumorales, la survie de cellules tumorales, et l'invasion et la métastase de cellules tumorales.
